# EUROPEAN PATENT APPLICATION

(11) **EP 4 782 449 A1**
(43) Date of publication of application: **29.07.2026**
(21) Application number: 24867386.5
(22) Date of filing: 14.09.2024
(51) Int. Cl.: C07D 471/04, C07D 487/04, C07D 487/14, C07D 491/04, C07D 491/147, A61K 31/4353, A61K 31/4375, A61P 25/16, A61P 25/28

(54) **TRICYCLIC PYRIDINE COMPOUND ACTING AS SARM1 ENZYME ACTIVITY INHIBITOR, AND USE THEREOF**

(30) Priority: 20.09.2023 CN 202311222898
(71) Applicant: Artivila (ShenZhen) Innovation Center, Ltd., Guangdong, 518172 (CN)
(72) Inventor: NIU, Deqiang, Shenzhen, Guangdong 518172 (CN); ZHU, Zhendong, Shenzhen, Guangdong 518172 (CN); FAN, Ming, Shenzhen, Guangdong 518172 (CN); ZHENG, Zihua, Shenzhen, Guangdong 518172 (CN)
(74) Representative: Boult Wade Tennant LLP
(86) International application number: PCT/CN2024/119050
(87) International publication number: WO 2025/060978

(57) **Abstract**

The present invention provides a use of an SARM1 enzyme activity inhibitor in treating neurodegenerative diseases or neurological diseases or conditions, and specifically provides a compound of formula (I) acting as an SARM1 enzyme activity inhibitor, and a pharmaceutical composition thereof.

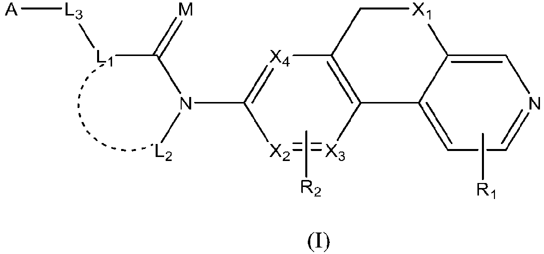

## Description

The present application claims the priority of Chinese Patent Application No. 202311222898.2, filed with the China National Intellectual Property Administration on September 20, 2023, and titled with "TRICYCLIC PYRIDINE COMPOUND ACTING AS SARM1 ENZYME ACTIVITY INHIBITOR, AND USE THEREOF", which is hereby incorporated by reference in its entirety.

### FIELD

The present application relates to compounds for inhibiting SARM1 enzyme activity and uses thereof in treating and/or preventing neurodegenerative or neurological diseases or disorders related to SARM1 enzyme activity.

### BACKGROUND

Neurodegenerative diseases are a class of diseases that can seriously harm human health and can cause destructive damage, such as progressive diseases with neuron death. As primary neurodegenerative diseases, Alzheimer's disease, Parkinson's disease, amyotrophic lateral sclerosis (ALS), Huntington's disease, and other central nervous system diseases and peripheral neuropathy such as diabetic peripheral neuropathy (DPN) are known. Most of them are related to aging, the onset of these diseases increases with age. However, there are also cases where onset occurs in middle-aged people or even younger individuals.

With deepening research into the brain's structure and function, the roles of neurotransmitters and neurotrophic factors have been gradually elucidated, but many local causes related to neurodegeneration are still unclear. Only for Parkinson's disease, the relationship between the disease and dopamine (a special neurotransmitter), has been elucidated, and dopamine precursor L-dopa has been used as a medicament for alleviating neurological symptoms and restoring neurological functions. However, L-dopa cannot prevent the progression of neurodegeneration, and gradually loses its effect as the disease advance, leading to the degeneration and loss of dopaminergic neurons. Similarly, Alzheimer's disease is also caused by degeneration and loss of various neurons such as cholinergic neurons and monoaminergic neurons, and as drugs for treating this disease, cholinesterase inhibitors have been marketed or are under development. However, L-dopa for treating Parkinson's disease is still limited to symptomatic treatment for temporarily improving neurological symptoms.

Therefore, until now, there is a particular lack of effective therapeutic drugs for neurodegenerative diseases.

Research has found that axonal injury occurs in various neurological diseases such as neurodegenerative diseases and accidental injuries. Axonal degeneration can cause structural necrosis and functional disorder of the peripheral nervous system, ultimately leading to acquired or inherited degenerative diseases of central nervous system.

Although there is currently no highly effective pharmacological method that can accurately assess the weight of the incidence rate of the disease by axonal degeneration, it has been found in histopathological studies that significant axonal injury and degeneration are observed in the early stages of various neuropathies such as Alzheimer's disease, Parkinson's disease, multiple sclerosis, amyotrophic lateral sclerosis, and peripheral neuropathy, indicating that axonal degeneration plays an important role in the occurrence and development of neuropathies (Fischer et al., Neuro-degenerative Diseases, 2007, 4:431-442). Therefore, by attenuating or even blocking axonal degeneration, maintaining the integrity of neuronal structure and function may be a therapeutic regimen that benefits various neurological diseases.

In the absence of effective therapeutic medicaments for neurodegenerative diseases, there is an urgent need in the prior art to research and develop new compounds, especially small molecule, including compounds that have effects on axonal degeneration.

### SUMMARY

After long-term research, inventors of the present disclosure surprisingly discovered a class of compounds having significant SARM1 enzyme activity inhibitory effect, and found that the compounds can ameliorate axonal degeneration and can be used for treating or preventing neurodegenerative diseases and disorders associated therewith.

SARM1 (Sterile alpha and TIR motif containing 1) protein consists of three domains, namely an N-terminal ARM (Armadillo/HEAT repeat) domain, two tandem SAM (Sterile alpha motif) domains, and a C-terminal TIR (Toll/Interleukin Receptor) domain, and furthermore, there is a mitochondrial targeting signal peptide at the N-terminus.

It is known that, in wild-type neurons, axonal injury induces NAD⁺ depletion and axonal degeneration; knockout SARM1 in neurons can inhibit axonal degeneration, and NAD⁺ is maintained at normal levels, indicating that SARM1 promotes consumption of NAD⁺ and exacerbates axonal degeneration.

The Milbrandt research group at Washington University School of Medicine produced the TIR domain of SARM1 (SARM1-TIR) and discovered that it has NAD⁺ hydrolase activity. Furthermore, high-purity SARM1-TIR was obtained through rigorous *Escherichia coli* expression and purification experiments and cell-free expression systems, finally proving that SARM1-TIR is capable of catalyzing NAD⁺ to produce adenosine 5'-diphosphate ribose (ADPR) and cyclic adenosine 5'-diphosphate ribose (cADPR).

SARM1 is a multifunctional signaling enzyme capable of catalyzing various substrates such as NAD⁺, NADP⁺, and NA to generate signaling molecules such as cADPR, ADPR, and NAADP. In various neurodegenerative diseases, SARM1 is activated, leading to NAD⁺ depletion, thereby initiating a novel cell death mechanism; knockout SARM1 can inhibit axonal degeneration and disease progression, and therefore SARM1 is considered a potential drug target for related neurological diseases, including TBI (traumatic brain injury), AD (Alzheimer's disease), CIPN (chemotherapy induced peripheral neuropathy), ALS (amyotrophic lateral sclerosis), and others.

In the present disclosure, inventors produced full-length SARM1 for NAD enzyme activity experiments, and used it for screening, and compound molecules of the present disclosure having inhibitory capability to the enzyme activity were obtained.

Therefore, based on the above discoveries, in a first aspect, the present disclosure provides a compound of formula (I) that can serve as a SARM1 enzyme activity inhibitor: 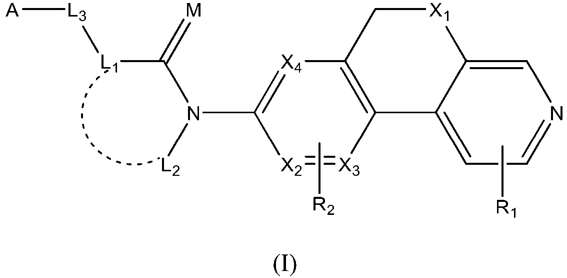
or a racemate, an enantiomer, a diastereomer, a deuterated compound, or a pharmaceutically acceptable salt thereof,
wherein,
X₁ is selected from the group consisting of -O-, -CH₂-, -OCH₂-, -NR₃CH₂-, -CH₂NR₃-, -N=CH-, and -CH=N-, preferably -O-;
wherein R₃ is selected from the group consisting of H and C₁-C₃ alkyl;
X₂, X₃, and X₄ are each independently selected from the group consisting of CH and N;
R₁ is independently selected from the group consisting of H, halogen, C₁-C₃ alkyl, cyano, trifluoromethyl, amino, hydroxy, methoxy, and -C(O)NH₂, preferably H, F, Cl, methyl, cyano, and -C(O)NH₂;
R₂ is independently selected from the group consisting of H, halogen, C₁-C₃ alkyl, cyano, trifluoromethyl, amino, hydroxy, methoxy, C₁-C₃ alkyl-OC(O)-, and C₁-C₃ alkyl-NHC(O)-, preferably H, cyano, Cl, methyl, and methoxy;
M represents O, S, or NH, preferably O;
L₁ and L₃ are each independently selected from the group consisting of -CH(R')-, -O-, -S-, and -N(R')-, provided that at least one of L₁ and L₃ is -CH(R')-, wherein R' is hydrogen or C₁-C₃ alkyl;
L₂ is selected from the group consisting of H, -CH₃, -CH₂-CH₃, -CH₂-CH₂-CH₃, -OR₈, and -NHR₉;
wherein R₈ and R₉ are independently selected from the group consisting of H and C₁-C₃ alkyl;
A is selected from the group consisting of H, a 5- to 10-membered aryl, a 5- to 10-membered heteroaryl, a 5- to 10-membered cycloalkyl, a 5- to 10-membered heterocycloalkyl, a 5- to 10-membered aryl fused with a 3- to 6-membered cycloalkyl, and a 5- to 10-membered aryl fused with a 3- to 6-membered heterocycloalkyl, wherein the heteroaryl or heterocycloalkyl may contain 1 or 2 heteroatoms selected from N, O, and S, and the 5- to 10-membered aryl, 5- to 10-membered heteroaryl, 5- to 10-membered aryl fused with a 3- to 6-membered cycloalkyl, and 5- to 10-membered aryl fused with a 3- to 6-membered heterocycloalkyl may be substituted by 1, 2, or 3 substituents selected from the group consisting of halogen, C₁-C₃ alkyl, C₁-C₃ alkoxy, halogenated C₁-C₃ alkyl, halogenated C₁-C₃ alkoxy, cyano, nitro, and (RaRa')N-, wherein R^{a} and R^{a}' are each independently selected from hydrogen and C₁-C₃ alkyl; preferably, the substituent is selected from the group consisting of methyl, methoxy, trifluoromethyl, trifluoromethoxy, F, Cl, and Br;
L₁ may be connected to L2 to form a 5-membered ring or a 6-membered ring, preferably forming a 5-membered ring.

In a preferred aspect, the compound of Formula (I) has the structure of Formula (II) as follows: 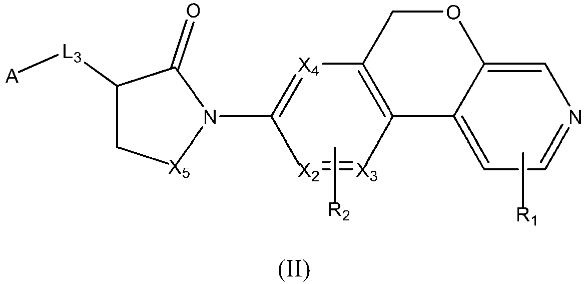 wherein,
X₂, X₃, X₄, R₁, R₂, L₃, and A are as defined above;
X₅ is selected from the group consisting of CH₂, O, and NH, preferably CH₂.

In a preferred aspect, the compound of formula (I) of the present disclosure has the following structure of formula (III): 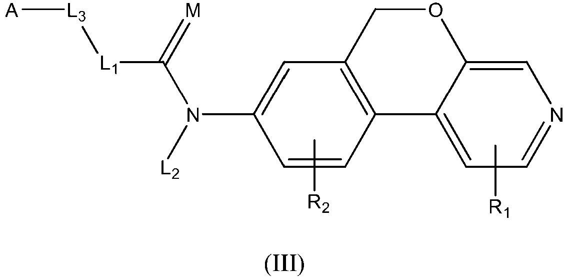 wherein
R₁, R₂, M, L₁, L₂, L₃, and A are as defined above.

In a preferred aspect, the compound of formula (I) of the present disclosure has the following structure of formula (IV): 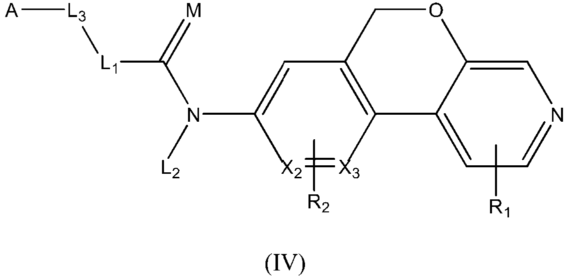 wherein
X₂ and X₃ are selected from the group consisting of CH and N, and X₂ and X₃ are not both CH;
R₁, R₂, M, L₁, L₂, L₃, and A are as defined above.

In a preferred aspect, the compound of formula (II) of the present disclosure has the following structure of formula (V): 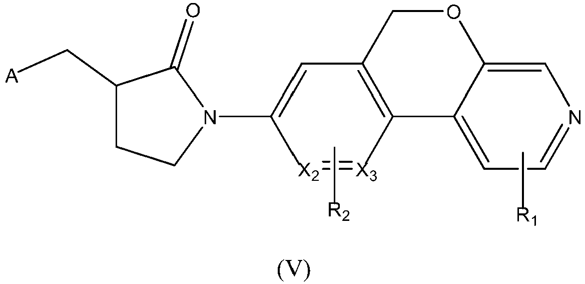 wherein
R₁, R₂, X₂, X₃, and A are as defined above.

In a preferred aspect, the compound of formula (III) of the present disclosure has the following structure of formula (VI): 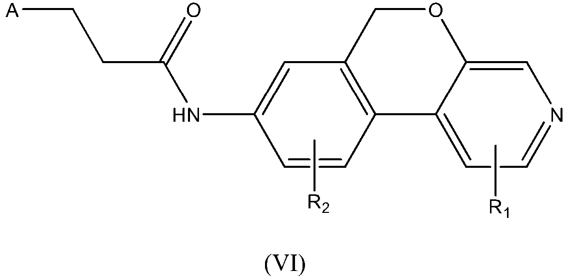 wherein
R₁, R₂, and A are as defined above.

In a preferred aspect, the compound of formula (IV) of the present disclosure has a structure of the following formula (VII): 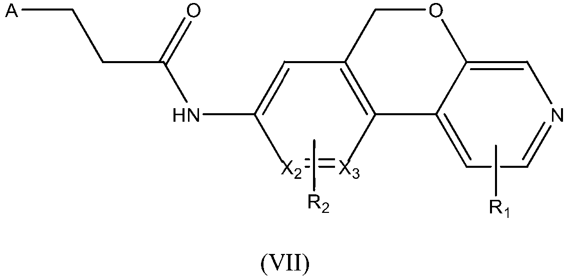 wherein
X₂ and X₃ are selected from the group consisting of CH and N, and X₂ and X₃ are not both CH;
R₁, R₂, and A are as defined above.

In some preferred embodiments, the compound described in the present disclosure is a compound selected from the group consisting of the following compounds, or a racemate, an enantiomer, a diastereomer, a deuterated compound, or a pharmaceutically acceptable salt thereof:

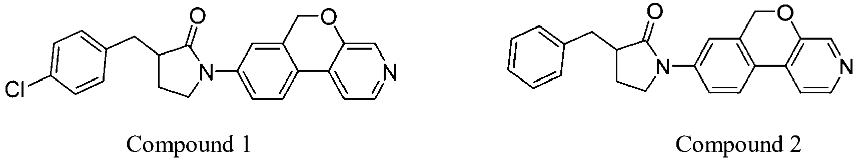

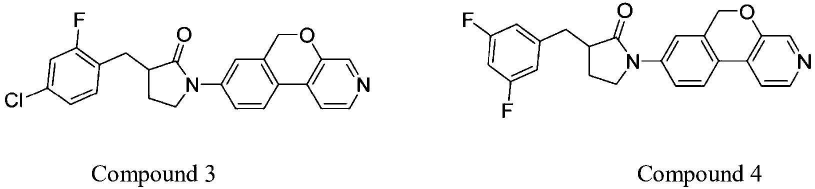

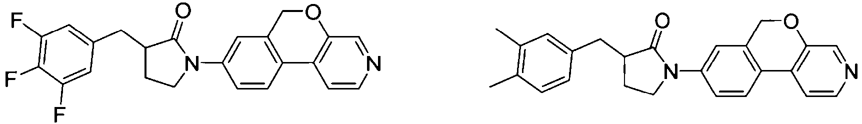

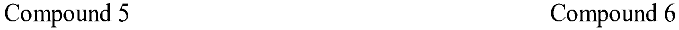

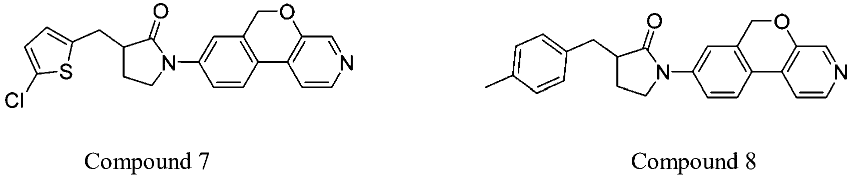

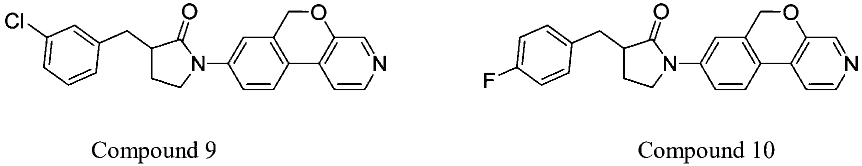

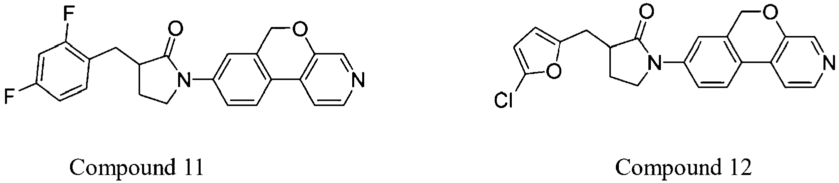

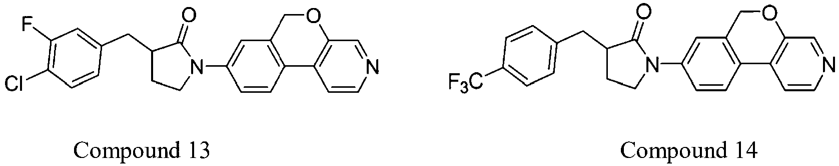

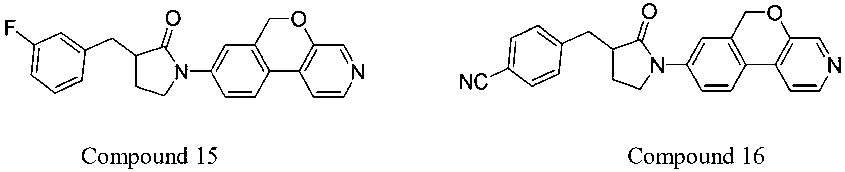

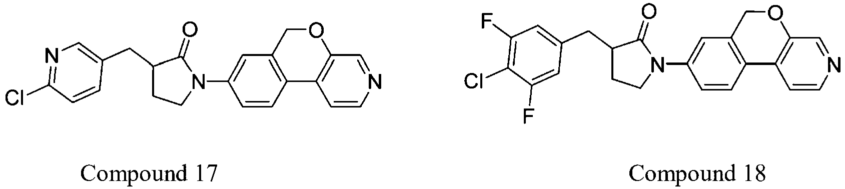

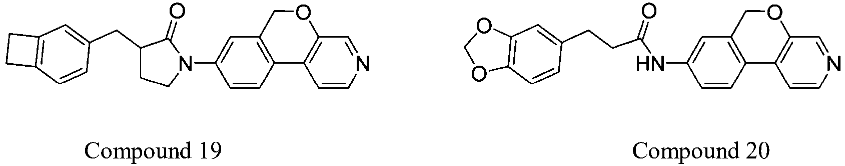

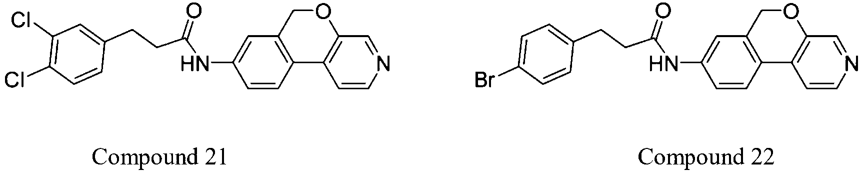

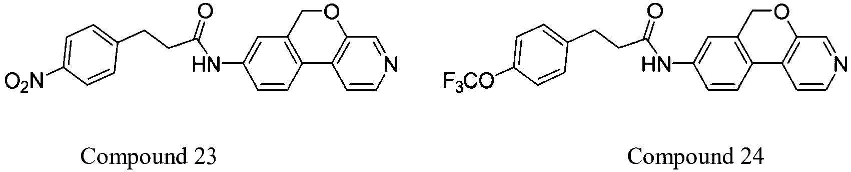

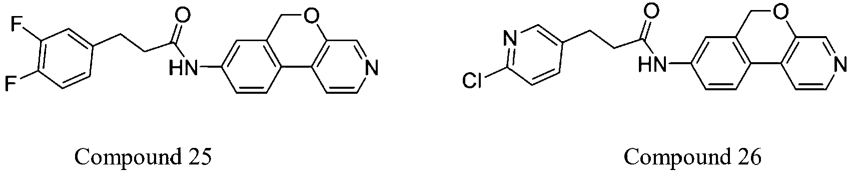

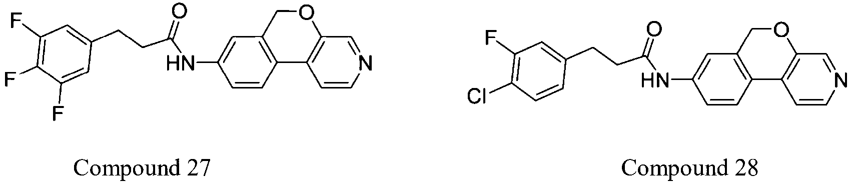

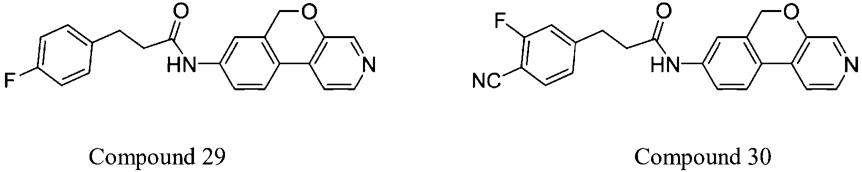

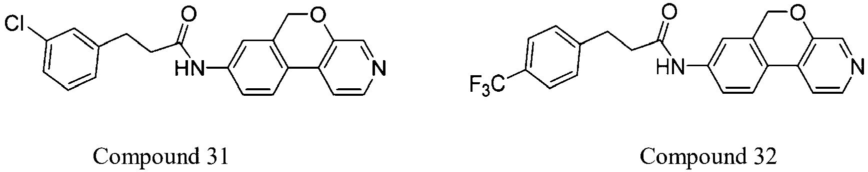

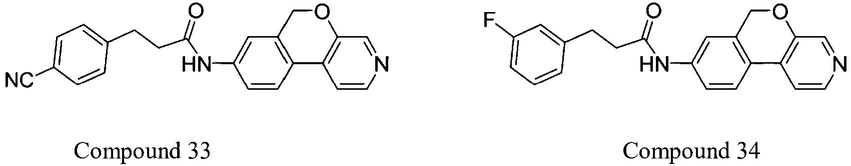

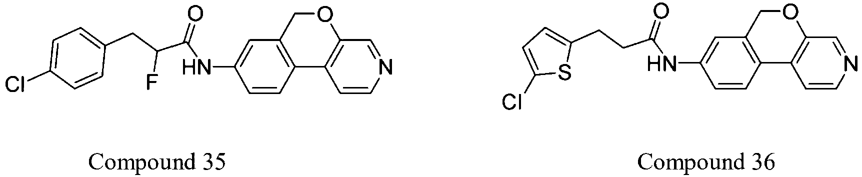

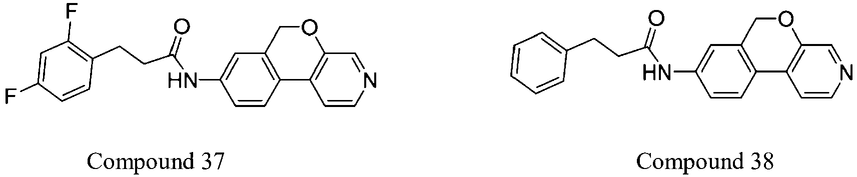

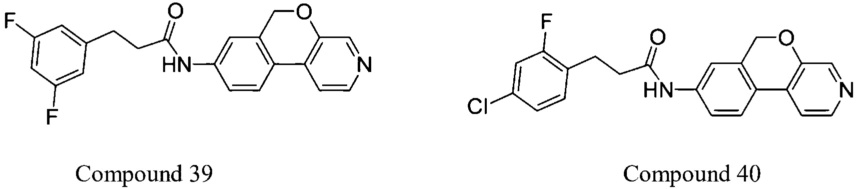

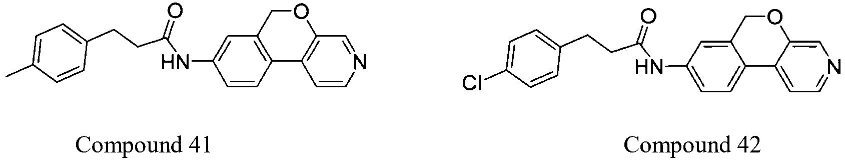

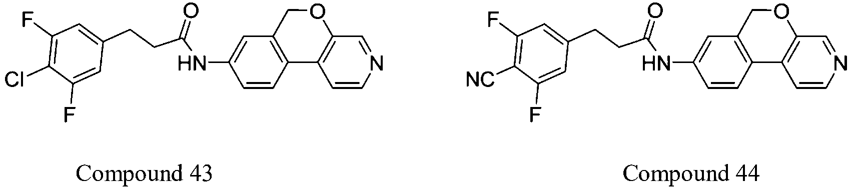

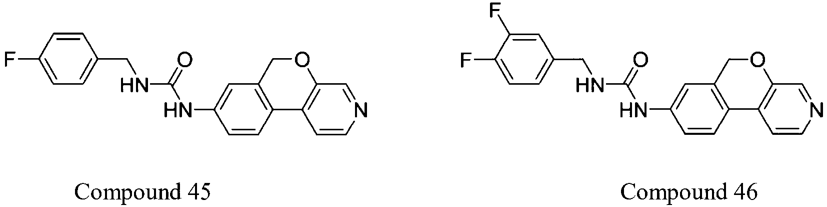

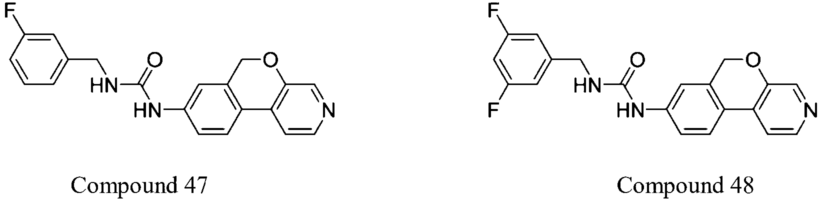

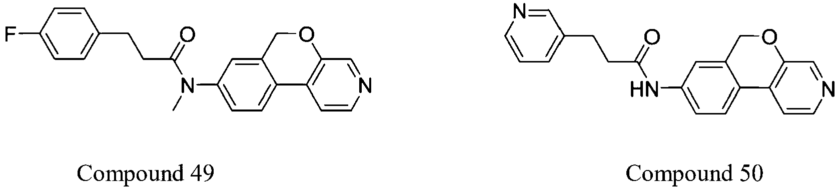

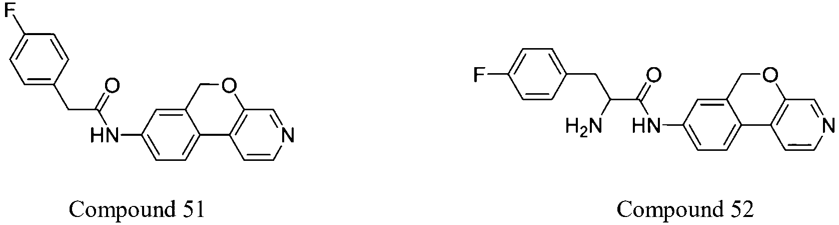

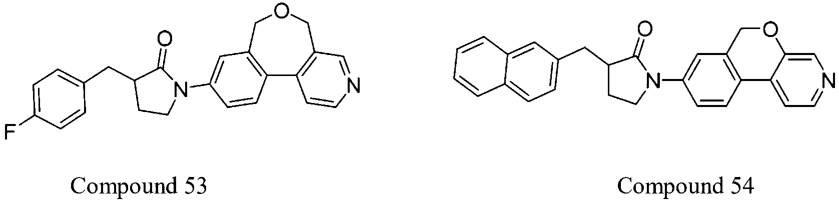

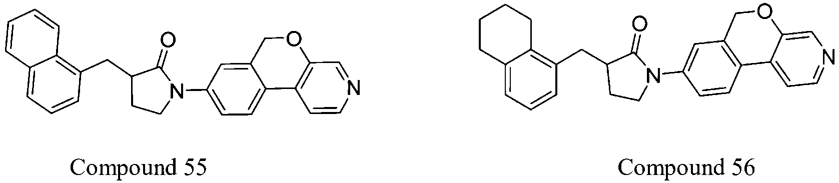

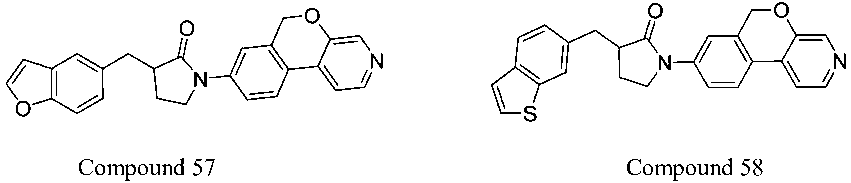

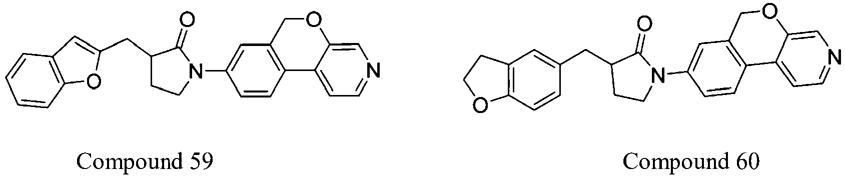

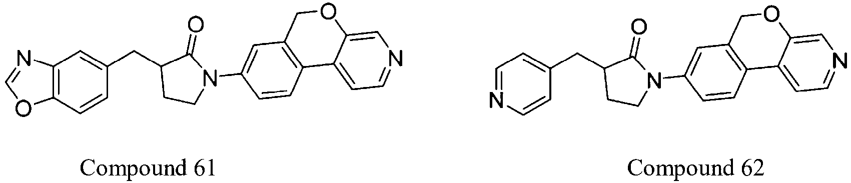

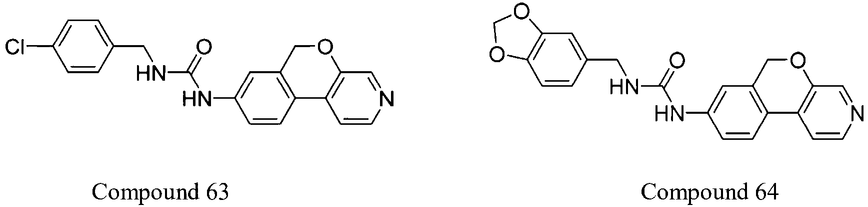

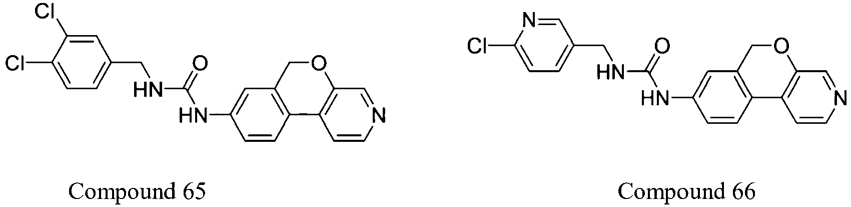

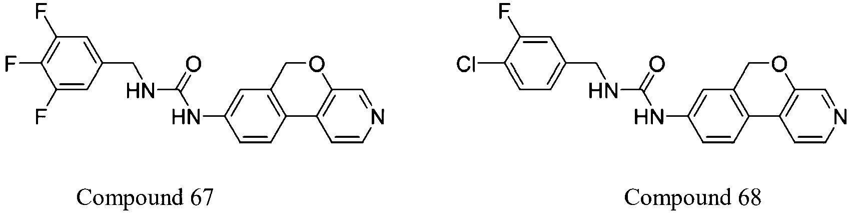

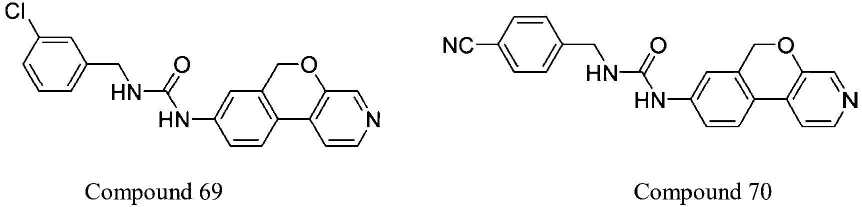

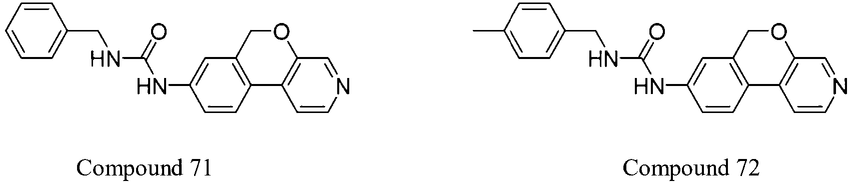

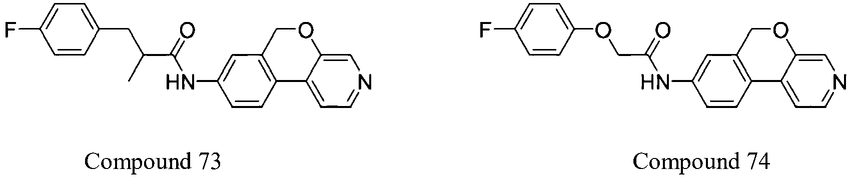

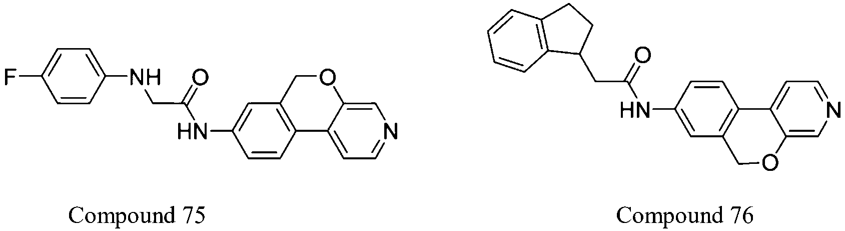

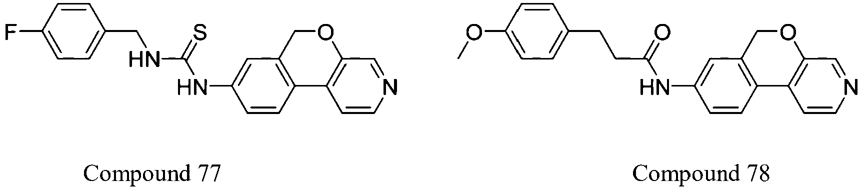

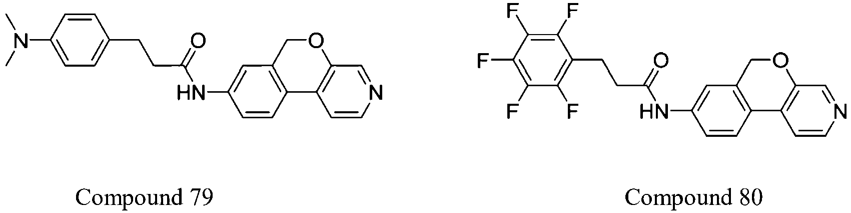

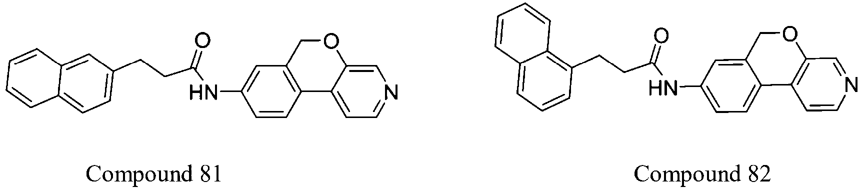

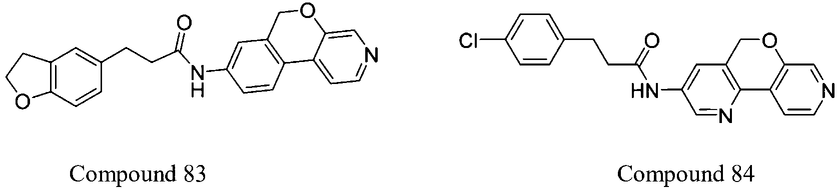

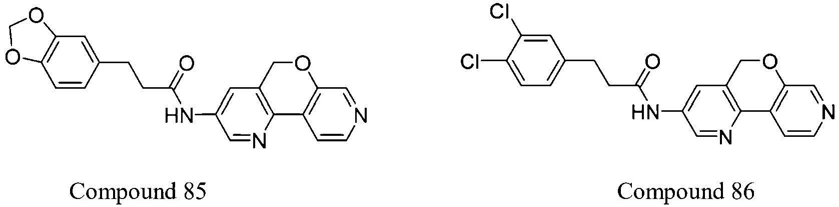

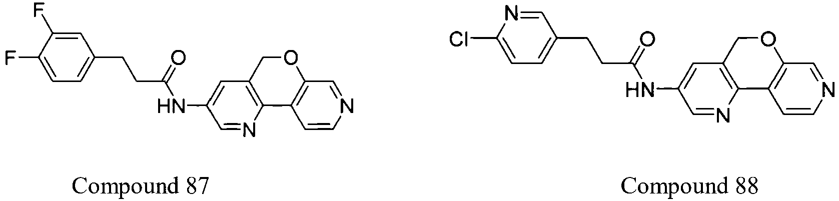

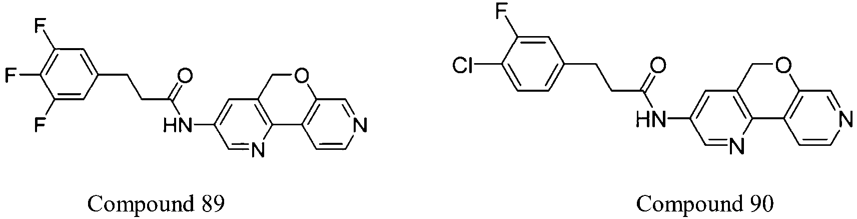

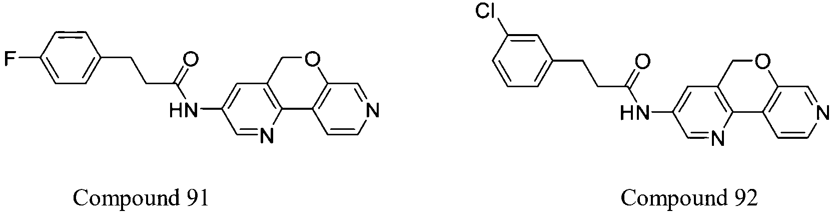

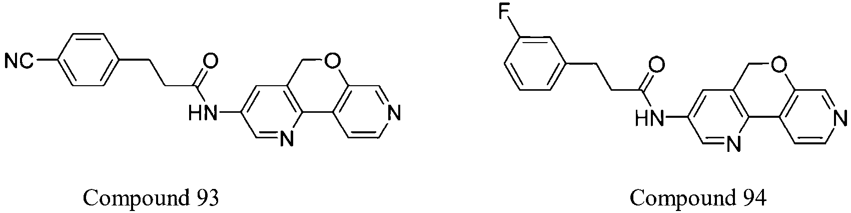

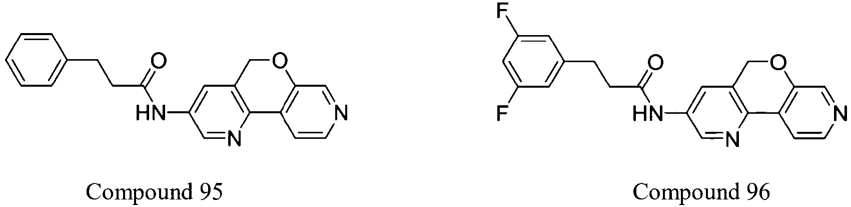

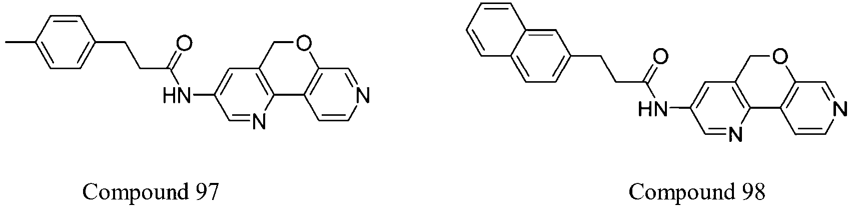

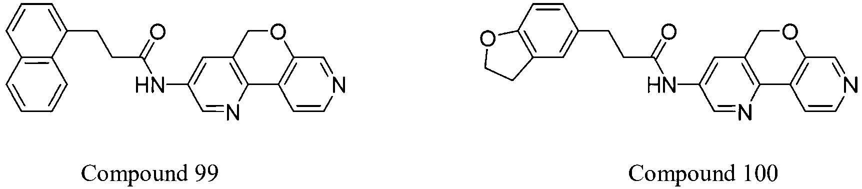

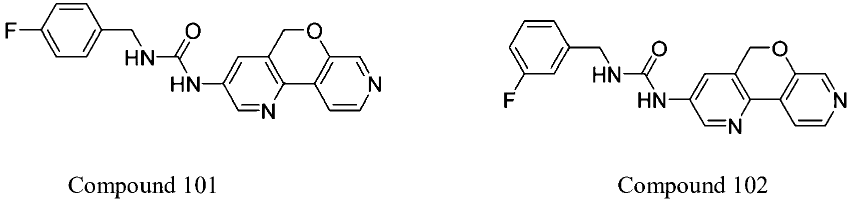

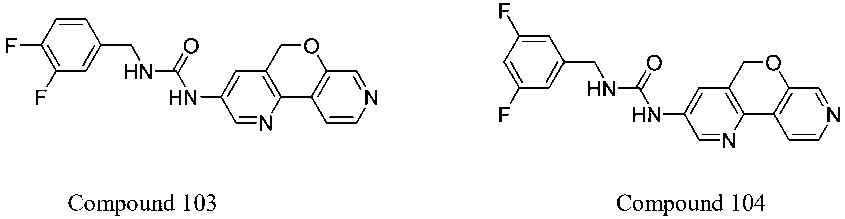

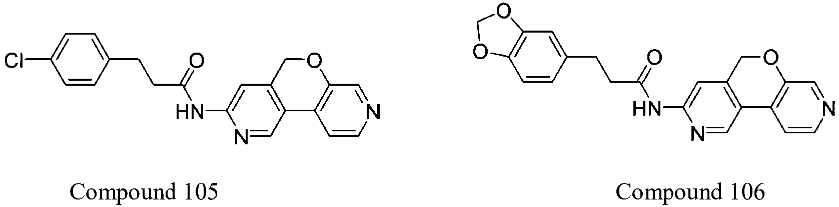

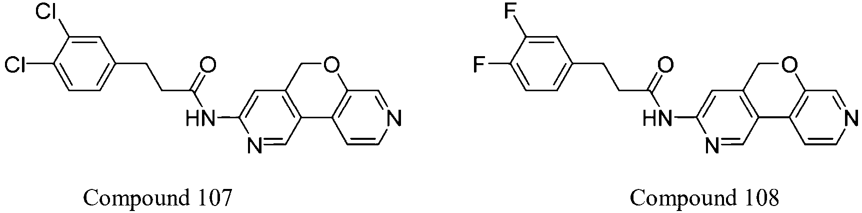

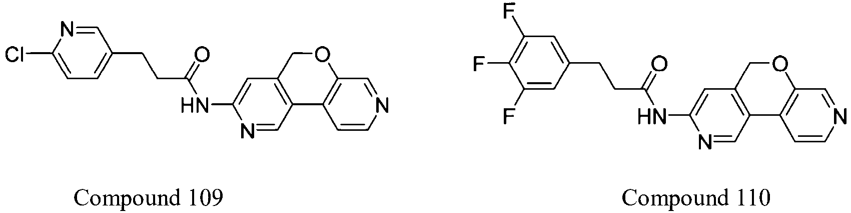

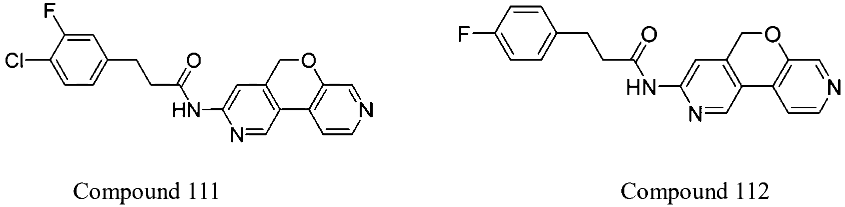

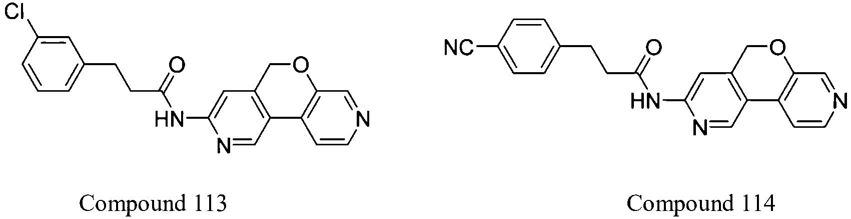

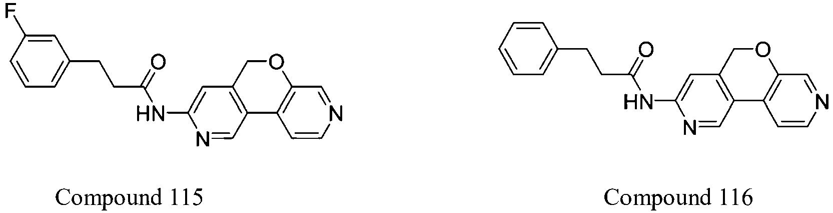

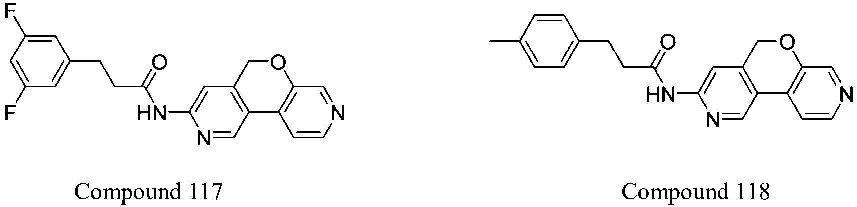

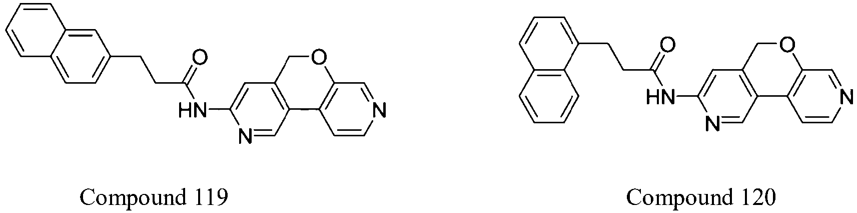

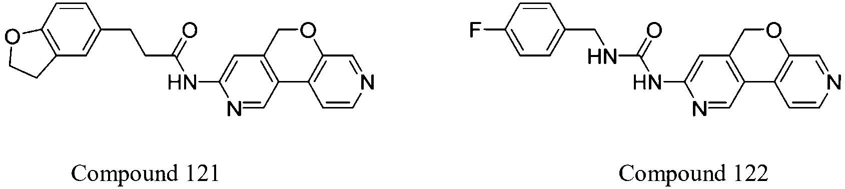

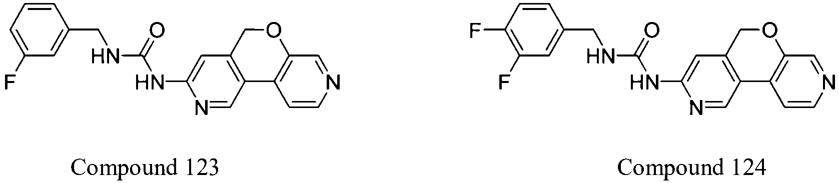

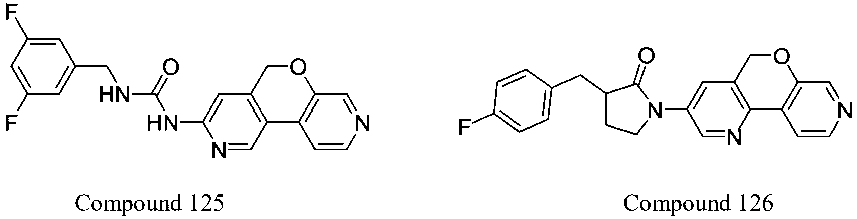

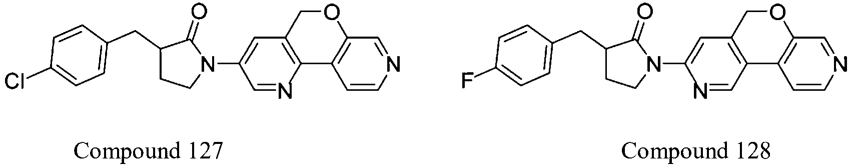

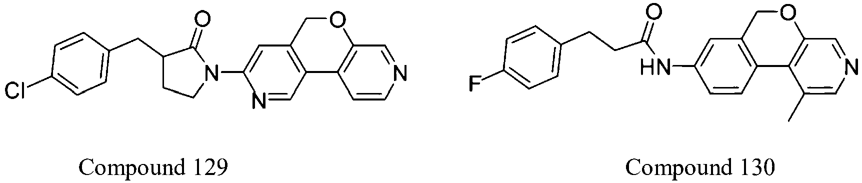

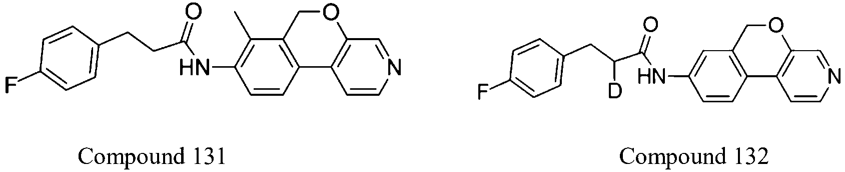

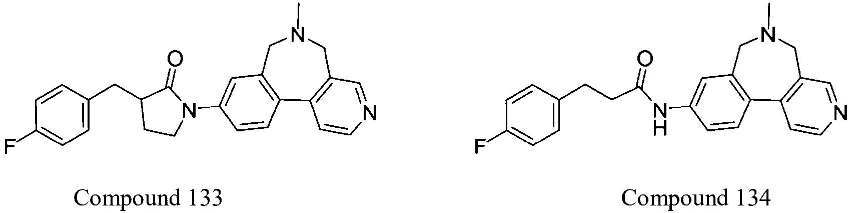

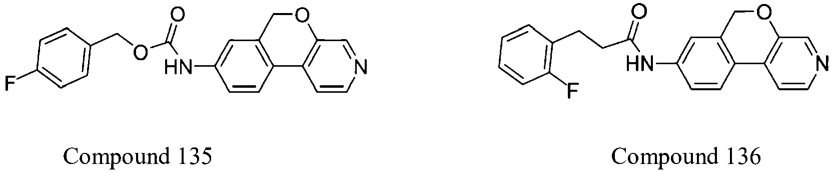

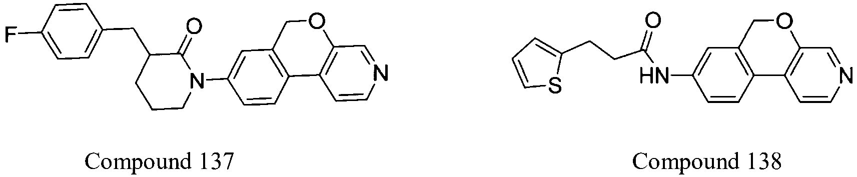

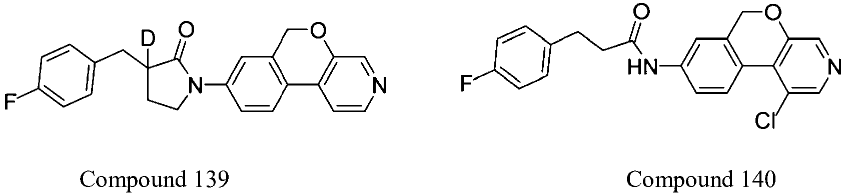

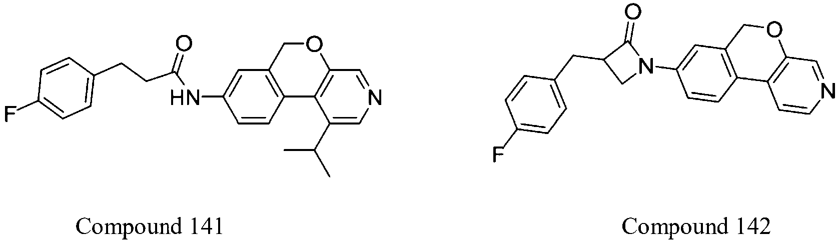

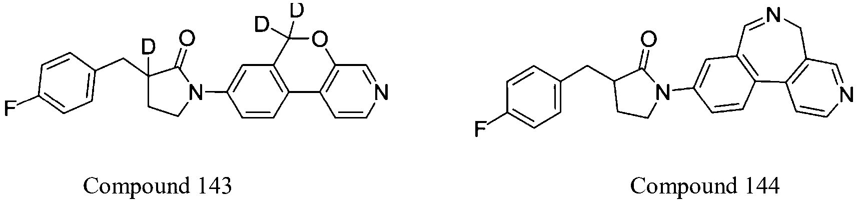

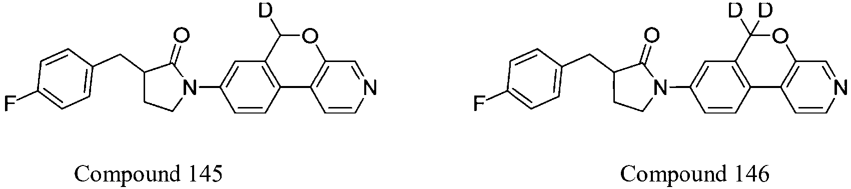

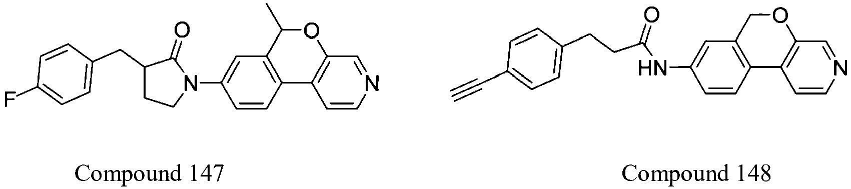

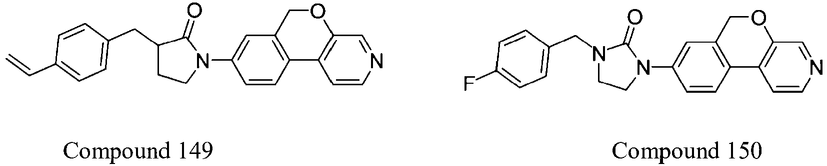

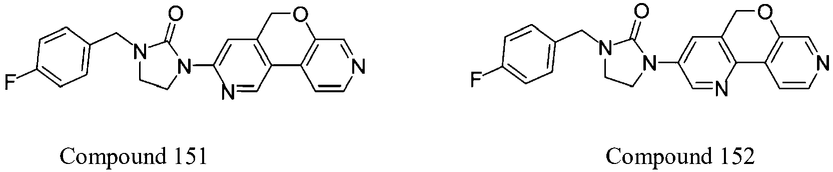

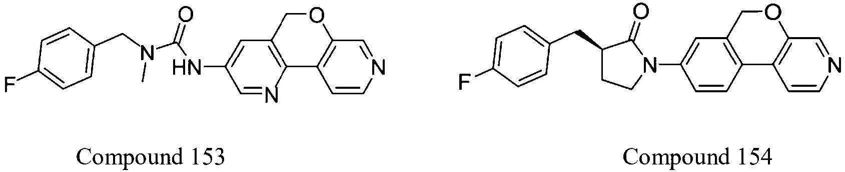

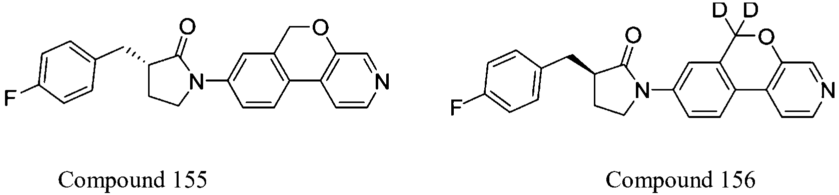

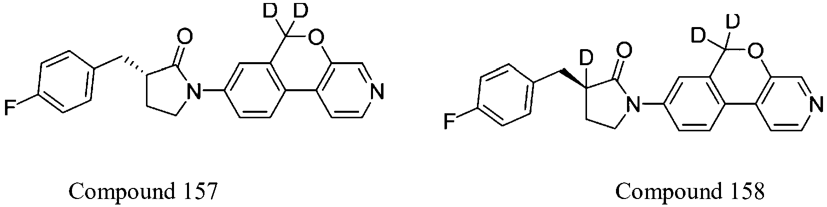

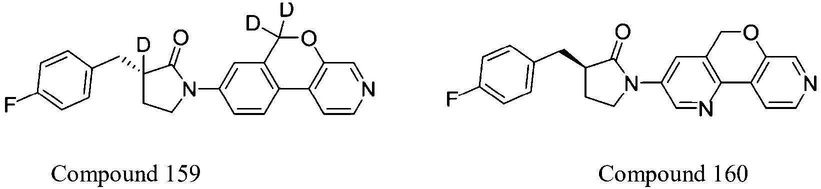

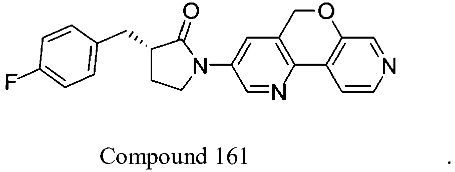

In the present disclosure, when referring to the compound of formula (I), it also includes a pharmaceutically acceptable salt of the compound of formula (I), or a stereoisomer thereof (including an enantiomer, a diastereomer, or a racemate thereof), or a tautomer thereof, or a deuterated compound thereof:
In another aspect, the present disclosure provides a use of the SARM1 enzyme activity inhibitor described in the present disclosure in the manufacture of a SARM1 enzyme activity inhibitor.

In another aspect, the present disclosure provides a use of the SARM1 enzyme activity inhibitor described in the present disclosure in the manufacture of a medicament for treating or preventing a disease or disorder associated with axonal degeneration.

In another aspect, the present disclosure provides a use of the SARM1 enzyme activity inhibitor described in the present disclosure in the manufacture of a medicament for treating or preventing a neurodegenerative disease or a neurological disease or disorder.

Accordingly, the present disclosure also relates to a method for treating or preventing a neurodegenerative disease or a neurological disease or disorder associated with neurodegeneration, comprising administering to a subject in need thereof the compound of the present disclosure as a SARM1 enzyme activity inhibitor. In particular, the present disclosure relates to a method for treating or preventing a disease or disorder associated with axonal degeneration, comprising administering to a subject in need thereof the compound of the present disclosure as a SARM1 enzyme activity inhibitor. More particularly, the present disclosure relates to a method for inhibiting SARM1 enzyme activity, comprising administering to a subject in need thereof the compound of the present disclosure; more particularly, the present disclosure relates to a method for inhibiting axonal degeneration, comprising administering to a subject in need thereof the compound of the present disclosure. The compound or composition of the present disclosure may be administered to a subject or patient in need thereof in an effective amount.

Accordingly, the present disclosure also relates to a use of the compound or a pharmaceutically acceptable salt or stereoisomer thereof described in the present disclosure in the manufacture of a medicament for treating or preventing a neurodegenerative disease or a neurological disease or disorder. The present disclosure also relates to a use of the compound or a pharmaceutically acceptable salt or stereoisomer thereof described in the present disclosure in the manufacture of a SARM1 enzyme activity inhibitor. The present disclosure also relates to a use of the compound or a pharmaceutically acceptable salt or stereoisomer thereof described in the present disclosure in the manufacture of a medicament for treating or preventing a disease or disorder associated with axonal degeneration. Preferably, the neurodegenerative disease or the neurological disease or disorder or the disease or disorder associated with axonal degeneration is selected from the group consisting of Alzheimer's disease, Parkinson's disease, multiple sclerosis, amyotrophic lateral sclerosis, and peripheral neuropathy.

### DETAILED DESCRIPTION

### Terminology

In the present disclosure, when referring to a "compound" having a specific structural formula, pharmaceutically acceptable salts, stereoisomers, diastereomers, enantiomers, racemic mixtures, and isotopic derivatives thereof are also generally encompassed.

The pharmaceutically acceptable salts described in the present disclosure may be formed using, for example, the following inorganic acids or organic acids: "pharmaceutically acceptable salt" refers to a salt that, within the scope of reasonable medical judgment, is suitable for contact with the tissues of humans and mammals without undue toxicity, irritation, allergic reactions, and the like, commensurate with a reasonable benefit/risk ratio. Such salts may be prepared in situ during the final isolation and purification of the compounds of the present disclosure, or may be prepared separately by reacting a free base or a free acid with a suitable reagent. For example, a free base may react with a suitable acid. Examples of pharmaceutically acceptable acid addition salts are salts formed by amino groups with inorganic acids (e.g., hydrochloric acid, hydrobromic acid, phosphoric acid, sulfuric acid, and perchloric acid) or organic acids (e.g., acetic acid, oxalic acid, maleic acid, tartaric acid, citric acid, succinic acid, or malonic acid), or salts formed by using other methods in the prior art such as ion exchange. Other pharmaceutically acceptable salts include sodium alginate, ascorbate, benzenesulfonate, adipate, camphorsulfonate, aspartate, benzoate, bisulfate, borate, butyrate, camphorate, citrate, dodecyl sulfate, ethanesulfonate, formate, fumarate, glucoheptonate, glycerophosphate, gluconate, heptanoate, hexanoate, hydroiodide, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate, and the like.

The pharmaceutically acceptable salts of the present disclosure may be prepared by conventional methods, for example, by dissolving the compound of the present disclosure in a water-miscible organic solvent (e.g., methanol, ethanol, acetone, and acetonitrile), adding an excess of an aqueous solution of an organic acid or an inorganic acid thereto, so that the salt precipitates from the resulting mixture, removing the solvent and the remaining free acid therefrom, and then isolating the precipitated salt.

The "stereoisomerism" described in the present disclosure is divided into conformational isomerism and configurational isomerism; configurational isomerism may further be divided into cis-trans isomerism and optical isomerism. Conformational isomerism refers to a stereoisomeric phenomenon in which an organic molecule having a certain configuration produces different spatial arrangements of various atoms or atomic groups of the molecule due to rotation or twisting of carbon-carbon single bonds. Common examples include structures of alkane and cycloalkane compounds, such as the chair conformation and boat conformation appearing in cyclohexane structures. "Stereoisomer" refers to when the compound of the present disclosure contains one or more asymmetric centers, and thus may exist as racemates and racemic mixtures, single enantiomers, diastereomeric mixtures, and single diastereomers. The compounds of the present disclosure have asymmetric centers, each asymmetric center will produce two optical isomers, and the scope of the present disclosure includes all possible optical isomers and diastereomeric mixtures, and pure or partially pure compounds.

In some cases, the compounds described in the present disclosure may exist in tautomeric forms, which have different points of hydrogen attachment through displacement of one or more double bonds. For example, a ketone and its enol form are keto-enol tautomers. Amides and imines may also form tautomeric forms. Each tautomer and mixtures thereof are included in the compounds of the present disclosure. Enantiomers, diastereomers, racemates, mesoforms, cis-trans isomers, tautomers, and mixtures thereof of all compounds are included within the scope of the present disclosure.

The compounds of the present disclosure may be used in combination with additional SARM1 enzyme activity inhibitors for treating or preventing neurodegenerative diseases or related neurological diseases or disorders, or may be used in combination with additional active medicaments for treating or preventing neurodegenerative diseases or related neurological diseases or disorders, for treating or preventing neurodegenerative diseases or related diseases or disorders.

The compounds of the present disclosure or mesoforms, racemates, enantiomers, diastereomers, or pharmaceutically acceptable salts thereof may be administered as active ingredients orally or parenterally, in an effective amount ranging from 0.1 to 2000 mg/kg body weight/day, preferably 0.1 to 100 mg/kg body weight/day, in the case of mammals including humans (body weight approximately 70 kg), and administered daily in single or divided doses, or following or not following a predetermined time. The dosage of the active ingredient may be adjusted according to several relevant factors (e.g., the condition of the subject to be treated, the type and severity of the disease, the rate of administration, and the physician's opinion). In some cases, an amount less than the above dose may be appropriate.

The compounds of the present disclosure or mesomers, racemates, enantiomers, diastereomers, or pharmaceutically acceptable salts thereof may be formulated in the form of pharmaceutical compositions. The pharmaceutical compositions may comprise the compound of the present disclosure or a mesoform, racemate, enantiomer, diastereomer, or pharmaceutically acceptable salt thereof, and a pharmaceutically acceptable carrier.

The pharmaceutical compositions of the present disclosure may be formulated into dosage forms for oral administration or parenteral administration (including intramuscular, intravenous, subcutaneous routes, and intratumoral injection) according to any of conventional methods, for example, tablets, granules, powders, capsules, syrups, emulsions, microemulsions, solutions, or suspensions.

The pharmaceutical compositions of the present disclosure for oral administration may be prepared by mixing the active ingredient with, for example, the following pharmaceutically acceptable carriers: cellulose, calcium silicate, magnesium stearate, calcium stearate, corn starch, lactose, sucrose, dextrose, calcium phosphate, stearic acid, surfactants, suspending agents, gelatin, talc, emulsifiers, and diluents. Examples of carriers employed in the injection compositions of the present disclosure are water, glycerides, saline solutions, alcohols, glycols, glucose solutions, ethers (e.g., polyethylene glycol 400), oils, fatty acids, fatty acid esters, surfactants, suspending agents, and emulsifiers.

Unless otherwise indicated, conventional methods of mass spectrometry, NMR, HPLC, protein chemistry, biochemistry, recombinant DNA technology, and pharmacology are used. In the present application, unless otherwise indicated, "or" or "and" may be used to mean "and/or".

In the specification and claims, a given chemical formula or name shall encompass all stereoisomers and optical isomers and racemates in which the aforementioned isomers exist. Unless otherwise indicated, all chiral (enantiomeric and diastereomeric) and racemic forms are within the scope of the present disclosure. Many geometric isomers of C=C double bonds, C=N double bonds, ring systems, and the like may exist in the compounds, and all such stable isomers are encompassed within the present disclosure.

The compounds of the present disclosure may be isolated in optically active or racemic form. All methods for preparing the compounds of the present disclosure and intermediates prepared therein are considered to be part of the present disclosure. When preparing enantiomeric or diastereomeric products, they may be separated by conventional methods (e.g., by chromatography or fractional crystallization). It should be understood that all isomeric forms that may be present are included within the present disclosure.

Unless otherwise defined, when a substituent is designated as "optionally substituted," the substituent is selected from, for example, the following substituents, such as C₁-C₃ alkyl, C₃-C₆ cycloalkyl, C₅-C₁₀ aryl, 3-10 membered heterocyclyl, halogen (including F, Cl, Br), hydroxy, C₁-C₃ alkoxy, nitro, cyano, oxo (=O), C₁-C₃ alkylacyl, C₃-C₁₀ aryloxy, C₁-C₃ alkylacyloxy, amino, C₁-C₃ alkylamino, C₅-C₁₀ arylamino, C₁-C₃ alkylthio, and the like.

The term "alkyl" or "alkylene" as used in the present disclosure is intended to include branched and straight-chain saturated aliphatic hydrocarbon groups having a specified number of carbon atoms. The alkyl in the present disclosure is preferably C₁-C₁₂ alkyl, C₁-C₁₀ alkyl, or C₁-C₈ alkyl, more preferably C₁-C₆ alkyl, particularly preferably C₁-C₄ alkyl, and especially C₁-C₃ alkyl. For example, "C₁-C₆ alkyl" denotes an alkyl having 1 to 6 carbon atoms. Examples of alkyl include, but are not limited to, methyl (Me), ethyl (Et), propyl (e.g., n-propyl and isopropyl), butyl (e.g., n-butyl, isobutyl, tert-butyl), and pentyl (e.g., n-pentyl, isopentyl, neopentyl). For the C₁-C₁₂ alkyl in the present disclosure, 1 to 4 -CH₂- units therein are optionally replaced by an O atom, an S atom, or -NH-.

The term "carbonyl" refers to an organic functional group (C=O or C(O)) formed by two atoms, carbon and oxygen, connected by a double bond.

The term "aromatic ring" or "aryl" means a 5-10 membered aromatic ring structure. An aromatic carbocycle refers to an aromatic carbocyclic structure in which all ring members are composed of carbon atoms. A non-aromatic carbocycle refers to a non-aromatic carbocyclic structure in which all ring members are composed of carbon atoms.

The term "heteroaryl" as used in the present disclosure refers to a 5-10 membered aromatic group comprising 1, 2, or 3 heteroatoms selected from O, N, and S.

"Halo" or "halogen" includes fluorine, chlorine, bromine, and iodine; preferably fluorine and chlorine.

The term "heterocycle" refers to a monocyclic heterocycloalkyl system or a bicyclic heterocycloalkyl system, and may also include spiro heterocycles or bridged heterocycloalkyls. A monocyclic heterocycloalkyl refers to a 3-8 membered, preferably 3-6 membered, saturated or unsaturated but non-aromatic cyclic alkyl system containing at least one heteroatom selected from O, N, S, and P (preferably containing 1, 2, or 3 heteroatoms selected from O, N, and S). A bicyclic heterocycloalkyl system refers to a heterocycloalkyl fused with a phenyl, a cycloalkyl, a cycloalkenyl, a heterocycloalkyl, or a heteroaryl. An aromatic heterocycle refers to an aromatic heterocyclic system, and a non-aromatic heterocycle refers to a non-aromatic heterocyclic system.

When any variable occurs more than once in any composition or formula of a compound, its definition at each occurrence is independent of its definition at every other occurrence. Thus, for example, if a group is shown to be substituted with 0-3 R groups, then the group may optionally be substituted with up to three R groups, and at each occurrence R is independently selected from the definition of R. Furthermore, combinations of substituents and/or variables are permitted only if such combinations result in stable compounds.

The term "effective amount" as used in the present disclosure refers to an amount of a drug or an agent (i.e., the compound of the present disclosure) that will elicit a biological or medical response in a tissue, system, animal, or human, for example, as sought by a researcher or clinician. Furthermore, the term "therapeutically effective amount" refers to an amount that, compared to a corresponding subject who does not receive such amount, results in improved treatment, cure, prevention, or alleviation of a disease, disorder, or side effect, or a reduction in the rate of progression of a disease or disorder. An effective amount may be administered in one or more administrations, applications, or doses and is not intended to be limited to a particular formulation or route of administration. The term also includes within its scope an effective amount that enhances normal physiological function.

The term "treatment" as used in the present disclosure includes any effect that results in an improvement of a condition, disease, disorder, or the like, for example, alleviation, reduction, modulation, amelioration, or elimination, or improvement of symptoms thereof.

The term "pharmaceutical" as used in the present disclosure refers to those compounds, substances, compositions, and/or dosage forms that, within the scope of reasonable medical judgment, are suitable for contact with the tissues of humans and animals without excessive toxicity, irritation, allergic reactions, and/or other problems or complications, and commensurate with a reasonable benefit/risk ratio.

The phrase "pharmaceutically acceptable carrier" as used in the present disclosure refers to a pharmaceutical substance, composition, or vehicle, such as a liquid or solid filler, diluent, excipient, manufacturing aid (e.g., lubricant, talc, magnesium stearate, calcium stearate, or zinc stearate, or stearic acid), or solvent encapsulating material, involved in carrying or transporting the subject compound from one organ or portion of the body to another organ or portion of the body. Each carrier must be "acceptable" in the sense of being compatible with the other components of the formulation and not harmful to the patient.

The term "pharmaceutical composition" refers to a composition comprising the compound of the present disclosure and at least one other pharmaceutically acceptable carrier. "Pharmaceutically acceptable carrier" refers to a medium generally accepted in the art for delivering biologically active agents to animals (specifically mammals), including (i.e.) adjuvants, excipients, or vehicles, such as diluents, preservatives, fillers, flow regulating agents, disintegrants, wetting agents, emulsifying agents, suspending agents, sweetening agents, flavoring agents, aromatic agents, antibacterial agents, antifungal agents, lubricants, and dispersing agents, depending on the mode of administration and the nature of the dosage form.

The compound or pharmaceutical composition of the present disclosure, after administration, may ameliorate a disease, symptom, or condition, in particular ameliorate the severity thereof, delay onset, slow disease progression, or reduce the duration of the disease. Whether fixed administration or temporary administration, continuous administration or intermittent administration, conditions that can be attributed to are associated with the administration.

### Route of Administration

Suitable routes of administration for the compound or pharmaceutical composition of the present disclosure include but are not limited to oral, intravenous, rectal, aerosol, parenteral, ocular, pulmonary, transdermal, vaginal, otic, nasal, and topical administration. In addition, by way of example only, parenteral administration includes intramuscular injection, subcutaneous injection, intravenous injection, intramedullary injection, intraventricular injection, intraperitoneal injection, intralymphatic injection, and intranasal injection.

In some embodiments, the compound of the present disclosure is administered topically. In other specific embodiments, the compound of the present disclosure may be administered by implantation (e.g., subcutaneously or intramuscularly) or by intramuscular injection. Furthermore, in other specific embodiments, the compound of the present disclosure is administered via a targeted delivery system.

In the pharmaceutical composition of the present disclosure, a pharmaceutical carrier may be formulated according to various factors within the knowledge of a person skilled in the art. These factors include, but are not limited to: the type and nature of the active agent being formulated; the subject to whom the composition containing the active agent is to be administered; the intended route of administration of the composition; and the therapeutic indication being targeted. Pharmaceutical carriers include aqueous and non-aqueous liquid media and various solid and semi-solid dosage forms.

The above-mentioned carriers may include various different ingredients and additives other than the active agent, and such other components are included in the formulation for various reasons well known to those skilled in the art, such as stabilizing the active agent, binders, and the like. Descriptions of suitable pharmaceutical carriers and factors involved in carrier selection can be found in various readily available sources, such as Allen L.V. Jr. et al., Remington: The Science and Practice of Pharmacy (2 Volumes), 22nd Edition (2012), Pharmaceutical Press.

The compound is typically administered in the form of a mixture with suitable pharmaceutical diluents, excipients, or carriers (collectively referred to in the present disclosure as pharmaceutical carriers) appropriately selected according to the intended dosage form (e.g., oral tablets, capsules, elixirs, and syrups) and in conformity with conventional pharmaceutical practice.

Although the compound of the present disclosure may be administered alone, it is preferable to administer the compound in the form of a pharmaceutical formulation (composition).

### Kits/Product Packages

For use in the treatment of the above-mentioned indications, kits/product packages are also described herein. These kits may consist of dispensers, pouches, or container boxes, which may be divided into compartments to accommodate one or more containers, such as vials, test tubes, and the like, each container containing a single ingredient in the method. Suitable containers include bottles, vials, syringes, and test tubes. The containers are made of acceptable materials such as glass or plastic.

For example, the container may hold one or more compounds described herein, and the compounds may exist in the form of a pharmaceutical ingredient or as a mixture with other components described herein. The container may have a sterile outlet (e.g., the container may be an intravenous infusion bag or bottle, the stopper of which may be pierced by a hypodermic needle). Such a kit may contain a compound and an instruction, a label, or an operating instruction for the methods of use described herein.

A typical kit may include one or more containers, each containing one or more materials (such as reagents, which may also be a concentrated stock solution, and/or an instrument) to meet commercial promotion needs and user requirements for compound use. These materials include but are not limited to buffers, diluents, filters, needles, syringes, dispensers, bags, containers, bottles, and/or test tubes, with a contents list and/or instruction for use, and internal packaging also with an instruction. A complete set of instructions is included.

The above-mentioned features in the present disclosure, or the features mentioned in the examples, may be combined in any manner. All features disclosed in the specification of this case may be used in combination with any composition form, and each feature disclosed in the specification may be replaced by any alternative feature that can provide the same, equivalent, or similar purpose. Therefore, unless specifically stated, the disclosed features are merely general examples of equivalent or similar features.

The present disclosure is further illustrated below in conjunction with specific examples. It should be understood that these examples are only intended to illustrate the present disclosure and are not intended to limit the scope of the present disclosure. In the following examples, experimental methods without specific conditions indicated are generally carried out according to conventional conditions or according to conditions suggested by manufacturers. Unless otherwise stated, all percentages, ratios, proportions, or parts are by weight.

The units of weight/volume percentage in the present disclosure are well known to those skilled in the art, for example, referring to the weight of solute in 100 mL of solution. Unless otherwise defined, all technical and scientific terms used in the present disclosure have the same meaning as commonly understood by one of ordinary skill in the art. Furthermore, any methods and materials similar or equivalent to those described in the present disclosure can be applied to the methods of the present disclosure. The preferred embodiments and materials described in the present disclosure are for illustrative purposes only.

In the terminology used in the present disclosure, "neurodegenerative disease" and "neurodegenerative disorder" have the same meaning, and "axonal degeneration" and "axonal mutation" have the same meaning. Those skilled in the art can understand that the terms have their commonly understood meanings.

The description including the examples in the present disclosure is not intended to limit the scope of any claim. The following non-limiting examples are provided to further illustrate the present disclosure. Based on the present disclosure, those skilled in the art will understand that many changes can be made to the specific embodiments disclosed without departing from the spirit and scope of the present teachings, and still obtain the same or similar results.

Unless otherwise stated, all materials/reagents were obtained from commercial suppliers and used without further purification. During the experiments, reactions were monitored by LC-MS and/or thin layer chromatography (TLC) on silica gel 60 F254 (0.2 mm) pre-coated glass backing and observed using UV light. The structures of the compounds in the following examples were characterized by nuclear magnetic resonance (NMR) and/or mass spectrometry (MS).

¹H NMR (400 MHz) spectra were recorded on a Bruker spectrometer at room temperature, with TMS or residual solvent peaks as internal standards. Chemical shift values or peak multiplicities are given in (δ), and coupling constants (J) are given as absolute values in Hertz (Hz). Multiplicity abbreviations in ¹H NMR spectra are as follows: s (singlet), d (doublet), t (triplet), q (quartet), m (multiplet), br or broad peak (broadened).

Preparative HPLC purification was performed on a Shimadzu LC-6AD. All purification work was completed using a Shim-pack PREP-DDS(H)KIT column. The mobile phase was water (containing 0.1% HCO₂H) and acetonitrile; all reagents used were HPLC grade. The flow rate was 10 mL/min.

LC-MS was performed on an Agilent 1260 infinity II; mobile phase: A: water (0.1% trifluoroacetic acid), B: ACN; 3.5 min column running; column: YMC-Triart C18 50 * 3 mm, 3 um; flow rate: 1.8 mL/min; oven temperature: 40°C; gradient: 5-100 (ACN%).

Preparative TLC was performed on Whatman LK6F Silica Gel 60A plates with dimensions of 20 x 20 cm and a thickness of 500 um.

The following examples are intended to illustrate embodiments of the present disclosure and are not intended to limit the same in any way.

### Synthesis Route of Intermediate BB1

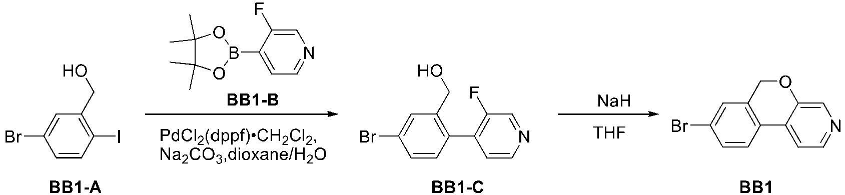

### Step 1: Intermediate BB1-C (5-bromo-2-(3-fluoropyridin-4-yl)phenyl)methanol

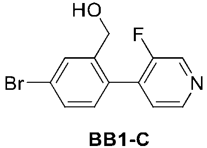

At room temperature, PdCl₂(dppf)•CH₂Cl₂ (1.3 g, 1.598 mmol) and sodium carbonate (4.23 g, 39.94 mmol) were added to a solution containing **intermediate BB1-A** (5-bromo-2-iodophenyl)methanol (5.0 g, 15.98 mmol) and **intermediate BB1-B** 3-fluoropyridin-4-ylboronic acid pinacol ester (3.56 g, 15.98 mmol) in 1,4-dioxane (100 mL) and water (10 mL). The reaction system was purged with nitrogen 3 times and heated to 80°C with stirring for 12 hours. TLC showed the reaction was complete. The reaction mixture was cooled to room temperature, filtered through celite, and concentrated under reduced pressure. The resulting crude product was purified by silica gel column chromatography (eluent: 50% ethyl acetate in petroleum ether) to obtain **intermediate BB1-C** (5-bromo-2-(3-fluoropyridin-4-yl)phenyl)methanol (1.71 g) as a brown oil.

LC_MS: (ES⁺): m/z 281.95 [M+H]⁺.

### Step 2: Intermediate BB1 8-bromo-6H-isochromeno[3,4-c]pyridine

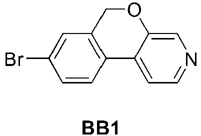

At 0°C, sodium hydride (484.9 mg, 12.123 mmol, 60% purity) was added to a solution containing **intermediate BB1-C** (5-bromo-2-(3-fluoropyridin-4-yl)phenyl)methanol (1.71 g, 6.061 mmol) in tetrahydrofuran (30 mL). The reaction mixture was stirred at room temperature for 12 hours. TLC showed the reaction was complete. The reaction mixture was slowly poured into a saturated ammonium chloride solution (30 mL), and extracted with ethyl acetate (30 mL x 2). The organic layers were combined, washed with saturated brine (30 mL), dried over anhydrous sodium sulfate, filtered, and concentrated under reduced pressure. The resulting crude product was purified by silica gel column chromatography (eluent: 33% to 50% ethyl acetate in petroleum ether) to obtain **intermediate BB1** 8-bromo-6H-isochromeno[3,4-c]pyridine (1.3 g) as a yellow solid.

LC_MS: (ES⁺): m/z 261.94 [M+H]⁺.

¹H NMR (400 MHz, CDCl₃) δ 8.38 (s, 1H), 8.33 (d, *J =* 5.1 Hz, 1H), 7.63 (d, *J =* 8.3 Hz, 1H), 7.60 - 7.52 (m, 2H), 7.38 (d, *J =* 1.4 Hz, 1H), 5.19 (s, 2H).

### Synthesis Route of Intermediate BB2

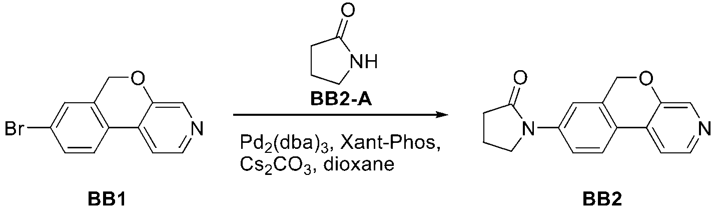

### Step 1: Intermediate BB2 1-(6H-isochromeno[3,4-c]pyridin-8-yl)pyrrolidin-2-one

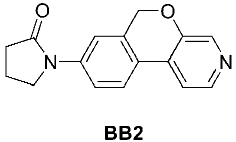

At room temperature, Pd₂(dba)₃ (225.4 mg, 0.246 mmol), Xant-Phos (142.3 mg, 0.246 mmol), and cesium carbonate (3.2 g, 9.844 mmol) were added to a solution containing **intermediate BB1** 8-bromo-6H-isochromeno[3,4-c]pyridine (1.3 g, 4.922 mmol) and **intermediate BB2-A** pyrrolidin-2-one (418.9 mg, 4.922 mmol) in 1,4-dioxane (20 mL). The reaction system was purged with nitrogen 3 times and heated to 100°C with stirring for 2 hours. TLC showed the reaction was complete. The reaction mixture was cooled to room temperature, filtered through celite, and concentrated under reduced pressure. The resulting crude product was purified by silica gel column chromatography (eluent: 7.2% methanol in dichloromethane) to obtain **intermediate BB2** 1-(6H-isochromeno[3,4-c]pyridin-8-yl)pyrrolidin-2-one (1.14 g) as a yellow solid.

LC_MS: (ES⁺): m/z 267.10 [M+H]⁺.

¹H NMR (400 MHz, CDCl₃) δ 8.35 (s, 1H), 8.29 (d, *J =* 5.0 Hz, 1H), 7.74 (d, *J* = 8.5 Hz, 1H), 7.69 (s, 1H), 7.58 (d, *J =* 8.4 Hz, 1H), 7.53 (d, *J =* 5.0 Hz, 1H), 5.22 (s, 2H), 3.92 (t, *J =* 7.0 Hz, 2H), 2.67 (t, *J =* 8.1 Hz, 2H), 2.27 - 2.16 (m, 2H).

### Synthesis Route of Intermediate BB3

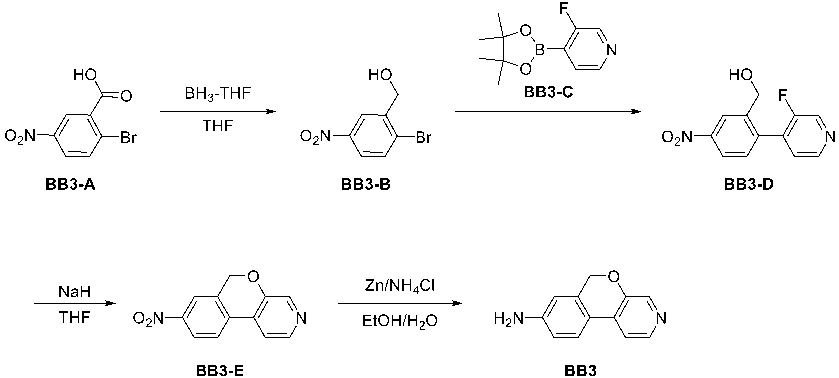

### Step 1: Intermediate BB3-B 2-bromo-5-nitrobenzyl alcohol

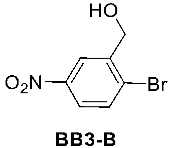

BH₃-THF (40.64 mL, 40.64 mmol) was added to a solution containing **intermediate BB3-A** 2-bromo-5-nitrobenzoic acid (5 g, 20.32 mmol) in tetrahydrofuran (50 mL) at room temperature, and the reaction mixture was reacted at 60°C for 4 hours. TLC monitoring showed the reaction was complete. The reaction mixture was quenched with methanol (50 mL), concentrated under reduced pressure, and the resulting crude product was purified by silica gel column chromatography (eluent: 20% ethyl acetate in petroleum ether solution) to obtain beige solid **intermediate BB3-B** 2-bromo-5-nitrobenzyl alcohol (4.2 g).

¹H NMR (400 MHz, CDCl₃) δ 8.45 (d, *J* = 2.7 Hz, 1H), 8.04 (dd, *J* = 8.7, 2.7 Hz, 1H), 7.74 (d, *J =* 8.7 Hz, 1H), 4.85 (s, 2H).

### Step 2: Intermediate BB3-D (2-(3-fluoropyridin-4-yl)-5-nitrophenyl)methanol

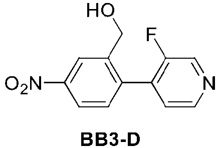

Under nitrogen protection at room temperature, Pd(dppf)Cl₂-CH₂Cl₂ (1.48 g, 1.81 mmol) was added to a mixture of **intermediate BB3-B** 2-bromo-5-nitrobenzyl alcohol (4.2 g, 18.1 mmol), **intermediate BB3-C** 3-fluoropyridin-4-ylboronic acid pinacol ester (6.05 g, 27.15 mmol) and sodium carbonate (5.75 g, 54.3 mmol) in 1,4-dioxane (60 mL) and water (10 mL). The reaction system was purged with nitrogen 3 times and heated to 90°C to react for 18 hours. TLC monitoring showed the reaction was complete. Water (60 mL) and dichloromethane (120 mL) were added to the reaction mixture for extraction. The combined organic phases were dried over anhydrous sodium sulfate, filtered, and concentrated under reduced pressure. The resulting crude product was purified by silica gel column chromatography (eluent: 40% ethyl acetate in petroleum ether solution) to obtain brown solid **intermediate BB3-D**

(2-(3-fluoropyridin-4-yl)-5-nitrophenyl)methanol (4.1 g).

LC_MS: (ES⁺): m/z 249.05 [M+H]⁺.

¹H NMR (400 MHz, CDCl₃) δ 8.56 (dd, *J* = 17.5, 2.7 Hz, 3H), 8.25 (dd, *J =* 8.4, 2.1 Hz, 1H), 7.45 (d, *J* = 8.4 Hz, 1H), 7.31 (s, 1H), 4.66 (s, 2H).

### Step 3: Intermediate BB3-E 8-nitro-6H-isochromeno[3,4-c]pyridine

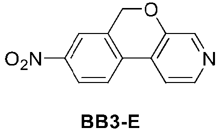

Sodium hydride (1.29 g, 32.23 mmol) was added to a solution containing **intermediate BB3-D** (2-(3-fluoropyridin-4-yl)-5-nitrophenyl)methanol (4 g, 16.11 mmol) in tetrahydrofuran (50 mL) at 0°C, and the reaction mixture was warmed to room temperature and stirred for 18 hours. TLC monitoring showed the reaction was complete. The reaction mixture was added to a saturated ammonium chloride solution (50 mL) for quenching, extracted with dichloromethane (20 mL x 2), and the combined organic layers were dried over anhydrous sodium sulfate, filtered, and concentrated under reduced pressure. The resulting crude product was purified by silica gel column chromatography (eluent: 4.76% methanol in dichloromethane solution) to obtain yellow solid **intermediate BB3-E** 8-nitro-6H-isochromeno[3,4-c]pyridine (3.3 g).

LC_MS: (ES⁺): m/z 229.05 [M+H]⁺.

### Step 4: Intermediate BB3 6H-isochromeno[3,4-c]pyridin-8-amine

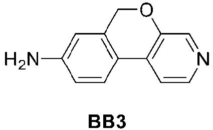

Zinc powder (4.59 g, 70.11 mmol) was added in portions to a solution containing **intermediate BB3-E** 8-nitro-6H-isochromeno[3,4-c]pyridine (3.2 g, 14.02 mmol) and ammonium chloride (3.75 g, 70.11 mmol) in ethanol (50 mL)/water (10 mL) at 80°C, and the reaction mixture was stirred at 80°C for 18 hours. TLC monitoring showed the reaction was complete. The reaction mixture was filtered, the filtrate was concentrated, water (20 mL) and dichloromethane (30 mL x 3) were added for extraction, and the combined organic phases were dried over anhydrous sodium sulfate, filtered, and concentrated. The resulting crude product was purified by silica gel column chromatography (eluent: 4.76% methanol in dichloromethane solution) to obtain brown solid intermediate BB3 6H-isochromeno[3,4-c]pyridin-8-amine (1.9 g).

LC_MS: (ES⁺): m/z 199.0 [M+H]⁺.

¹H NMR (400 MHz, DMSO) δ 8.16 - 8.10 (m, 2H), 7.59 (t, *J =* 6.8 Hz, 2H), 6.61 (dd, *J* = 8.4, 2.3 Hz, 1H), 6.41 (d, *J* = 2.2 Hz, 1H), 5.71 (s, 2H), 5.06 (s, 2H).

### Synthesis Route of Intermediate BB4

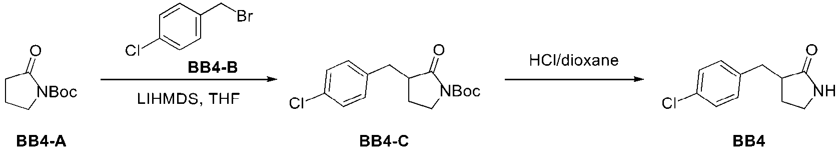

### Step 1: Intermediate BB4-C tert-butyl 3-(4-chlorobenzyl)-2-oxopyrrolidine-1-carboxylate

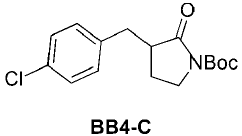

Under nitrogen protection at -60°C, LiHMDS (21.6 mL, 21.6 mmol) was slowly added to a solution containing **intermediate BB4-A** tert-butyl 2-oxopyrrolidine-1-carboxylate (4 g, 21.6 mmol) in tetrahydrofuran (60 mL). After reacting at -60°C for 1 hour, a solution (10 mL) containing **intermediate BB4-B** 4-chlorobenzyl bromide (4.44 g, 21.6 mmol) in tetrahydrofuran was slowly added dropwise. The reaction mixture was reacted at -60°C for 2 hours, then warmed to room temperature and continued to stir for 18 hours. TLC monitoring showed the reaction was complete. The reaction mixture was added to a saturated ammonium chloride solution (50 mL) for quenching, extracted with ethyl acetate, and the combined organic phases were dried over anhydrous sodium sulfate and concentrated under reduced pressure. The resulting crude product was purified by silica gel column chromatography (eluent: 10-15% ethyl acetate in petroleum ether solution) to obtain colorless oil **intermediate BB4-C** tert-butyl 3-(4-chlorobenzyl)-2-oxopyrrolidine-1-carboxylate (1.7 g).

¹H NMR (400 MHz, CDCl₃) δ 7.30 - 7.27 (m, 2H), 7.15 (d, *J =* 8.4 Hz, 2H), 3.69 (ddd, *J =* 11.2, 8.7, 2.7 Hz, 1H), 3.59 - 3.51 (m, 1H), 3.23 (dd, *J =* 13.6, 3.9 Hz, 1H), 2.83 - 2.74 (m, 1H), 2.68 (t, *J* = 6.8 Hz, 1H), 2.06 - 1.99 (m, 1H), 1.71 - 1.65 (m, 1H), 1.55 (s, 9H).

### Step 2: Intermediate BB4 3-(4-chlorobenzyl)pyrrolidin-2-one

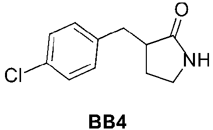

At room temperature, **intermediate BB4-C** tert-butyl 3-(4-chlorobenzyl)-2-oxopyrrolidine-1-carboxylate (1.7 g, 5.488 mmol) was added to a 4M hydrochloric acid-dioxane (20 mL) solution and stirred for 1 hour. TLC monitoring showed the reaction was complete. The mixture was concentrated under reduced pressure to obtain white solid **intermediate BB4** 3-(4-chlorobenzyl)pyrrolidin-2-one (1.15 g).

LC_MS: (ES⁺): m/z 210.06 [M+H]⁺

### Synthesis Route of Intermediate BB5

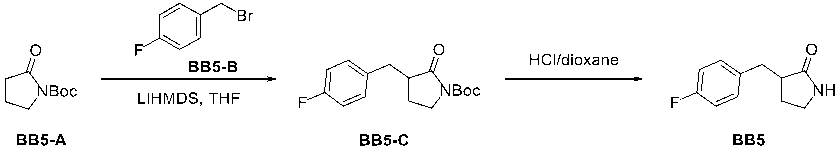

### Step 1: Intermediate BB5-C tert-butyl 3-(4-fluorobenzyl)-2-oxopyrrolidine-1-carboxylate

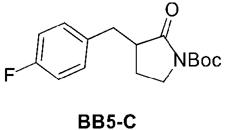

**Intermediate BB5-A** tert-butyl 2-oxopyrrolidine-1-carboxylate (7404 mg, 40 mmol) was dissolved in THF (120 mL), cooled to -78°C under nitrogen protection, and lithium bis(trimethylsilyl)amide (42 mmol, 42 mL) was added. The reaction was performed at a controlled temperature for 1 hour. **Intermediate BB5-B** 1-(bromomethyl)-4-fluorobenzene (7518 mg, 40 mmol) was dissolved in tetrahydrofuran (40 mL) and added to the reaction mixture along the wall within 5 minutes. The reaction was performed at a controlled temperature for 1 hour. A saturated ammonium chloride solution (30 mL) was added for quenching. The mixture was concentrated under reduced pressure to remove organic solvents, and the aqueous phase was extracted with ethyl acetate (50 mL x 3). The combined organic phases were washed with water (50 mL x 2) and saturated brine (50 mL), dried over anhydrous sodium sulfate, filtered, and concentrated under reduced pressure. The residue was purified by silica gel column chromatography (eluent: 27% ethyl acetate in n-hexane solution) to obtain **intermediate BB5-C** tert-butyl 3-(4-fluorobenzyl)-2-oxopyrrolidine-1-carboxylate (5240 mg, 18.22 mg), with a yield of 42.2%.

¹H NMR (400 MHz, Chloroform-d) δ 7.21 (dd, *J* = 7.4, 1.4 Hz, 1H), 7.11 (s, 1H), 7.06 (d, *J* = 7.5 Hz, 1H), 4.67 (s, 3H), 3.19 (s, 4H).

### Step 2: Intermediate BB5 3-(4-fluorobenzyl)pyrrolidin-2-one

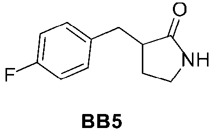

**Intermediate BB5-C** tert-butyl 3-(4-fluorobenzyl)-2-oxopyrrolidine-1-carboxylate (5240 mg, 18.22 mmol) was dissolved in dichloromethane (20 mL), cooled to 0°C, and a hydrochloric acid solution in 1,4-dioxane (45 mL, 182.2 mmol) was added. The mixture was stirred at room temperature for 1 hour, concentrated under reduced pressure to dryness, and the residue was purified by silica gel column chromatography (eluent: 7.6% methanol in dichloromethane solution) to obtain **intermediate BB5** 3-(4-fluorobenzyl)pyrrolidin-2-one (2950 mg), with a yield of 84%.

LC_MS: (ES⁺): m/z 194.07 [M+H]⁺

### Synthesis Route of Intermediate BB6

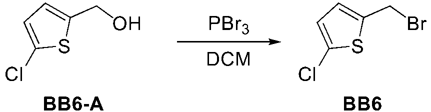

### Step 1: Intermediate BB6 2-(bromomethyl)-5-chlorothiophene

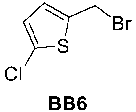

Phosphorus tribromide (136.6 mg, 0.505 mmol) was added to a solution containing **intermediate BB6-A** 5-chloro-2-thiophenemethanol (50 mg, 0.336 mmol) in dichloromethane (5 mL) at 0°C, and the reaction mixture was warmed from 0°C to room temperature and stirred for 4 hours. TLC monitoring showed the reaction was complete. Ice water was added to the reaction mixture, extraction was performed with dichloromethane, and the combined organic phases were dried over anhydrous sodium sulfate and concentrated. The resulting crude product was purified by preparative thin layer chromatography (eluent: 5% ethyl acetate in petroleum ether) to obtain brown oil **intermediate BB6** 2-(bromomethyl)-5-chlorothiophene (90 mg).

### Synthesis Route of Intermediate BB7

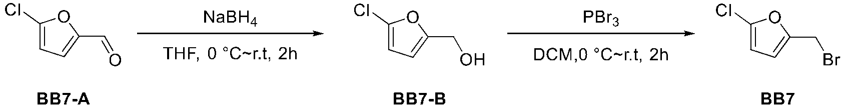

### Step 1: Intermediate BB7-B (5-chlorofuran-2-yl)methanol

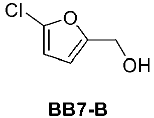

**Intermediate BB7-A** 5-chlorofuran-2-carbaldehyde (100 mg, 0.77 mmol) was dissolved in anhydrous tetrahydrofuran (2 mL), placed in an ice bath, then NaBH₄ (30 mg, 0.79 mmol) was added, and the reaction was performed at room temperature for 2 h. After completion of the reaction, saturated aqueous ammonium chloride solution (5 mL) was added dropwise, extraction was performed with ethyl acetate (5 mL) for 3 times, the organic phases were combined, dried over anhydrous sodium sulfate, and filtered, and the resulting crude **intermediate BB7-B** (5-chlorofuran-2-yl)methanol (100 mg) was used directly in the next step.

### Step 2: Intermediate BB7 2-(bromomethyl)-5-chlorofuran

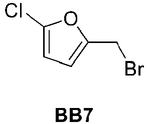

**Intermediate BB7-B** (5-chlorofuran-2-yl)methanol (100 mg, 0.77 mmol) was dissolved in dichloromethane (2 mL), and PBr₃ (200 µL, 1.54 mmol) was ad
ded dropwise in an ice bath. After addition, the reaction was performed at room temperature for 2 h. After completion of the reaction, the mixture was directly evaporated to dryness, diluted with dichloromethane (5 mL), placed in an ice bath, and saturated aqueous sodium bicarbonate solution (5 mL) was added dropwise. Extraction was performed with dichloromethane (5 mL) once, dried over anhydrous sodium sulfate, filtered, and the resulting crude **intermediate BB7** 2-(bromomethyl)-5-chlorofuran (110 mg) was used directly in the next step.

### Synthesis Route of Intermediate BB8

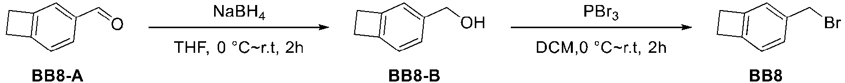

### Step 1: Intermediate BB8-B bicyclo[4.2.0]octane-1(6),2,4-trien-3-ylmethanol

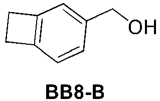

The synthesis of this step was performed using **intermediate BB8-A** 4-formylbenzocyclobutene as starting material following step 1 of the synthesis route of intermediate BB7.

¹H NMR (400 MHz, Chloroform-d) δ 7.21 (dd, *J* = 7.4, 1.4 Hz, 1H), 7.11 (s, 1H), 7.06 (d, *J* = 7.5 Hz, 1H), 4.67 (s, 3H), 3.19 (s, 4H).

### Step 2: Intermediate BB8 3-(bromomethyl)bicyclo[4.2.0]octane-1(6),2,4-triene

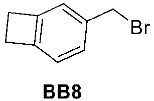

The synthesis of this step was performed using **intermediate BB8-B** 4-bicyclo[4.2.0]octane-1(6),2,4-trien-3-ylmethanol as starting material following step 2 of the synthesis route of intermediate BB7.

### Synthesis Route of Intermediate BB9

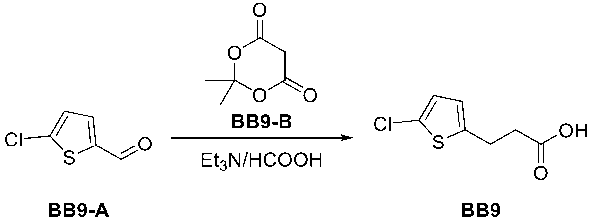

### Step 1: Intermediate BB9 3-(5-chlorothiophen-2-yl)propanoic acid

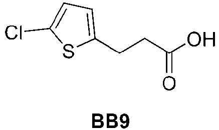

Triethylamine (834.2 mg, 8.186 mmol) was added to formic acid (941.8 mg, 20.465 mmol) at room temperature, and the mixture was stirred for 30 minutes. **Intermediate BB9-A** 5-chlorothiophene-2-carbaldehyde (1.0 g, 6.822 mmol) and **intermediate BB9-B** 2,2-dimethyl-1,3-dioxane-4,6-dione (983 mg, 6.822 mmol) were added separately, and the reaction mixture was heated to 95°C and stirred for 20 hours. LCMS monitoring showed the reaction was complete. The reaction mixture was diluted with water (15 mL) and extracted with ethyl acetate (15 mL x 2). The organic layers were combined, dried over anhydrous sodium sulfate, filtered, and concentrated under reduced pressure. The resulting crude product was purified by silica gel column chromatography (eluent: 5% methanol in dichloromethane) to obtain yellow oil **intermediate BB9** 3-(5-chlorothiophen-2-yl)propanoic acid (940 mg).

LC_MS: (ES⁻): m/z 188.95 [M-H] ⁻.

### Synthesis Route of Intermediate BB10

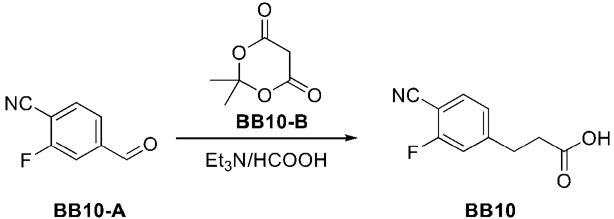

### Step 1: Intermediate BB10 3-(4-cyano-3-fluorophenyl)propanoic acid

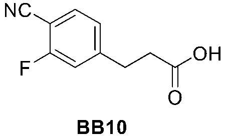

The synthesis of this step was performed using **intermediate BB10-A** 2-fluoro-4-formylbenzonitrile as starting material following step 1 of the synthesis route of intermediate BB9.

LC_MS: (ES⁻): m/z 385.10 [2M-H] ⁻.

### Synthesis Route of Intermediate BB11

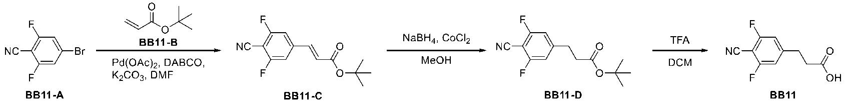

### Step 1: Intermediate BB11-C tert-butyl (E)-3-(4-cyano-3,5-difluorophenyl)acrylate

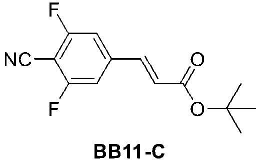

Under nitrogen protection, palladium acetate (156.3 mg, 0.7 mmol) was added to N,N-dimethylformamide (10 mL) containing **intermediate BB11-A** 4-bromo-2,6-difluorobenzonitrile (1 g, 4.6 mmol), **intermediate BB11-B** tert-butyl acrylate (882 mg, 6.9 mmol), DABCO (17 mg, 0.152 mmol) and potassium carbonate (565.83 mg, 4.1 mmol). The reaction system was purged with nitrogen 3 times and heated to 120°C for 2 hours. TLC monitoring showed the reaction was complete. The reaction mixture was extracted with ethyl acetate (30 mL x 2) and water (30 mL). The organic phases were combined, dried over anhydrous sodium sulfate, and concentrated under reduced pressure. The resulting crude product was purified by silica gel column chromatography (eluent: 0%-20% ethyl acetate in petroleum ether) to obtain white solid **intermediate BB11-C** tert-butyl (E)-3-(4-cyano-3,5-difluorophenyl)acrylate (350 mg).

¹H NMR (400 MHz, CDCl₃) δ 7.47 (d, *J* = 15.9 Hz, 1H), 7.18 (d, *J* = 8.2 Hz, 2H), 6.47 (d, *J=* 15.9 Hz, 1H), 1.56 (s, 9H).

### Step 2: Intermediate BB11-D tert-butyl 3-(4-cyano-3,5-difluorophenyl)propanoate

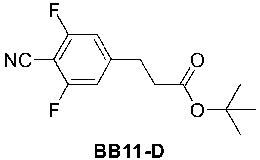

Sodium borohydride (178.18 mg, 4.1 mmol) was slowly added to a solution containing **intermediate BB11-C** (E)-3-(4-cyano-3,5-difluorophenyl)acrylic acid tert-butyl ester (250 mg, 0.942 mmol) and cobalt chloride (25 mg, 0.192 mmol) in methanol (5 mL) at 0°C. The reaction mixture was stirred at 0°C for 3 hours. TLC monitoring showed the reaction was complete. The reaction mixture was quenched with saturated ammonium chloride solution (10 mL) and extracted with ethyl acetate (15 mL x 2) and water (10 mL). The organic phases were combined, dried over anhydrous sodium sulfate, and concentrated under reduced pressure. The resulting crude product was purified by silica gel column chromatography (eluent: 0%-20% ethyl acetate in petroleum ether) to obtain white solid **intermediate BB11-D** tert-butyl 3-(4-cyano-3,5-difluorophenyl)propanoate (60 mg).

¹H NMR (400 MHz, CDCl₃) δ 6.93 (d, *J* = 8.3 Hz, 2H), 2.98 (t, *J* = 7.3 Hz, 2H), 2.58 (td, *J =* 7.4, 2.2 Hz, 2H), 1.44 (s, 9H).

### Step 3: Intermediate BB11 3-(4-cyano-3,5-difluorophenyl)propanoic acid

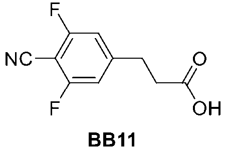

Trifluoroacetic acid (200 µL) was added to a solution containing **intermediate BB11-D** tert-butyl 3-(4-cyano-3,5-difluorophenyl)propanoate (60 mg, 0.224 mmol) in dichloromethane (1 mL), and the reaction mixture was stirred at room temperature for 5 hours. TLC monitoring showed the reaction was complete. The reaction mixture was concentrated to obtain **intermediate BB11** 3-(4-cyano-3,5-difluorophenyl)propanoic acid (55 mg), which was used directly in the next step.

LC_MS: (ES⁻): m/z 210.1 [M-H] ⁻.

### Synthesis Route of Intermediate BB12

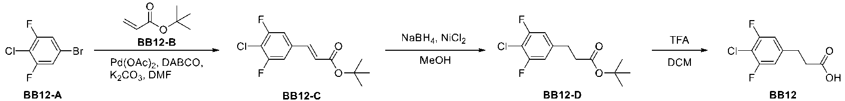

### Step 1: Intermediate BB12-C tert-butyl (E)-3-(4-chloro-3,5-difluorophenyl)acrylate

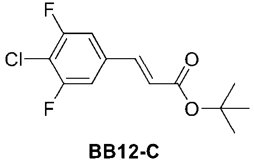

The synthesis of this step was performed using **intermediate BB12-A** 5-bromo-2-chloro-1,3-difluorobenzene as a starting material following step 1 of the synthesis route of intermediate BB11.

¹H NMR (400 MHz, CDCl₃) δ 7.45 (d, *J* = 15.9 Hz, 1H), 7.17 - 7.10 (m, 2H), 6.36 (d, *J* = 15.9 Hz, 1H), 1.55 (s, 9H).

### Step 2: Intermediate BB12-D tert-butyl 3-(4-cyano-3,5-difluorophenyl)propanoate

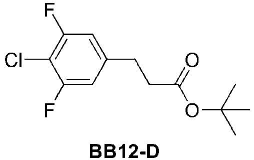

At 0°C, sodium borohydride (165.24 mg, 4.37 mmol) was slowly added to a solution containing **intermediate BB12-C** tert-butyl (E)-3-(4-chloro-3,5-difluorophenyl)acrylate (400 mg, 1.46 mmol) and nickel chloride (188.72 mg, 1.46 mmol) in methanol (10 mL). The reaction mixture was stirred at 0°C for 3 hours. TLC monitoring showed the reaction was complete. The reaction mixture was quenched with saturated ammonium chloride solution (10 mL), extracted with ethyl acetate (10 mL x 2) and water (10 mL), and the organic phases were combined, dried over anhydrous sodium sulfate, and concentrated under reduced pressure. The resulting crude product was purified by silica gel column chromatography (eluent: 0%-20% ethyl acetate in petroleum ether) to obtain white solid **intermediate BB12-D** tert-butyl 3-(4-cyano-3,5-difluorophenyl)propanoate (380 mg).

¹H NMR (400 MHz, CDCl₃) δ 6.86 (d, *J* = 7.6 Hz, 2H), 2.89 (t, *J* = 7.5 Hz, 2H), 2.54 (dd, *J* = 8.6, 6.5 Hz, 2H), 1.44 (s, 9H).

### Step 3: Intermediate BB12 3-(4-chloro-3,5-difluorophenyl)propanoic acid

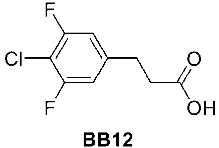

The synthesis of this step was performed using **intermediate BB12-D** tert-butyl 3-(4-cyano-3,5-difluorophenyl)propanoate as a starting material following step 3 of the synthesis route of intermediate BB11.

LC_MS: (ES⁻): m/z 219.2 [M-H] ⁻.

### Synthesis Route of Intermediate BB13

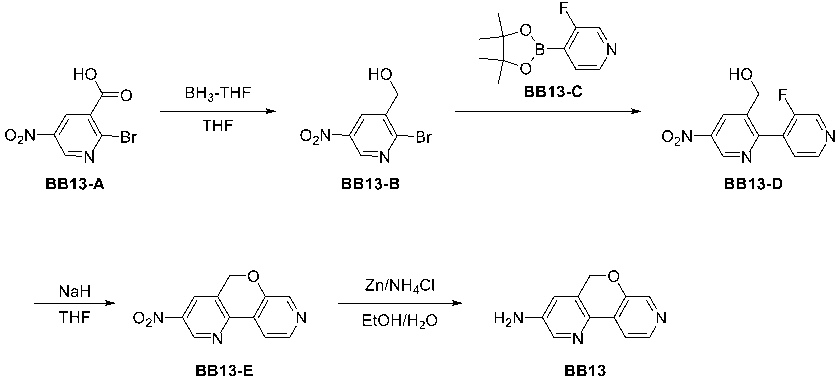

### Step 1: Intermediate BB13-B (2-bromo-5-nitropyridin-3-yl)methanol

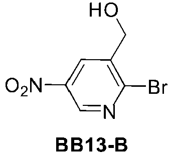

The synthesis of this step was performed using **intermediate BB13-A** 2-bromo-5-nitronicotinic acid as a starting material following step 1 of the synthesis route of intermediate BB3.

LC_MS: (ES⁺): m/z 232.9 [M+H]⁺.

### Step 2: Intermediate BB13-D (3'-fluoro-5-nitro-[2,4'-bipyridin]-3-yl)methanol

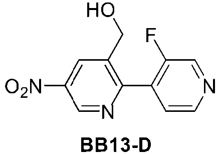

The synthesis of this step was performed using **intermediate BB13-B** (2-bromo-5-nitropyridin-3-yl)methanol and **intermediate BB13-C** 3-fluoropyridin-4-ylboronic acid pinacol ester as starting materials following step 2 of the synthesis route of intermediate BB3.

LC_MS: (ES⁺): m/z 250.0 [M+H]⁺.

### Step 3: Intermediate BB13-E 3-nitro-5H-pyrano[4,5-b:2,3-c']dipyridine

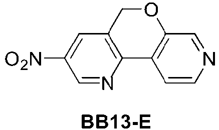

The synthesis of this step was performed using **intermediate BB13-D** (3'-fluoro-5-nitro-[2,4'-bipyridin]-3-yl)methanol as a starting material following step 3 of the synthesis route of intermediate BB3.

LC_MS: (ES⁺): m/z 230.0 [M+H]⁺.

### Step 4: Intermediate BB13 5H-pyrano[4,5-b:2,3-c']dipyridin-3-amine

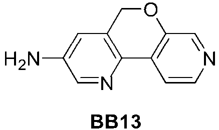

The synthesis of this step was performed using **intermediate BB13-E** 3-nitro-5H-pyrano[4,5-b:2,3-c']dipyridine as a starting material following step 4 of the synthesis route of intermediate BB3.

LC_MS: (ES⁺): m/z 200.1 [M+H]⁺.

### Synthesis Route of Intermediate BB14

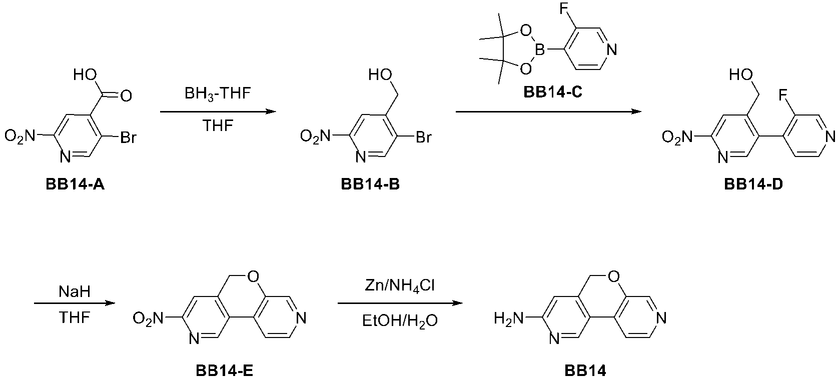

### Step 1: Intermediate BB14-B (5-bromo-2-nitropyridin-4-yl)methanol

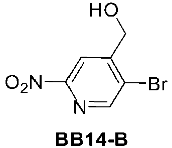

The synthesis of this step was performed using **intermediate BB14-A** 5-bromo-2-nitroisonicotinic acid as a starting material following step 1 of the synthesis route of intermediate BB3.

LC_MS: (ES⁺): m/z 232.9 [M+H]⁺.

### Step 2: Intermediate BB14-D (3'-fluoro-6-nitro-[3,4'-bipyridin]-4-yl)methanol

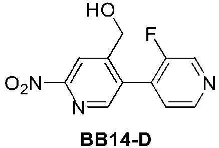

The synthesis of this step was performed using **intermediate BB14-B** (5-bromo-2-nitropyridin-4-yl)methanol and **intermediate BB14-C** 3-fluoropyridin-4-ylboronic acid pinacol ester as starting materials following step 2 of the synthesis route of intermediate BB3.

LC_MS: (ES⁺): m/z 250.0 [M+H]⁺.

### Step 3: Intermediate BB14-E 3-nitro-5H-pyrano[2,3-c:4,5-c']dipyridine

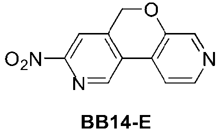

The synthesis of this step was performed using **intermediate BB14-D** (3'-fluoro-6-nitro-[3,4'-bipyridin]-4-yl)methanol as a starting material following step 3 of the synthesis route of intermediate BB3.

LC_MS: (ES⁺): m/z 230.0 [M+H]⁺.

### Step 4: Intermediate BB14 5H-pyrano[2,3-c:4,5-c']dipyridin-3-amine

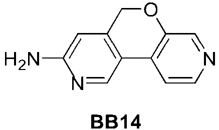

The synthesis of this step was performed using **intermediate BB14-E** 3-nitro-5H-pyrano[2,3-c:4,5-c']dipyridine as a starting material following step 4 of the synthesis route of intermediate BB3.

LC_MS: (ES⁺): m/z 200.1 [M+H]⁺.

### Synthesis Route of Intermediate BB15

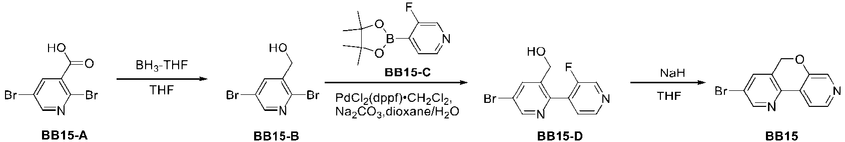

### Step 1: Intermediate BB15-B (2,5-dibromopyridin-3-yl)methanol

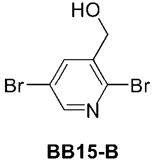

The synthesis of this step was performed using **intermediate BB15-A** 2,5-dibromonicotinic acid as a starting material following step 1 of the synthesis route of intermediate BB3.

LC_MS: (ES⁺): m/z 265.8 [M+H]⁺.

### Step 2: Intermediate BB15-D (5-bromo-3'-fluoro-[2,4'-bipyridin]-3-yl)methanol

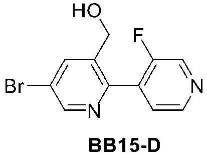

The synthesis of this step was performed using **intermediate BB15-B** (2,5-dibromopyridin-3-yl)methanol and **intermediate BB15-C** 3-fluoropyridin-4-ylboronic acid pinacol ester as starting materials following step 1 of the synthesis route of intermediate BB1.

LC_MS: (ES⁺): m/z 283.0 [M+H]⁺.

### Step 3: Intermediate BB15 3-bromo-5H-pyrano[4,5-b:2,3-c']dipyridine

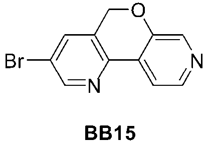

The synthesis of this step was performed using **intermediate BB15-D** (5-bromo-3'-fluoro-[2,4'-bipyridin]-3-yl)methanol as a starting material following step 2 of the synthesis route of intermediate BB1.

LC_MS: (ES⁺): m/z 263.0 [M+H]⁺.

### Synthesis Route of Intermediate BB16

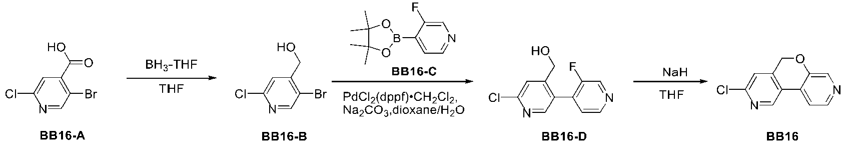

### Step 1: Intermediate BB16-B (5-bromo-2-chloropyridin-4-yl)methanol

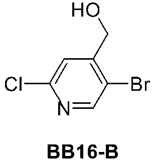

The synthesis of this step was performed using **intermediate BB16-A** 5-bromo-2-chloroisonicotinic acid as a starting material following step 1 of the synthesis route of intermediate BB3.

LC_MS: (ES⁺): m/z 221.9 [M+H]⁺.

### Step 2: Intermediate BB16-D (6-chloro-3'-fluoro-[3,4'-bipyridin]-4-yl)methanol

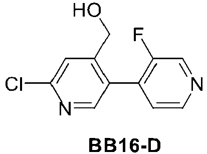

The synthesis of this step was performed using **intermediate BB16-B** (5-bromo-2-chloropyridin-4-yl)methanol and **intermediate BB16-C** 3-fluoropyridin-4-ylboronic acid pinacol ester as starting materials following step 1 of the synthesis route of intermediate BB1.

LC_MS: (ES⁺): m/z 239.0 [M+H]⁺.

### Step 3: Intermediate BB16 3-chloro-5H-pyrano[2,3-c:4,5-c']dipyridine

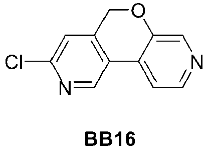

The synthesis of this step was performed using **intermediate BB16-D** (6-chloro-3'-fluoro-[3,4'-bipyridin]-4-yl)methanol as a starting material following step 2 of the synthesis route of intermediate BB1.

LC_MS: (ES⁺): m/z 219.0 [M+H]⁺.

### Synthesis Route of Intermediate BB17

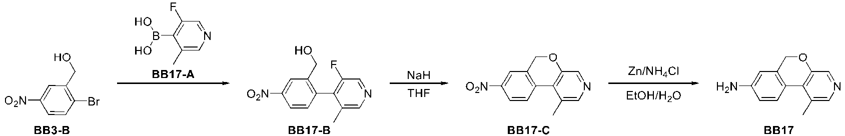

### Step 1: Intermediate BB17-B (2-(3-fluoro-5-methylpyridin-4-yl)-5-nitrophenyl)methanol

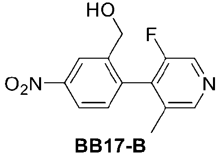

The synthesis of this step was performed using **intermediate BB3-B** (2-bromo-5-nitrophenyl)methanol and **intermediate BB17-A** (3-fluoro-5-methylpyridin-4-yl)boronic acid as starting materials following step 2 of the synthesis route of intermediate BB3.

LC_MS: (ES⁺): m/z 263.1 [M+H]⁺.

### Step 2: Intermediate BB17-C 1-methyl-8-nitro-6H-isochromeno[3,4-c]pyridine

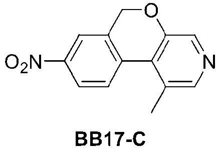

The synthesis of this step was performed using **intermediate BB17-B** (2-(3-fluoro-5-methylpyridin-4-yl)-5-nitrophenyl)methanol as a starting material following step 3 of the synthesis route of intermediate BB3.

LC_MS: (ES⁺): m/z 243.1 [M+H]⁺.

### Step 3: Intermediate BB17 1-methyl-6H-isochromeno[3,4-c]pyridin-8-amine

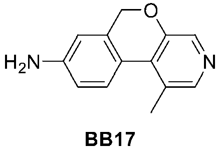

The synthesis of this step was performed using **intermediate BB17-C** 1-methyl-8-nitro-6H-isochromeno[3,4-c]pyridine as a starting material following step 4 of the synthesis route of intermediate BB3.

LC_MS: (ES⁺): m/z 213.1 [M+H]⁺.

### Synthesis Route of Intermediate BB18

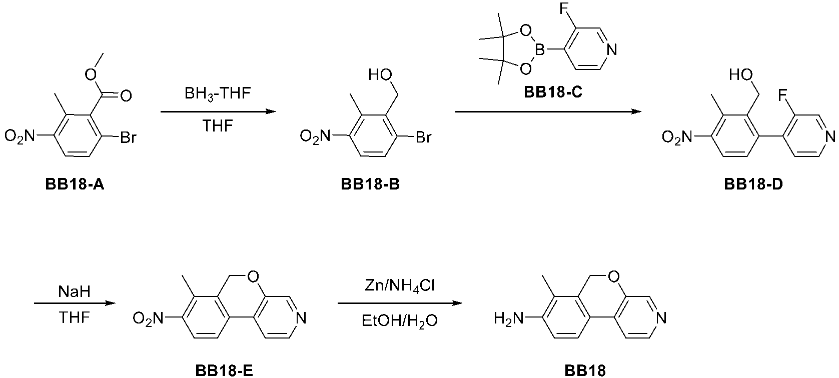

### Step 1: Intermediate BB18-B (6-bromo-2-methyl-3-nitrophenyl)methanol

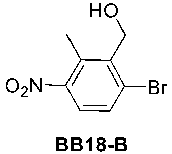

The synthesis of this step was performed using **intermediate BB18-A** methyl 6-bromo-2-methyl-3-nitrobenzoate as a starting material following step 1 of the synthesis route of intermediate BB3.

LC_MS: (ES⁺): m/z 246.0 [M+H]⁺.

### Step 2: Intermediate BB18-D (6-(3-fluoropyridin-4-yl)-2-methyl-3-nitrophenyl)methanol

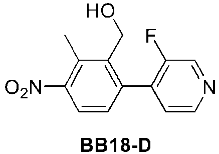

The synthesis of this step was performed using **intermediate BB18-B** (6-bromo-2-methyl-3-nitrophenyl)methanol and **intermediate BB18-C** 3-fluoropyridin-4-ylboronic acid pinacol ester as starting materials following step 2 of the synthesis route of intermediate BB3.

LC_MS: (ES⁺): m/z 263.1 [M+H]⁺.

### Step 3: Intermediate BB18-E 7-methyl-8-nitro-6H-isochromeno[3,4-c]pyridine

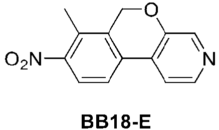

The synthesis of this step was performed using **intermediate BB18-D** (6-(3-fluoropyridin-4-yl)-2-methyl-3-nitrophenyl)methanol as a starting material following step 3 of the synthesis route of intermediate BB3.

LC_MS: (ES⁺): m/z 243.1 [M+H]⁺.

### Step 4: Intermediate BB18 7-methyl-6H-isochromeno[3,4-c]pyridin-8-amine

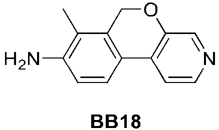

The synthesis of this step was performed using **intermediate BB18-E** 7-methyl-8-nitro-6H-isochromeno[3,4-c]pyridine as a starting material following step 4 of the synthesis route of intermediate BB3.

LC_MS: (ES⁺): m/z 213.1 [M+H]⁺.

### Synthesis Route of Intermediate BB19

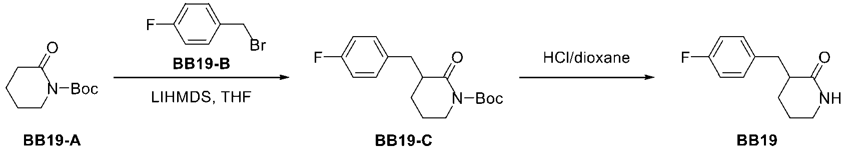

### Step 1: Compound BB19-C tert-butyl 3-(4-fluorobenzyl)-2-oxopiperidine-1-carboxylate

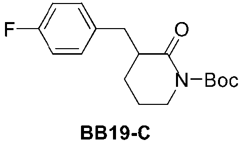

The synthesis of this step was performed following step 1 of the synthesis route of intermediate BB4.

LC_MS: (ES⁺): m/z 308.16 [M+H]⁺.

### Step 2: Compound BB19 3-(4-fluorobenzyl)piperidin-2-one

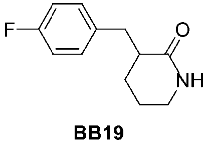

The synthesis of this step was performed following step 2 of the synthesis route of intermediate BB4.

LC_MS: (ES⁺): m/z 208.11 [M+H]⁺.

### Synthesis Route of Intermediate BB20

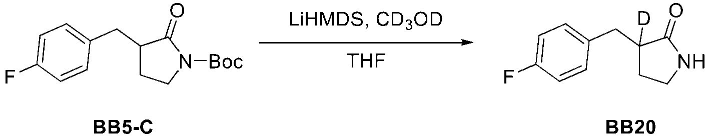

### Step 1: Compound BB20 3-(4-fluorobenzyl)pyrrolidin-2-one-3-d

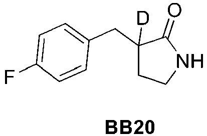

LiHMDS (4.09 mL, 4.09 mmol) was added to a solution containing **compound BB5-C** (1 g, 3.41 mmol) in tetrahydrofuran (15 mL) at 0°C, and the reaction mixture was stirred at 0°C for 0.5 hour. Deuterated methanol (5 mL) was added, and the mixture was warmed to room temperature and stirred for 2 hours. LCMS monitoring showed the reaction was complete. The reaction mixture was poured into a saturated ammonium chloride solution (30 mL), extracted with ethyl acetate (20 mL x 2), and the combined organic phases were washed with saturated brine (30 mL), dried over anhydrous sodium sulfate, and concentrated under reduced pressure. The resulting crude product was isolated and purified by silica gel column chromatography (eluent: 0%-5% methanol in dichloromethane solution) to obtain white solid **compound BB20** 3-(4-fluorobenzyl)pyrrolidin-2-one-3-d (500 mg).

LC_MS: (ES⁺): m/z 195.1 [M+H]⁺.

¹H NMR (400 MHz, CDCl₃) δ 7.22 - 7.15 (m, 2H), 7.02 - 6.95 (m, 2H), 6.36 (s, 1H), 3.32 - 3.21 (m, 2H), 3.17 (d, *J =* 14.0 Hz, 1H), 2.70 (t, *J =* 10.6 Hz, 1H), 2.19 - 2.11 (m, 1H), 1.88 - 1.80 (m, 1H).

### Synthesis Route of Intermediate BB21

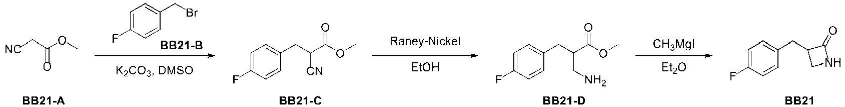

### Step 1: Intermediate BB21-C methyl 2-cyano-3-(4-fluorophenyl)propanoate

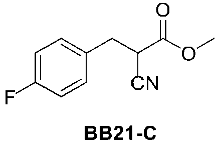

**Intermediate BB21-A** methyl cyanoacetate (3.93 g, 0.04 mol) was dissolved in DMSO (20 mL), potassium carbonate (5.48 g, 0.04 mol) was added, and the mixture was stirred at room temperature for 30 min. Then **intermediate BB21-B** 4-fluorobenzyl bromide (5.0 g, 0.026 mol) was added at 0°C, and the reaction mixture was stirred at room temperature for 6 h. TLC monitoring showed the reaction was complete. The reaction mixture was poured into water (60 mL), extracted twice with ethyl acetate (50 mL), and the combined organic phases were washed three times with saturated brine (60 mL), dried over anhydrous sodium sulfate, filtered, and concentrated by rotary evaporation. The resulting crude product was isolated and purified by silica gel column chromatography (eluent PE:EtOAc=20:1→15:1→10:1) to obtain **intermediate BB21-C** methyl 2-cyano-3-(4-fluorophenyl)propanoate (1.13 g, yield 20%) as a colorless oil.

¹H NMR (400 MHz, CDCl₃): δ 7.26-7.23 (m, 2H), 7.06-7.02 (m, 2H), 3.80 (s, 3H), 3.74-3.70 (m, 1H), 3.28-3.16 (m, 1H).

### Step 2: Intermediate BB21-D methyl 2-(aminomethyl)-3-(4-fluorophenyl)propanoate

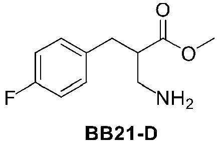

**Intermediate BB21-C** methyl 2-cyano-3-(4-fluorophenyl)propanoate (1.13 g, 5.4 mmol) was dissolved in ethanol (20 mL), Raney nickel (1.5 g) was added, and the reaction was carried out at room temperature overnight under hydrogen atmosphere (hydrogen balloon). TLC monitoring showed the reaction was complete. The reaction mixture was filtered through celite, the filter cake was washed with ethanol (20 mL), and the filtrate was collected and concentrated by rotary evaporation to obtain crude **intermediate BB21-D** methyl 2-(aminomethyl)-3-(4-fluorophenyl)propanoate (1.1 g, crude) as a colorless oil, which was used directly in the next step without purification.

LC_MS: (ES⁺): m/z 212.10 [M+H]⁺.

### Step 3: Intermediate BB21 3-[(4-fluorophenyl)methyl]azetidin-2-one

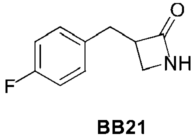

**Intermediate BB21-D** methyl 2-(aminomethyl)-3-(4-fluorophenyl)propanoate (1.1 g, 5.2 mmol) was dissolved in anhydrous diethyl ether (10 mL), and the temperature was lowered to 0°C under N₂ atmosphere. Then CH₃MgI (7 mL, 20.8 mmol, 3 mol/L) was slowly added dropwise to the reaction mixture, the ice bath was removed, and the mixture was stirred at room temperature overnight. LCMS monitoring showed the reaction was complete. The reaction mixture was quenched with saturated sodium bicarbonate (20 mL) in an ice bath, filtered, and the filter cake was washed with EtOAc. The mixture was separated, and the aqueous phase was extracted with EtOAc (20 mL x 2). The combined organic phases were washed once with saturated brine (20 mL), dried over anhydrous sodium sulfate, filtered, and concentrated by rotary evaporation. The resulting crude product was first purified by column chromatography (mobile phase DCM:CH₃OH=200:1→150:1), then stirred with n-hexane (5 mL) for 30 min and filtered to obtain **intermediate BB21** 3-[(4-fluorophenyl)methyl]azetidin-2-one (125 mg, yield 13%) as a white solid.

LC_MS: (ES⁺): m/z 180.00 [M+H]⁺.

¹H NMR (400 MHz, CDCl₃): δ 7.21-7.17 (m, 2H), 6.99 (t, *J =* 8.8 Hz, 2H), 5.66 (brs, 1H), 3.53-3.51 (m, 1H), 3.39 (t, *J* = 5.6 Hz, 1H), 3.13-3.08 (m, 1H), 3.04-3.02 (m, 1H), 2.97-2.92 (m, 1H).

### Synthesis Route of Intermediate BB22

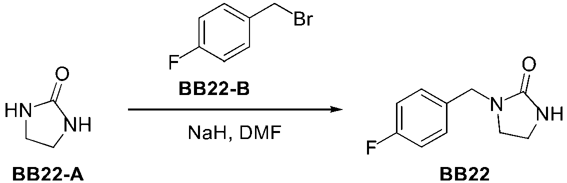

### Step 1: Intermediate BB22 1-(4-fluorobenzyl)imidazolidin-2-one

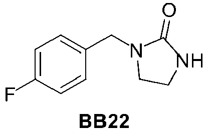

Sodium hydride (211.6 mg, 5.29 mmol, 60% wt) was added to a solution containing BB22-A imidazolidin-2-one (910.9 mg, 10.58 mmol) in DMF under an ice bath, and the mixture was stirred for half an hour. Then **BB22-B** 1-(bromomethyl)-4-fluorobenzene (1.0 g, 5.29 mmol) was added dropwise, and the mixture was stirred at room temperature for 12 hours. LCMS showed the reaction was complete. The reaction mixture was poured into saturated ammonium chloride solution (20 mL), extracted with ethyl acetate (10 mL x 3), and the organic layers were combined, washed with saturated brine (20 mL), dried over anhydrous sodium sulfate, and concentrated under reduced pressure. The residue was purified by silica gel column chromatography (eluent: 50-70% ethyl acetate in petroleum ether) to obtain yellow solid **intermediate BB22** 1-(4-fluorobenzyl)imidazolidin-2-one (350 mg).

LC_MS: (ES⁺): m/z 195.1 [M+H]⁺.

### Synthesis Route of Intermediate BB23

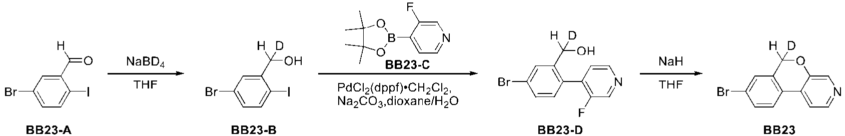

### Step 1: Intermediate BB23-B (5-bromo-2-iodophenyl)methan-d-ol

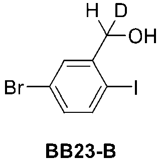

NaBD₄ (134.6 mg, 3.216 mmol) was added to a solution containing **intermediate BB23-A** 5-bromo-2-iodobenzaldehyde (1.0 g, 3.216 mmol) in tetrahydrofuran (15 mL) at 0°C. The reaction mixture was stirred at 25°C for one hour. TLC showed the reaction was complete. The reaction mixture was quenched with water (20 mL), extracted with ethyl acetate (15 mL x 2), and the organic layers were combined, washed with saturated brine (20 mL), dried over anhydrous sodium sulfate, concentrated under reduced pressure, filtered, and concentrated. The residue was purified by silica gel column chromatography (eluent: 20% ethyl acetate in petroleum ether) to obtain white solid **intermediate BB23-B** (5-bromo-2-iodophenyl)methan-d-ol (940 mg).

¹H NMR (400 MHz, CDCl₃) δ 7.67 (d, *J =* 8.3 Hz, 1H), 7.65 (dd, *J =* 2.4, 0.5 Hz, 1H), 7.18 - 7.14 (m, 1H), 4.64 (s, 1H), 2.03 (s, 1H).

### Step 2: Intermediate BB23-D (5-bromo-2-(3-fluoropyridin-4-yl)phenyl)methan-d-ol

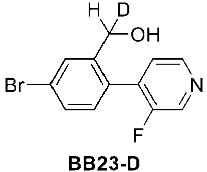

The synthesis of this step was performed following step 1 of the synthesis route of intermediate BB1.

LC_MS: (ES⁺): m/z 282.9 [M+H]⁺.

### Step 3: Intermediate BB23 8-bromo-6H-isochromeno[3,4-c]pyridin-6-d

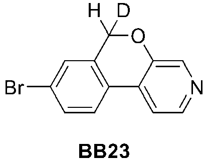

The synthesis of this step was performed following step 2 of the synthesis route of Intermediate BB1.

LC_MS: (ES⁺): m/z 262.9 [M+H]⁺.

### Synthesis Route of Intermediate BB24

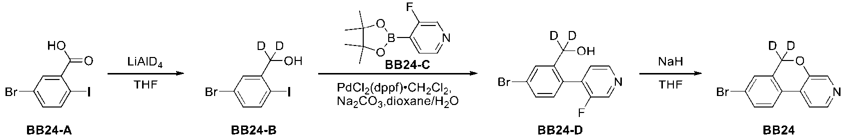

### Step 1: Intermediate BB24-B (5-bromo-2-iodophenyl)methan-d2-ol

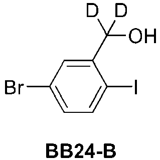

LiAlD₄ (423.9 mg, 10.08 mmol) was slowly added to a solution containing **intermediate BB24-A** 5-bromo-2-iodobenzoic acid (3 g, 9.18 mmol) in tetrahydrofuran (30 mL) at 0°C, and the reaction mixture was stirred at 0°C for 3 hours. TLC showed the reaction was complete. Water (0.424 mL) and 10% sodium hydroxide solution (0.424 mL) were sequentially added to the reaction mixture at 0°C to quench the reaction. The mixture was filtered, the filtrate was concentrated, and the obtained crude product was purified by silica gel column chromatography (eluent: 0%-20% ethyl acetate in petroleum ether) to obtain white solid **intermediate BB24-B** (5-bromo-2-iodophenyl)methan-d2-ol (1.3 g).

¹H NMR (400 MHz, CDCl₃) δ 7.68 (d, *J =* 8.3 Hz, 1H), 7.65 (d, *J =* 2.4 Hz, 1H), 7.16 (dd, *J* = 8.3, 2.4 Hz, 1H), 2.01 (s, 1H).

### Step 2: Intermediate BB24-D (5-bromo-2-(3-fluoropyridin-4-yl)phenyl)methan-d2-ol

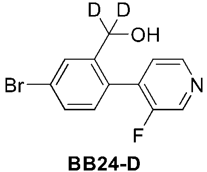

The synthesis of this step was performed following step 1 of the synthesis route of intermediate BB1.

LC_MS: (ES⁺): m/z 284.0 [M+H]⁺.

### Step 3: Intermediate BB24 8-bromo-6H-isochromeno[3,4-c]pyridin-6,6-d2

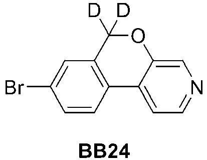

The synthesis of this step was performed following step 2 of the synthesis route of Intermediate BB1.

LC_MS: (ES⁺): m/z 263.9 [M+H]⁺.

### Synthesis Route of Intermediate BB25

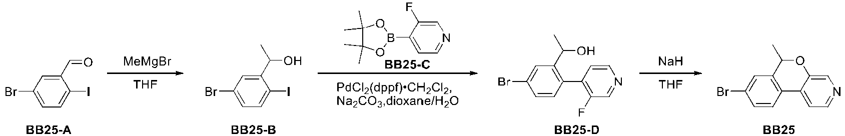

### Step 1: Intermediate BB25-B 1-(5-bromo-2-iodophenyl)ethan-1-ol

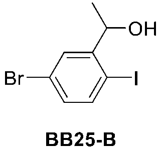

Methylmagnesium bromide (1.07 mL, 3.21 mmol) was slowly added to a solution containing **intermediate BB25-A** 5-bromo-2-iodobenzaldehyde (1 g, 3.21 mmol) in tetrahydrofuran (10 mL) at 0°C, and the reaction mixture was stirred at 0°C for 1 hour. TLC showed the reaction was complete. The reaction mixture was quenched with saturated ammonium chloride solution (15 mL), extracted with ethyl acetate (10 mL x 2), and the combined organic phases were dried over anhydrous sodium sulfate, filtered, and concentrated. The obtained crude product was purified by silica gel column chromatography (eluent: 0%-20% ethyl acetate in petroleum ether) to obtain yellow gum compound **intermediate BB25-B** 1-(5-bromo-2-iodophenyl)ethan-1-ol (960 mg).

### Step 2: Intermediate BB25-D 1-(5-bromo-2-(3-fluoropyridin-4-yl)phenyl)ethan-1-ol

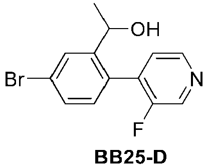

The synthesis of this step was performed following step 1 of the synthesis route of intermediate BB1.

LC_MS: (ES⁺): m/z 296.0 [M+H]⁺.

### Step 3: Intermediate BB25 8-bromo-6-methyl-6H-isochromeno[3,4-c]pyridine

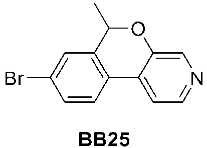

The synthesis of this step was performed following step 2 of the synthesis route of intermediate BB1.

LC_MS: (ES⁺): m/z 275.9 [M+H]⁺.

### Synthesis Route of Intermediate BB26

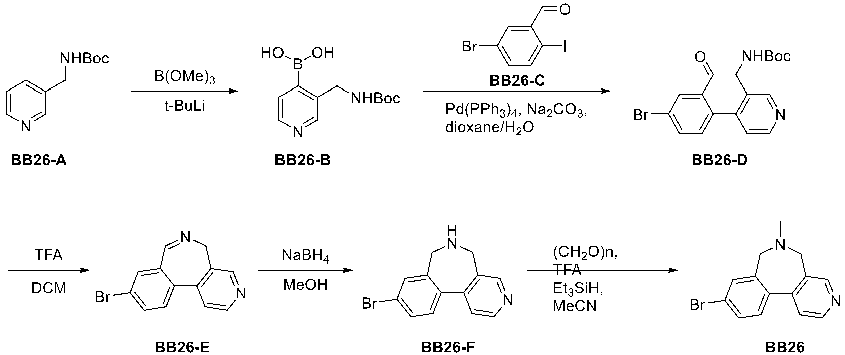

### Step 1: Intermediate BB26-B (3-(((tert-butoxycarbonyl)amino)methyl)pyridin-4-yl)boronic acid

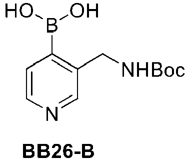

3 g (14.4 mmol, 1.0 eq) of **Intermediate BB26-A** was weighed and placed in a 100 mL three-necked flask, 15 mL of dry THF was added, and after displacement with a nitrogen balloon, the temperature was lowered to -60°C. 23 mL (30.3 mmol, 2.1 eq, 1.3 M in pentane) of tert-butyllithium was slowly added dropwise into the three-necked flask at -60°C. After the addition was complete, the mixture was slowly warmed to -20°C, and 6.0 g (57.6 mmol, 4.0 eq) of trimethyl borate was slowly added dropwise at this temperature. After the addition was complete, the mixture was allowed to warm to room temperature naturally and stirred overnight. LCMS monitoring showed about 25% of the product. 1N dilute hydrochloric acid was slowly added to the reaction mixture until the pH of the solution reached 6. The low-boiling solvents were removed by concentration, the residue was diluted with water to 30 mL, and extracted twice with a dichloromethane/isopropanol=3/1 solution (30 mL). The organic phases were combined, dried over anhydrous sodium sulfate, filtered and concentrated to obtain 3.1 g of a yellow oily crude product **Intermediate BB26-B,** which was used directly in the next step reaction.

LC_MS: (ES⁺): m/z 253.00 [M+H]⁺.

### Step 2: Intermediate BB26-D tert-butyl ((4-(4-bromo-2-formylphenyl)pyridin-3-yl)methyl)carbamate

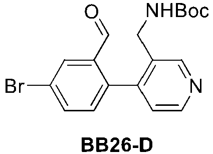

The crude **Intermediate BB26-B** obtained from the previous step was added to a 250 mL single-necked flask, and 30 mL of 1,4-dioxane and 15 mL of water were added. Then **Intermediate BB26-C** 5-bromo-2-iodobenzaldehyde (1.9 g, 6.13 mmol, 0.5 eq), sodium carbonate (1.3 g, 12.25 mmol, 2.0 eq), and tetrakis(triphenylphosphine)palladium (700 mg, 0.61 mmol, 0.1 eq) were weighed sequentially, the flask was purged with a nitrogen balloon 3 times, and the reaction was carried out at 90°C overnight. LCMS monitoring showed that starting material 2 had been consumed completely. The reaction mixture was poured into 100 mL of water, extracted twice with ethyl acetate (50 mL), the organic phases were combined, dried over anhydrous sodium sulfate, and the obtained crude product after concentration was purified by column chromatography (petroleum ether/ethyl acetate=2/1-1/1) to obtain 2 g of a white viscous crude product **Intermediate BB26-D,** which was used directly in the next step reaction.

LC_MS: (ES⁺): m/z 390.75 [M+H]⁺.

### Step 3: Intermediate BB26-E 9-bromo-5H-benzo[c]pyrido[4,3-e]azepine

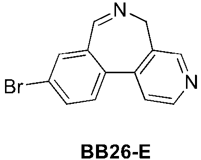

The crude **Intermediate BB26-D** was dissolved in dichloromethane (20 mL), trifluoroacetic acid (10 mL) was added, and the mixture was stirred at room temperature for 1 hour. TLC monitoring showed that the starting material had been consumed completely. The reaction mixture was poured into water (50 mL), the pH was adjusted to >7 with solid sodium bicarbonate, and extracted with dichloromethane (20 mL). The organic phase was collected, washed with saturated brine, dried over anhydrous sodium sulfate and concentrated, and the crude product was purified by silica gel column chromatography (eluent: dichloromethane/ethyl acetate=1/1 to 1/2) to obtain 170 mg of a light yellow solid **Intermediate BB26-E** 9-bromo-5H-benzo[c]pyrido[4,3-e]azepine.

LC_MS: (ES⁺): m/z 272.75 [M+H]⁺.

### Step 4: Intermediate BB26-F 9-bromo-6,7-dihydro-5H-benzo[c]pyrido[4,3-e]azepine

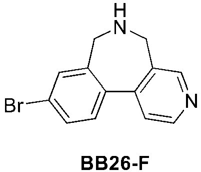

**Intermediate BB26-E** 9-bromo-5H-benzo[c]pyrido[4,3-e]azepine (200 mg, 0.73 mmol, 1.0 eq, crude product with 85% purity) was dissolved in methanol (2 mL), cooled to 0°C, and then sodium borohydride (28 mg, 0.73 mmol, 1.0 eq) was added in 3 portions. After the addition was complete, the mixture was warmed to room temperature and stirred for 1 hour. TLC monitoring showed that Intermediate BB26-E had been consumed completely. The reaction mixture was concentrated directly, and purified by silica gel column chromatography (eluent: dichloromethane/methanol=30/1-10/1) to obtain 220 mg of a light yellow viscous product **Intermediate BB26-F** 9-bromo-6,7-dihydro-5H-benzo[c]pyrido[4,3-e]azepine.

LC_MS: (ES⁺): m/z 274.70 [M+H]⁺.

### Step 5: Intermediate BB26 9-bromo-6-methyl-6,7-dihydro-5H-benzo[c]pyrido[4,3-e]azepine

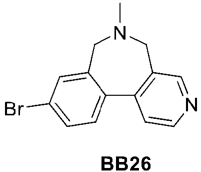

**Intermediate BB26-F** 9-bromo-6,7-dihydro-SH-benzo[c]pyrido[4,3-e]azepine (228 mg, 0.83 mmol, 1.0 eq) was dissolved in 5 mL of acetonitrile, then paraformaldehyde (199 mg, 6.63 mmol, 8.0 eq), triethylsilane (293 mg, 2.57 mmol, 3.1 eq), and trifluoroacetic acid (288 mg, 2.49 mmol, 3.0 eq) were added sequentially, and the mixture was stirred at room temperature overnight. TLC monitoring showed that starting material BB26-F had been consumed completely. The reaction mixture was poured into saturated sodium bicarbonate solution (50 mL), and extracted with ethyl acetate (50 mL). The organic phase was washed with saturated brine, dried over anhydrous sodium sulfate, and the crude product after concentration was purified by silica gel column chromatography (eluent: dichloromethane/methanol=50/1-20/1) to obtain 210 mg of colorless viscous product **Intermediate BB26** 9-bromo-6-methyl-6,7-dihydro-5H-benzo[c]pyrido[4,3-e]azepine.

LC_MS: (ES⁺): m/z 288.85 [M+H]⁺.

¹H NMR (400 MHz, CDCl₃): δ 8.70 (d, *J =* 5.2 Hz, 1H), 8.60 (s, 1H), 7.65-7.62 (m, 1H), 7.56 (d, *J =* 1.6 Hz, 1H), 7.41-7.38 (m, 2H), 3.39 (d, *J =* 10.8 Hz, 4H), 2.50 (s, 3H).

### Synthesis Route of Intermediate BB27

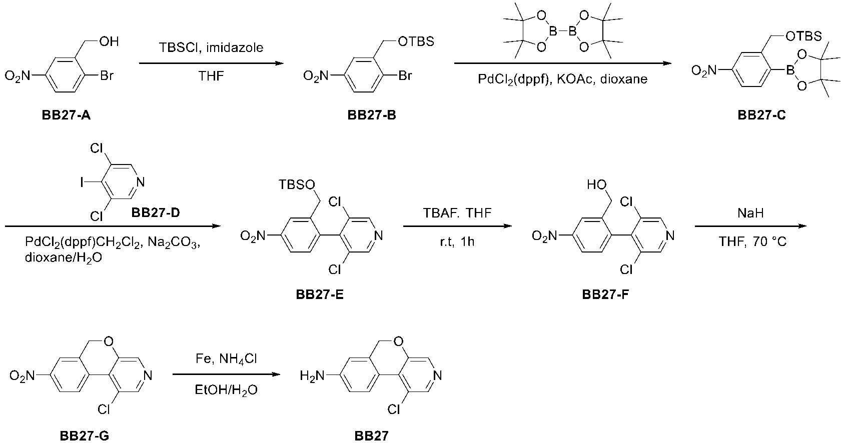

### Step 1: Intermediate BB27-B ((2-bromo-5-nitrobenzyl)oxy)(tert-butyl)dimethylsilane

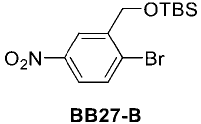

**Intermediate BB27-A** (1.0 g, 4.31 mmol, 1.0 eq) was dissolved in 10 mL of tetrahydrofuran, TBSCl (780 mg, 5.17 mmol, 1.2 eq) and imidazole (734 mg, 10.78 mmol, 2.5 eq) were added, and the mixture was stirred at room temperature overnight. TLC showed a small amount of BB27-A remaining. The reaction mixture was poured into water (20 mL), extracted with ethyl acetate (20 mL), the organic phase was washed with saturated brine (20 mL), dried over anhydrous sodium sulfate, and the crude product after concentration was purified by silica gel column chromatography (eluent: petroleum ether/ethyl acetate=50/1) to obtain 1.39 g of colorless oily liquid **Intermediate BB27-B** ((2-bromo-5-nitrobenzyl)oxy)(tert-butyl)dimethylsilane, with a yield of 93%.

¹H NMR (400 MHz, CDCl₃): δ 8.26 (d, *J =* 2.8 Hz, 1H), 7.83-7.80 (m, 1H), 7.50 (d, *J =* 8.8 Hz, 1H), 4.59 (s, 2H), 0.83 (s, 9H), 0.00 (s, 6H).

### Step 2: Intermediate BB27-C tert-butyldimethyl((5-nitro-2-(4,4,5,5-tetramethyl-1,3,2-dioxaborolan-2-yl)benzyl)oxy)silane

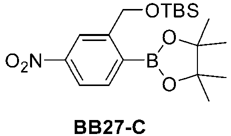

**Intermediate BB27-B** ((2-bromo-5-nitrobenzyl)oxy)(tert-butyl)dimethylsilane (790 mg, 2.28 mmol, 1.0 eq) was dissolved in 8 mL of 1,4-dioxane, then bis(pinacolato)diboron (693 mg, 2.74 mmol, 1.2 eq), potassium acetate (447 mg, 4.56 mmol, 2.0 eq), and Pd(dppf)Cl₂ (83 mg, 0.11 mmol, 0.05 eq) were added sequentially, a reflux condenser was attached, the flask was purged with nitrogen 3 times, and the reaction was carried out at 100°C for 3 hours. TLC monitoring showed that BB27-B had been consumed completely. The reaction mixture was filtered directly to remove insoluble matter, and the filtrate was concentrated to obtain 1.8 g of black oily crude product **Intermediate BB27-C** tert-butyldimethyl((5-nitro-2-(4,4,5,5-tetramethyl-1,3,2-dioxaborolan-2-yl)benzyl)oxy)silane, which was used directly in the next step reaction.

### Step 3: Intermediate BB27-E 4-(2-(((tert-butyldimethylsilyl)oxy)methyl)-4-nitrophenyl)-3,5-dichloropyridine

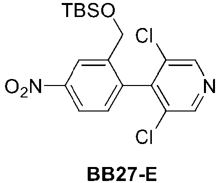

Crude **Intermediate BB27-C** tert-butyldimethyl((5-nitro-2-(4,4,5,5-tetramethyl-1,3,2-dioxaborolan-2-yl)benzyl)oxy)silane (897 mg, 2.28 mmol, 1.0 eq), **Intermediate BB27-D** 3,5-dichloro-4-iodopyridine (624 mg, 2.28 mmol, 1.0 eq), and sodium carbonate (725 mg, 6.84 mmol, 3.0 eq) were added to a mixture of 1,4-dioxane (12 mL) and water (2 mL), then Pd(dppf)Cl₂•DCM (186 mg, 0.23 mmol, 0.1 eq) was added, the flask was purged with a nitrogen balloon 3 times, and the mixture was stirred at 70°C overnight. LCMS monitoring showed the reaction was complete, the reaction mixture was poured into water (50 mL), extracted with ethyl acetate (20 mL x 2), the organic phases were combined, dried over anhydrous sodium sulfate, and the crude product after concentration was purified by silica gel column chromatography (eluent: petroleum ether/ethyl acetate=50/1 to 40/1) to obtain 258 mg of a white solid **Intermediate BB27-E** 4-(2-(((tert-butyldimethylsilyl)oxy)methyl)-4-nitrophenyl)-3,5-dichloropyridine.

¹H NMR (400 MHz, CDCl₃): δ 8.63 (brs, 2H), 8.53 (d, *J =* 2.4 Hz, 1H), 8.25-8.22 (m, 1H), 7.22 (s, 1H), 4.45 (s, 2H), 0.89 (s, 9H), 0.00 (s, 6H).

### Step 4: Intermediate BB27-F (2-(3,5-dichloropyridin-4-yl)-5-nitrophenyl)methanol

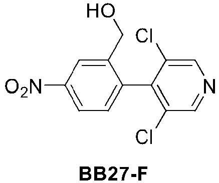

**Intermediate** BB27-E 4-(2-(((tert-butyldimethylsilyl)oxy)methyl)-4-nitrophenyl)-3,5-dichloropyridine (1.6 g, 3.87 mmol, 1.0 eq) was dissolved in 15 mL of tetrahydrofuran, TBAF (11.6 mL, 11.61 mmol, 3.0 eq, 1M in THF) was added, and the mixture was stirred at room temperature for 1 hour. TLC monitoring showed that starting material BB27-E had been consumed completely. The reaction mixture was poured into saturated ammonium chloride solution (50 mL), extracted with ethyl acetate (30 mL x 2), dried over anhydrous sodium sulfate, and the concentrated crude product was purified by silica gel column chromatography (eluent: petroleum ether/ethyl acetate = 30/1 to 2/1) to obtain 591 mg of white solid **intermediate BB27-F** (2-(3,5-dichloropyridin-4-yl)-5-nitrophenyl)methanol, with a yield of 51%.

LC_MS: (ES⁺): m/z 298.65 [M+H]⁺.

### Step 5: Intermediate BB27-G 1-chloro-8-nitro-6H-isochromeno[3,4-c]pyridine

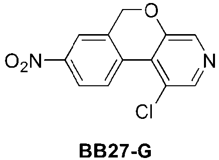

**Intermediate BB27-F** (2-(3,5-dichloropyridin-4-yl)-5-nitrophenyl)methanol (240 mg, 0.80 mmol, 1.0 eq) was dissolved in THF (5 mL), cooled in an ice-water bath for 10 minutes, sodium hydride (48 mg, 1.2 mmol, 1.5 eq) was rapidly added, and after nitrogen displacement, the mixture was stirred at room temperature for 1 hour. TLC monitoring showed that approximately half of the starting material remained. The reaction mixture was carefully poured into water (50 mL), extracted with ethyl acetate (50 mL), the organic phase was washed with saturated brine (20 mL), dried over anhydrous sodium sulfate and concentrated, and the residue was purified by silica gel column chromatography (eluent: petroleum ether/ethyl acetate = 30/1 to 2/1) to obtain 60 mg of yellow viscous oily **intermediate BB27-G** 1-chloro-8-nitro-6H-isochromeno[3,4-c]pyridine, with a yield of 22%.

LC_MS: (ES⁺): m/z 262.65 [M+H]⁺.

### Step 6: Intermediate BB27 1-chloro-6H-isochromeno[3,4-c]pyridin-8-amine

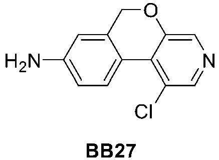

Iron powder (21.26 mg, 380.74 µmol) and ammonium chloride (20.37 mg, 380.74 µmol) were added to a mixed solution of ethanol (2 mL)/H₂O (0.4 mL) containing **intermediate BB27-G** 1-chloro-8-nitro-6H-isochromeno[3,4-c]pyridine (20 mg, 76.15 µmol), and the reaction mixture was stirred at 80°C for 2 hours. LCMS monitoring showed the reaction was complete. The reaction mixture was cooled to room temperature, filtered, the filter cake was washed with ethanol (2 mL x 2), and the filtrate was concentrated. Water (2 mL) and dichloromethane (2 mL x 2) were added for extraction, the organic layers were combined, dried over anhydrous sodium sulfate, concentrated under reduced pressure, and the residue was purified by preparative thin layer chromatography (developing solvent: dichloromethane:methanol = 20:1) to obtain **intermediate BB27** 1-chloro-6H-isochromeno[3,4-c]pyridin-8-amine (5 mg).

LC_MS: (ES⁺): m/z 233.04 [M+H]⁺.

### Synthesis Route of Intermediate BB28

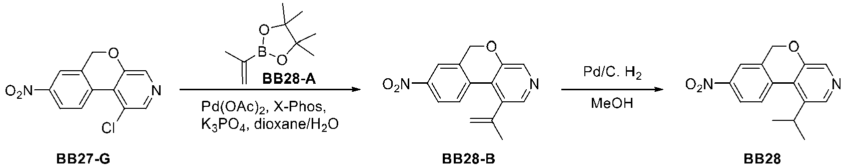

### Step 1: Intermediate BB28-B 8-nitro-1-(prop-1-en-2-yl)-6H-isochromeno[3,4-c]pyridine

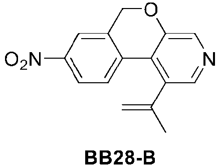

**Intermediate BB27-G** 1-chloro-8-nitro-6H-isochromeno[3,4-c]pyridine (35 mg, 0.133 mmol, 1.0 eq), isopropenylboronic acid pinacol ester (33.5 mg, 0.200 mmol, 1.5 eq), and tripotassium phosphate (56 mg, 0.266 mmol, 2.0 eq) were added to a mixed solution of 1,4-dioxane (1 mL) and water (0.2 mL), then palladium acetate (3 mg, 0.013 mmol, 0.1 eq) and X-phos (13 mg, 0.027 mmol, 0.2 eq) were added, and after nitrogen displacement for 3 times, the mixture was stirred at 100°C for 2 hours. LCMS monitoring showed that intermediate BB27-G had been consumed completely. The reaction mixture was poured into water (20 mL), extracted with ethyl acetate (20 mL), washed with saturated brine, dried over anhydrous sodium sulfate, and the concentrated crude product was purified by preparative thin layer chromatography (developing solvent: petroleum ether/ethyl acetate/methanol = 25/8/1) to obtain 8 mg of white solid **intermediate BB28-B** 8-nitro-1-(prop-1-en-2-yl)-6H-isochromeno[3,4-c]pyridine, with a yield of 22%.

LC_MS: (ES⁺): m/z 269.10 [M+H]⁺.

### Step 2: Intermediate BB28 1-isopropyl-8-nitro-6H-isochromeno[3,4-c]pyridine

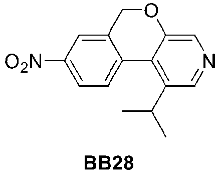

**Intermediate BB28-B** 8-nitro-1-(prop-1-en-2-yl)-6H-isochromeno[3,4-c]pyridine (8 mg) was dissolved in 3 mL of methanol, 2 mg of Pd/C was carefully added, hydrogen balloon displacement was performed 3 times, and the mixture was stirred at room temperature for 24 hours. LCMS showed that 30% of the intermediate remained. The mixture was heated to 45°C and continued stirring for 8 hours, and LCMS showed the reaction had proceeded completely. The mixture was filtered directly to remove palladium on carbon, the filtrate was concentrated and purified by preparative thin layer chromatography (developing solvent: petroleum ether/ethyl acetate = 3/2, dichloromethane/methanol = 30/1) to obtain 3 mg of off-white solid **intermediate BB28** 1-isopropyl-8-nitro-6H-isochromeno[3,4-c]pyridine, with a yield of 30%.

LC_MS: (ES⁺): m/z 241.15 [M+H]⁺.

¹H NMR (400 MHz, CD3OD): δ 8.18 (s, 1H), 7.99 (s, 1H), 7.48 (d, *J =* 8.4 Hz, 1H), 6.76-6.73 (m, 1H), 6.61 (d, *J =* 2.4 Hz, 1H), 4.86 (s, 2H), 3.71-3.64 (m, 1H), 1.39 (d, *J =* 6.8 Hz, 6H).

### Example 1: Synthesis Route of Compound 1

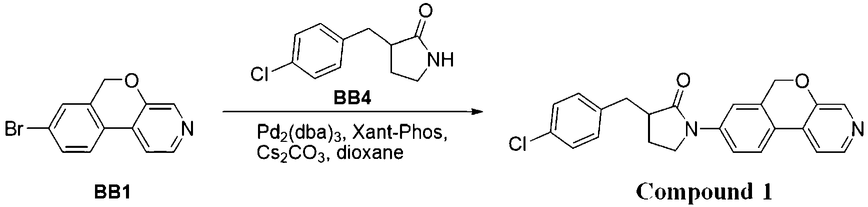

### Step 1: Compound 42 3-(4-chlorobenzyl)-1-(6H-isochromeno[3,4-c]pyridin-8-yl)pyrrolidin-2-one

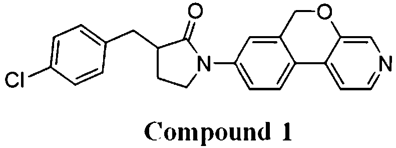

Under nitrogen protection, Pd₂(dba)₃-CHCl₃ (7.82 mg, 0.008 mmol) was added to 1,4-dioxane (3 mL) containing **intermediate BB1** 8-bromo-6H-isochromeno[3,4-c]pyridine (40 mg, 0.153 mmol), **intermediate BB4** 3-(4-chlorobenzyl)pyrrolidin-2-one (35.2 mg, 0.168 mmol), Xant-Phos (9.72 mg, 0.0168 mmol), and cesium carbonate (149.5 mg, 0.459 mmol), the reaction system was subjected to nitrogen displacement 3 times, and heated to 100°C to react for 2 hours. TLC monitoring showed the reaction was complete. The reaction mixture was extracted with ethyl acetate (5 mL x 2) and water (10 mL), the organic phases were combined, dried over anhydrous sodium sulfate, concentrated under reduced pressure, and the resulting crude product was separated and purified by preparative thin layer chromatography (eluent: a 4.76% methanol solution in dichloromethane) to obtain white solid **Compound 1** 3-(4-chlorobenzyl)-1-(6H-isochromeno[3,4-c]pyridin-8-yl)pyrrolidin-2-one (38 mg).

LC_MS: (ES⁺): m/z 391.1 [M+H]⁺.

¹H NMR (400 MHz, CDCl₃) δ 8.35 (s, 1H), 8.30 (d, *J =* 4.4 Hz, 1H), 7.75 (d, *J =* 8.6 Hz, 1H), 7.70 (d, *J =* 2.0 Hz, 1H), 7.61 - 7.55 (m, 2H), 7.33 - 7.29 (m, 2H), 7.20 (d, *J =* 8.4 Hz, 1H), 5.23 (s, 2H), 3.83 - 3.75 (m, 1H), 3.68 (td, *J =* 9.1, 3.1 Hz, 1H), 3.27 (dd, *J =* 13.7, 4.2 Hz, 1H), 2.96 (ddd, *J =* 17.8, 8.9, 4.2 Hz, 1H), 2.84 (dd, *J =* 13.7, 8.9 Hz, 1H), 2.32 - 2.20 (m, 1H), 1.90 (dt, *J =* 8.8, 6.9 Hz, 1H).

### Example 2: Synthesis Route of Compound 2

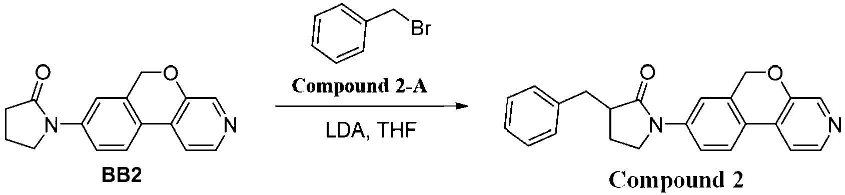

### Step 1: Compound 42 3-benzyl-1-(6H-isochromeno[3,4-c]pyridin-8-yl)pyrrolidin-2-one

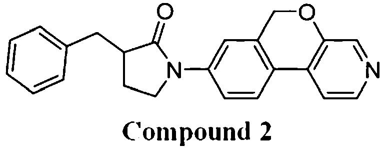

**Intermediate BB2** 1-(6H-isochromeno[3,4-c]pyridin-8-yl)pyrrolidin-2-one (50 mg, 0.188 mmol) was dissolved in anhydrous tetrahydrofuran (5 mL), the system was cooled to -78°C under nitrogen protection, LDA (2M in THF, 0.141 mL) was slowly added dropwise, after stirring for half an hour, a THF (5 mL) solution containing compound 2-A benzyl bromide (32.1 mg, 0.188 mmol) was injected along the wall of the reaction vessel in one portion using a syringe, and the reaction was continued at -78°C for 1 hour. TLC monitoring showed the reaction was complete, the reaction was quenched with saturated ammonium chloride solution (10 mL), extracted with ethyl acetate (10 mL x 2), the organic layers were combined, washed with saturated brine (20 mL), dried over anhydrous sodium sulfate, concentrated under reduced pressure, and the residue was purified by Pre-TLC (eluted with a 5% methanol solution in dichloromethane) to obtain white solid **Compound 2** 3-benzyl-1-(6H-isochromeno[3,4-c]pyridin-8-yl)pyrrolidin-2-one (18 mg).

LC_MS: (ES⁺): m/z 357.50 [M+H]⁺

¹H NMR (400 MHz, CDCl₃) δ 8.33 (s, 1H), 8.28 (d, *J* = 5.1 Hz, 1H), 7.78 - 7.64 (m, 2H), 7.59 - 7.46 (m, 2H), 7.34 - 7.21 (m, 5H), 5.2 (s, 2H), 3.88 - 3.69 (m, 1H), 3.64 (td, J1 = 9.0 Hz, J2 = 3.4 Hz, 1H), 3.31 (dd, J1 = 13.7 Hz, J2 = 4.2 Hz, 1H), 2.96 (qd, J1 = 8.9 Hz, J2 = 4.1 Hz, 1H), 2.82 (dd, J1 = 13.7 Hz, J2 = 9.2 Hz, 1H), 2.26-2.18 (m, 1H), 1.90 (dq, J1 = 12.8 Hz, J2 = 8.6 Hz, 1H).

### Example 3: Synthesis Route of Compound 42

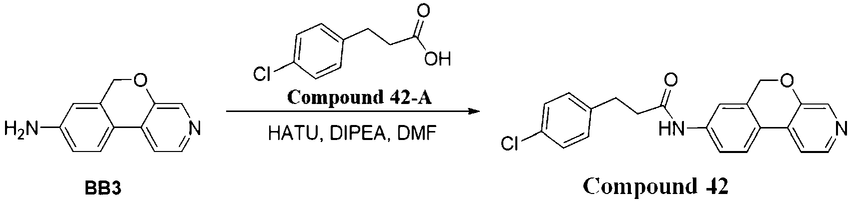

### Step 1: Compound 42 3-(4-chlorophenyl)-N-(6H-isochromeno[3,4-c]pyridin-8-yl)propanamide

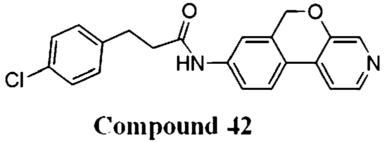

At room temperature, HATU (69.1 mg, 0.182 mmol) was added to a solution containing **intermediate BB3** 6H-isochromeno[3,4-c]pyridin-8-amine (30 mg, 0.151 mmol), **compound 42-A** (33.5 mg, 0.182 mmol), and N,N-diisopropylethylamine (58.7 mg, 0.454 mmol) in DMF (1 mL). The reaction mixture was stirred at room temperature for 3 hours. LCMS monitoring showed the reaction was complete. Water (5 mL) was added for dilution, and the mixture was extracted with ethyl acetate (5 mL x 2). The organic layers were combined, washed with saturated brine (10 mL), dried over anhydrous sodium sulfate, and concentrated under reduced pressure. The residue was separated and purified by Pre-TLC (eluting with a dichloromethane solution containing 5% methanol) to obtain white solid **compound 42** 3-(4-chlorophenyl)-N-(6H-isochromeno[3,4-c]pyridin-8-yl)propanamide (46 mg).

LC_MS: (ES⁺): m/z 365.40 [M+H]⁺.

¹H NMR (400 MHz, DMSO) δ 10.18 (s, 1H), 8.27 - 8.20 (m, 2H), 7.89 (d, *J=* 8.5 Hz, 1H), 7.76 (d, *J =* 5.0 Hz, 1H), 7.66 - 7.53 (m, 2H), 7.37 - 7.33 (m, 2H), 7.29 (d, *J =* 8.5 Hz, 2H), 5.21 (s, 2H), 2.92 (t, *J =* 7.5 Hz, 2H), 2.66 (t, *J =* 7.6 Hz, 2H).

### Example 4: Synthesis Route of Compound 10

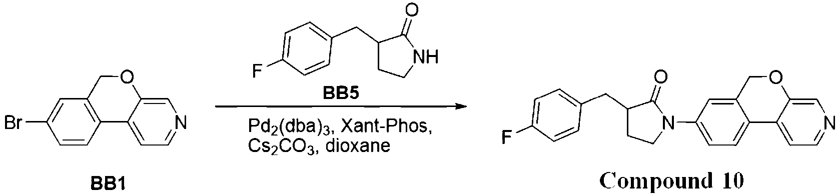

### Step 1: Compound 10 3-(4-fluorobenzyl)-1-(6H-isochromeno[3,4-c]pyridin-8-yl)pyrrolidin-2-one

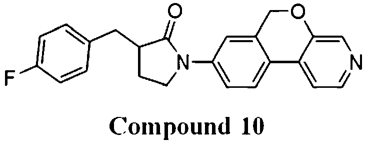

The synthesis of this step was performed using **intermediate BB1** 8-bromo-6H-isochromeno[3,4-c]pyridine and **intermediate BB5** 3-(4-fluorobenzyl)pyrrolidin-2-one as starting materials following Step 1 of the synthesis route of Example 1.

LC_MS: (ES⁺): m/z 375.2 [M+H]⁺.

¹H NMR (400 MHz, DMSO-d6) δ 8.33 - 8.21 (m, 2H), 7.97 (d, *J =* 8.6 Hz, 1H), 7.86 - 7.77 (m, 2H), 7.67 (d, *J =* 2.2 Hz, 1H), 7.37 - 7.26 (m, 2H), 7.18 - 7.07 (m, 2H), 5.25 (s, 2H), 3.74 (dtd, *J =* 18.3, 9.5, 6.9 Hz, 2H), 3.12 (dd, *J =* 13.6, 4.4 Hz, 1H), 2.97 (qd, *J =* 9.2, 4.4 Hz, 1H), 2.74 (dd, *J =* 13.7, 9.3 Hz, 1H), 2.08 (ddt, *J* = 9.8, 7.3, 5.2 Hz, 1H), 1.78 (dq, *J =* 12.1, 8.8 Hz, 1H).

### Example 5: Synthesis Route of Compound 45

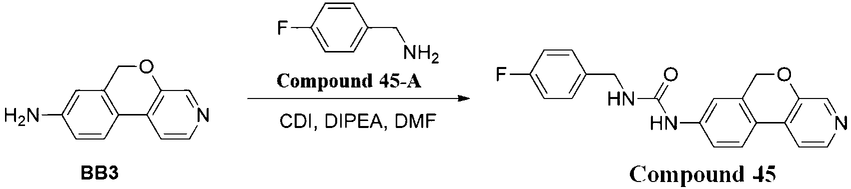

### Step 1: Compound 45 1-(4-fluorobenzyl)-3-(6H-isochromeno[3,4-c]pyridin-8-yl)urea

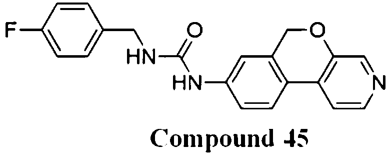

CDI (24.5 mg, 0.151 mmol) and DIPEA (58.5 mg, 0.453 mmol) were added to a DMF (0.5 mL) solution containing **intermediate BB3** 6H-isochromeno[3,4-c]pyridin-8-amine (30 mg, 0.151 mmol). After stirring at room temperature for 30 minutes, **compound 45-A** (4-fluorophenyl)methanamine (18.9 mg, 0.151 mmol) was added, and the mixture was stirred at room temperature for 16 hours. After the reaction was completed, ice water was added dropwise for dilution. The precipitated solid was collected by filtration, washed with water, and the filter cake was dissolved in DCM/MeOH=10:1 (2 mL). The solution was separated and purified by preparative thin layer chromatography (eluted with a dichloromethane solution containing 5% methanol) to obtain **compound 45** 1-(4-fluorobenzyl)-3-(6H-isochromeno[3,4-c]pyridin-8-yl)urea (18 mg).

LC_MS: (ES⁺): m/z 350.1 [M+H]⁺.

¹H NMR (400 MHz, Chloroform-d) δ 8.32 (s, 1H), 8.27 (d, *J =* 5.1 Hz, 1H), 7.66 (d, *J =* 8.4 Hz, 1H), 7.50 (s, 1H), 7.43 (d, *J =* 2.2 Hz, 1H), 7.33 (dd, *J =* 8.5, 5.4 Hz, 2H), 7.24 (dd, *J =* 8.5, 2.3 Hz, 1H), 7.06 (t, *J =* 8.6 Hz, 2H), 6.57 (s, 1H), 5.17 (s, 2H), 5.08 (s, 1H), 4.46 (d, *J* = 5.7 Hz, 2H). MS: [M+H]⁺: 350.1.

### Example 6: Synthesis Route of Compound 49

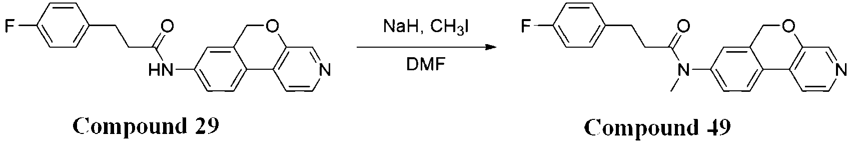

### Step 1: Compound 49 3-(4-fluorophenyl)-N-(6H-isochromeno[3,4-c]pyridin-8-yl)-N-methylpropanamide

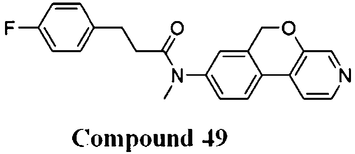

Under an ice bath, NaH (2.76 mg, 0.0689 mmol, 60% purity) was added to a solution containing **compound 29** 3-(4-fluorophenyl)-N-(6H-isochromeno[3,4-c]pyridin-8-yl)propanamide (20 mg, 0.0574 mmol) in DMF (1 mL). After stirring for half an hour, iodomethane (9.8 mg, 0.0689 mmol) was added, and the mixture was warmed to room temperature and stirred for 12 hours. LCMS monitoring showed the reaction was complete. The reaction mixture was poured into a saturated ammonium chloride solution (5 mL) and extracted with ethyl acetate (5 mL x 2). The organic layers were combined, washed with saturated brine (10 mL), dried over anhydrous sodium sulfate, and concentrated under reduced pressure. The residue was separated and purified by Pre-TLC (eluted with a dichloromethane solution containing 5% methanol) to obtain white solid **compound 49** 3-(4-fluorophenyl)-N-(6H-isochromeno[3,4-c]pyridin-8-yl)-N-methylpropanamide (15 mg).

LC_MS: (ES⁺): m/z 363.12 [M+H]⁺.

### Example 7: Synthesis Route of Compound 52

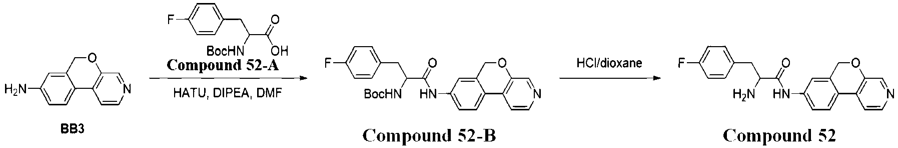

### Step 1: Compound 52-B tert-butyl (1-((6H-isochromeno[3,4-c]pyridin-8-yl)amino)-3-(4-fluorophenyl)-1-oxopropan-2-yl)carbamate

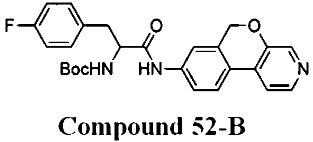

The synthesis of this step was performed using **compound 52-A** 2-((tert-butoxycarbonyl)amino)-3-(4-fluorophenyl)propanoic acid and **intermediate BB3** 6H-isochromeno[3,4-c]pyridin-8-amine as starting materials following Step 1 of the synthesis route of Example 3.

LC_MS: (ES⁺): m/z 464.2 [M+H]⁺.

### Step 2: Compound 52 2-amino-3-(4-fluorophenyl)-N-(6H-isochromeno[3,4-c]pyridin-8-yl)propanamide

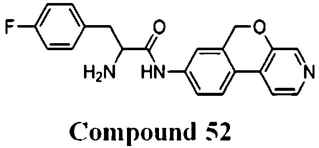

A solution containing **compound 52-B** tert-butyl (1-((6H-isochromeno[3,4-c]pyridin-8-yl)amino)-3-(4-fluorophenyl)-1-oxopropan-2-yl)carbamate (25 mg, 0.0539 mmol) in HCl/dioxane (1 mL, 4M) was stirred at room temperature for 1 hour. LCMS monitoring showed the reaction was complete. The mixture was concentrated under reduced pressure to obtain white solid hydrochloride **compound 52** 2-amino-3-(4-fluorophenyl)-N-(6H-isochromeno[3,4-c]pyridin-8-yl)propanamide (18 mg).

LC_MS: (ES⁺): m/z 364.2 [M+H]⁺.

¹H NMR (400 MHz, MeOH-d₄): δ 8.52 (d, *J =* 0.8 Hz, 1H), 8.49 - 8.37 (m, 2H), 8.12 (d, *J =* 8.5 Hz, 1H), 7.83 - 7.69 (m, 2H), 7.47 - 7.33 (m, 2H), 7.25 - 7.05 (m, 2H), 5.49 (s, 2H), 4.32 (t, *J =* 7.3 Hz, 1H), 3.48 - 3.34 (m, 1H), 3.20 (dd, *J* ₁= 14.0 Hz, J₂ = 7.9 Hz, 1H).

### Example 8: Synthesis Route of Compound 53

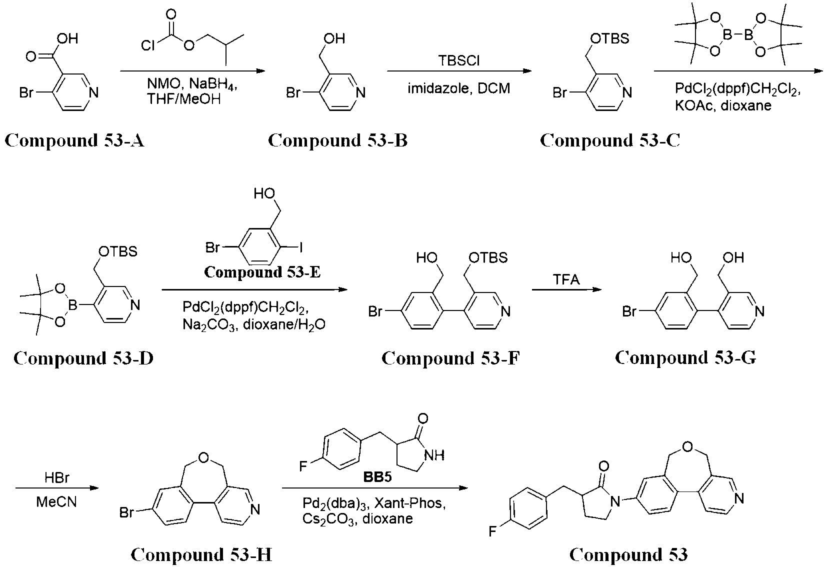

### Step 1: Compound 53-B (4-bromopyridin-3-yl)methanol

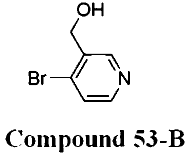

Under an ice bath, isobutyl chloroformate (3.24 g, 23.761 mmol) was added to a solution containing **compound 53-A** 4-bromonicotinic acid (4.8 g, 23.761 mmol) and N-methylmorpholine (2.90 g, 28.513 mmol) in THF (50 mL). After stirring for half an hour, the solid produced in the reaction was filtered off. The filtrate was dispersed in methanol (20 mL), NaBH₄ (1.35 g, 35.642 mmol) was added, and the mixture was stirred at room temperature for 1 hour. LCMS monitoring showed the reaction was complete. The reaction mixture was concentrated under reduced pressure, water (30 mL) was added, and the mixture was extracted with ethyl acetate (30 mL x 2). The organic layers were combined, washed with saturated brine (30 mL), dried over anhydrous sodium sulfate, filtered, and concentrated under reduced pressure. The residue was purified by silica gel column chromatography (eluted with a petroleum ether solution containing 25% ethyl acetate) to obtain a yellow solid **compound 53-B** (4-bromopyridin-3-yl)methanol (860 mg).

LC_MS: (ES⁺): m/z 188.02 [M+H]⁺.

### Step 2: Compound 53-C 4-bromo-3-(((tert-butyldimethylsilyl)oxy)methyl)pyridine

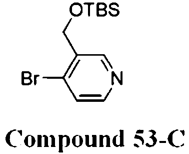

TBSCl (1.378 g, 9.148 mmol) was added to a solution containing **compound 53-B** (4-bromopyridin-3-yl)methanol (860 mg, 4.574 mmol) and imidazole (934.2 mg, 13.722 mmol) in DCM (20 mL). The mixture was stirred at room temperature for 12 hours. TLC monitoring showed the reaction was complete. Water (20 mL) was added to the reaction mixture, and the mixture was extracted with DCM (15 mL x 2). The organic layers were combined, washed with saturated brine (20 mL), dried over anhydrous sodium sulfate, filtered, and concentrated under reduced pressure. The residue was purified by silica gel column chromatography (eluted with a petroleum ether solution containing 2-4% ethyl acetate) to obtain a colorless oil **compound 53-C** 4-bromo-3-(((tert-butyldimethylsilyl)oxy)methyl)pyridine (1.39 g).

LC_MS: (ES⁺): m/z 302.03 [M+H]⁺.

### Step 3: Compound 53-D 3-(((tert-butyldimethylsilyl)oxy)methyl)-4-(4,4,5,5-tetramethyl-1,3,2-dioxaborolan-2-yl)pyridine

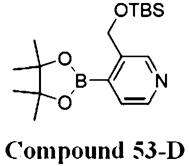

PdCl₂(dppf)CH₂Cl₂ (348.5 mg, 0.4267 mmol) and potassium acetate (1.26 g, 12.802 mmol) were added to a solution of **compound 53-C** 4-bromo-3-(((tert-butyldimethylsilyl)oxy)methyl)pyridine (1.29 g, 4.267 mmol) and bis(pinacolato)diboron (2.17 g, 8.535 mmol) in 1,4-dioxane (20 mL). The reaction mixture was stirred at 100°C under a nitrogen atmosphere for 2 hours. LCMS monitored that the reaction was complete. The reaction mixture was filtered through celite and concentrated under reduced pressure to obtain **compound 53-D** 3-(((tert-butyldimethylsilyl)oxy)methyl)-4-(4,4,5,5-tetramethyl-1,3,2-dioxaborolan-2-yl)pyridine (1.491 g) as a yellow oil, and the resulting crude product was used directly in the next step without further purification.

LC_MS: (ES⁺): m/z 268.12 [M-82+H] +.

### Step 4: Compound 53-F (5-bromo-2-(3-(((tert-butyldimethylsilyl)oxy)methyl)pyridin-4-yl)phenyl)methanol

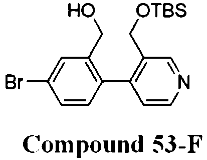

PdCl₂(dppf)CH₂Cl₂ (348.5 mg, 0.4267 mmol) and sodium carbonate (1.13 g, 10.668 mmol) were added to a solution of **compound 53-D** 3-(((tert-butyldimethylsilyl)oxy)methyl)-4-(4,4,5,5-tetramethyl-1,3,2-dioxaborolan-2-yl)pyridine (1.491 g, 4.267 mmol) and **compound 53-E** (5-bromo-2-iodophenyl)methanol (1.335 g, 4.267 mmol) in 1,4-dioxane (20 mL) and water (2 mL). The reaction mixture was stirred at 80°C under a nitrogen atmosphere for 12 hours. LCMS monitored that the reaction was complete. The reaction mixture was filtered through celite and concentrated under reduced pressure, and the residue was purified by silica gel column chromatography (eluted with a petroleum ether solution containing 50% ethyl acetate) to obtain crude **compound 53-F** (5-bromo-2-(3-(((tert-butyldimethylsilyl)oxy)methyl)pyridin-4-yl)phenyl)methanol (500 mg) as a yellow oil.

LC_MS: (ES⁺): m/z 408.10 [M+H]⁺.

### Step 5: Compound 53-G (5-bromo-2-(3-(hydroxymethyl)pyridin-4-yl)phenyl)methanol

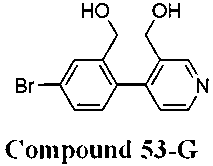

A solution of **compound 53-F** (5-bromo-2-(3-(((tert-butyldimethylsilyl)oxy)methyl)pyridin-4-yl)phenyl)methanol (450 mg, 1.1 mmol) in trifluoroacetic acid (10 mL) was stirred at room temperature for 2 hours. LCMS monitored that the reaction was complete. The reaction mixture was concentrated under reduced pressure to remove trifluoroacetic acid, saturated NaHCO₃ solution (20 mL) was added, and the mixture was extracted with ethyl acetate (15 mL x 2). The organic layers were combined, washed with saturated brine (20 mL), dried over anhydrous sodium sulfate, filtered, and concentrated under reduced pressure. The residue was purified by silica gel column chromatography (eluted with a dichloromethane solution containing 5% methanol) and Prep-HPLC to obtain **compound** 53-G (5-bromo-2-(3-(hydroxymethyl)pyridin-4-yl)phenyl)methanol (60 mg) as a yellow oil.

LC_MS: (ES⁺): m/z 294.00 [M+H]⁺.

### Step 6: Compound 53-H 9-bromo-5,7-dihydrobenzo[5,6]oxepino[3,4-c]pyridine

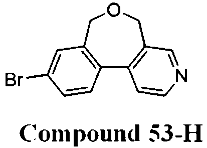

48% hydrobromic acid (0.1 mL) was added to a solution of **compound 53-G** (5-bromo-2-(3-(hydroxymethyl)pyridin-4-yl)phenyl)methanol (50 mg, 169.98 umol) in acetonitrile (2.5 mL). The reaction mixture was stirred at 80°C for 1 hour. LCMS monitored that the reaction was complete. The reaction mixture was adjusted to pH=9 with saturated sodium bicarbonate solution and extracted with ethyl acetate (8 mL x 2). The organic layers were combined, washed with saturated brine (10 mL), dried over anhydrous sodium sulfate, filtered, and concentrated under reduced pressure. The residue was purified by Pre-TLC (eluted with a dichloromethane solution containing 5% methanol) to obtain **compound 53-H** 9-bromo-5,7-dihydrobenzo[5,6]oxepino[3,4-c]pyridine (5 mg) as a yellow oil.

LC_MS: (ES⁺): m/z 276.10 [M+H]⁺.

### Step 7: Compound 53 1-(5,7-dihydrobenzo[5,6]oxepino[3,4-c]pyridin-9-yl)-3-(4-fluorobenzyl)pyrrolidin-2-one

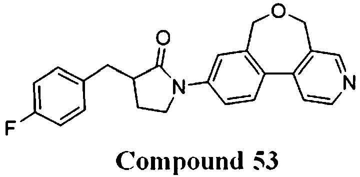

The synthesis of this step was performed using **compound 53-H** 9-bromo-5,7-dihydrobenzo[5,6]oxepino[3,4-c]pyridine and **intermediate BB5** 3-(4-fluorobenzyl)pyrrolidin-2-one as starting materials following Step 1 of the synthesis route of Example 1.

LC_MS: (ES⁺): m/z 389.18 [M+H]⁺.

### Example 9: Synthesis Route of Compound 91

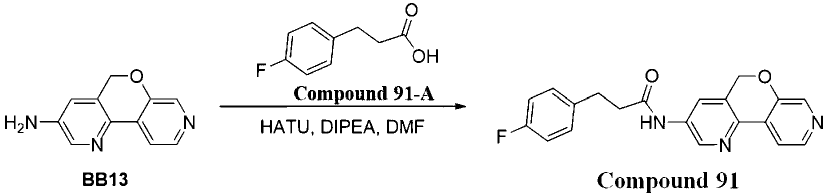

### Step 1: Compound 91 3-(4-fluorophenyl)-N-(5H-pyrano[4,5-b:2,3-c']dipyridin-3-yl)propanamide

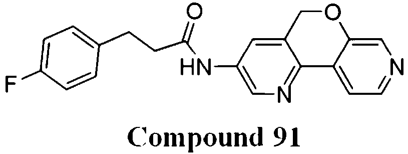

The synthesis of this step was performed using **intermediate BB13** 5H-pyrano[4,5-b:2,3-c']dipyridin-3-amine and **compound 91-A** 3-(4-fluorophenyl)propanoic acid as starting materials following Step 1 of the synthesis route of Example 3.

LC_MS: (ES⁺): m/z 350.1 [M+H]⁺.

### Example 10: Synthesis Route of Compound 112

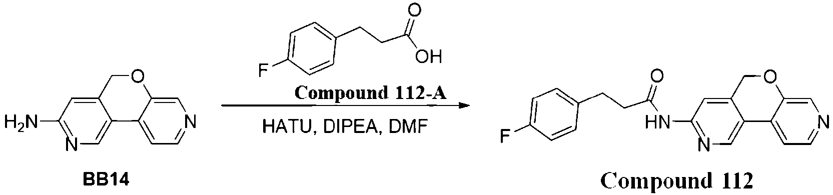

### Step 1: Compound 112 3-(4-fluorophenyl)-N-(5H-pyrano[2,3-c:4,5-c']dipyridin-3-yl)propanamide

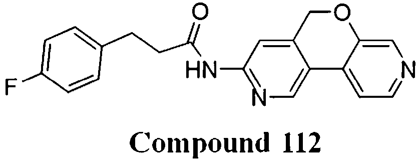

The synthesis of this step was performed using **intermediate BB14** 5H-pyrano[2,3-c:4,5-c']dipyridin-3-amine and **compound 112-A** 3-(4-fluorophenyl)propanoic acid as starting materials following Step 1 of the synthesis route of Example 3, .

LC_MS: (ES⁺): m/z 350.1 [M+H]⁺.

### Example 11: Synthesis Route of Compound 77

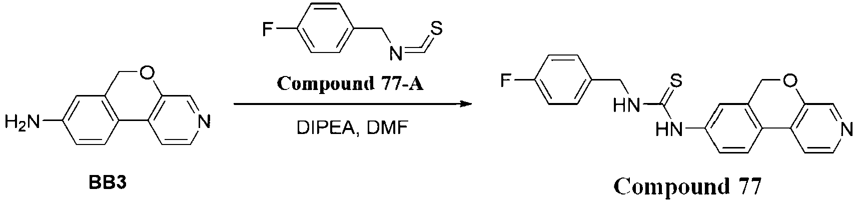

### Step 1: Compound 77 1-(4-fluorobenzyl)-3-(6H-isochromeno[3,4-c]pyridin-8-yl)thiourea

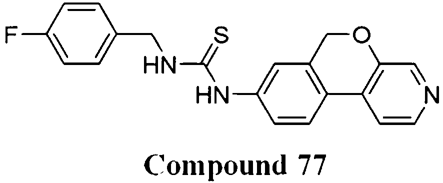

**Compound 77-A** 4-fluorobenzyl thiocyanate (25.3 mg, 151.34 umol) was added to a solution of **intermediate BB3** 6H-isochromeno[3,4-c]pyridin-8-amine (30 mg, 151.34 umol) and N,N-diisopropylethylamine (58.7 mg, 454.03 umol) in DMF (1 mL). The reaction mixture was heated to 100°C and stirred for 12 hours. LCMS monitored that the reaction was complete. The reaction mixture was diluted with water (10 mL) and extracted with ethyl acetate (8 mL x 2). The organic layers were combined, washed with saturated brine (10 mL), dried over anhydrous sodium sulfate, filtered, and concentrated under reduced pressure. The residue was purified by Pre-TLC (eluted with a dichloromethane solution containing 5% methanol) to obtain **compound 77** 1-(4-fluorobenzyl)-3-(6H-isochromeno[3,4-c]pyridin-8-yl)thiourea (19.3 mg) as a yellow solid.

LC_MS: (ES⁺): m/z 366.9 [M+H]⁺.

¹H NMR (400 MHz, DMSO) δ 9.87 (s, 1H), 8.40 (s, 1H), 8.27 (s, 1H), 8.24 (d, *J =* 5.0 Hz, 1H), 7.91 (d, *J =* 8.4 Hz, 1H), 7.79 (d, *J =* 5.1 Hz, 1H), 7.53 (dd, *J =* 8.4, 1.7 Hz, 1H), 7.48 (s, 1H), 7.40 (dd, *J =* 8.4, 5.7 Hz, 2H), 7.18 (t, *J =* 8.8 Hz, 2H), 5.22 (s, 2H), 4.74 (d, *J =* 4.6 Hz, 2H).

### Example 12: Synthesis Route of Compound 101

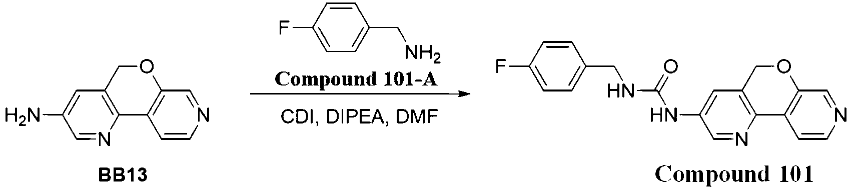

### Step 1: Compound 101 1-(4-fluorobenzyl)-3-(5H-pyrano[4,5-b:2,3-c']dipyridin-3-yl)urea

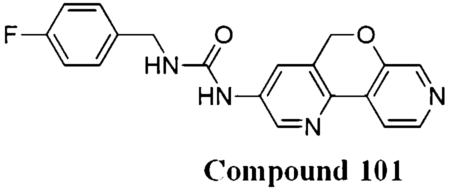

The synthesis of this step was performed using **intermediate BB13** 5H-pyrano[4,5-b:2,3-c']dipyridin-3-amine and **compound 101-A** (4-fluorophenyl)methanamine as starting materials following Step 1 of the synthesis route of Example 5.

LC_MS: (ES⁺): m/z 351.1 [M+H]⁺.

### Example 13: Synthesis Route of Compound 122

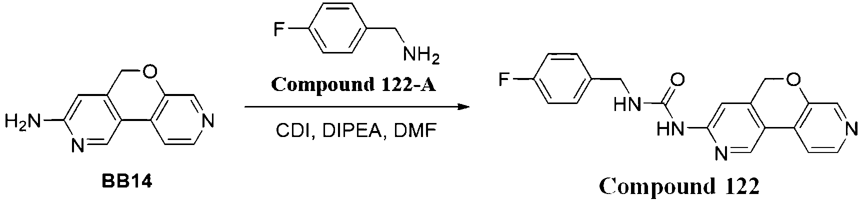

### Step 1: Compound 122 1-(4-fluorobenzyl)-3-(5H-pyrano[2,3-c:4,5-c']dipyridin-3-yl)urea

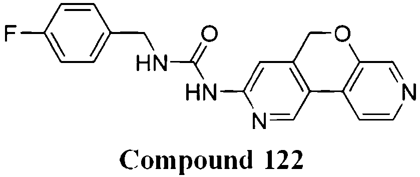

The synthesis of this step was performed with **intermediate BB14** 5H-pyrano[2,3-c:4,5-c']dipyridin-3-amine and **compound 122-A** (4-fluorophenyl)methanamine as starting materials following Step 1 of the synthesis route of Example 5.

LC_MS: (ES⁺): m/z 351.1 [M+H]⁺.

### Example 14: Synthesis Route of Compound 130

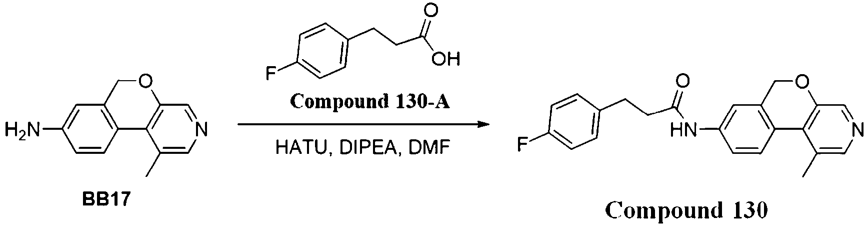

### Step 1: Compound 130 3-(4-fluorophenyl)-N-(1-methyl-6H-isochromeno[3,4-c]pyridin-8-yl)propanamide

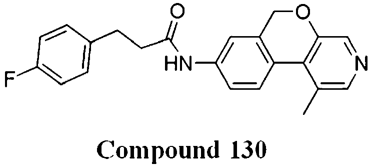

The synthesis of this step was performed with **intermediate BB17** 1-methyl-6H-isochromeno[3,4-c]pyridin-8-amine and **compound 130-A** 3-(4-fluorophenyl)propanoic acid as starting materials following Step **1** of the synthesis route of Example 3.

LC_MS: (ES⁺): m/z 363.1 [M+H]⁺.

### Example 15: Synthesis Route of Compound 131

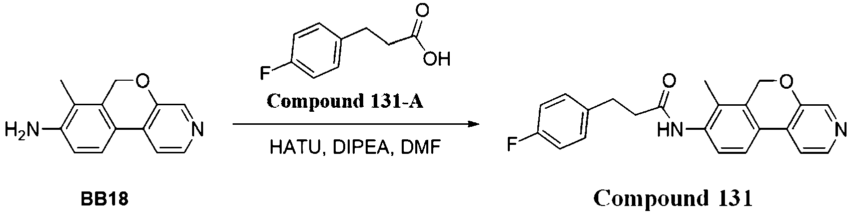

### Step 1: Compound 131 3-(4-fluorophenyl)-N-(7-methyl-6H-isochromeno[3,4-c]pyridin-8-yl)propanamide

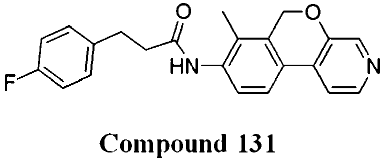

The synthesis of this step was performed with **intermediate BB18** 7-methyl-6H-isochromeno[3,4-c]pyridin-8-amine and **compound 131-A** 3-(4-fluorophenyl)propanoic acid as starting materials following Step 1 of the synthesis route of Example 3.

LC_MS: (ES⁺): m/z 363.1 [M+H]⁺.

### Example 16: Synthesis Route of Compound 139

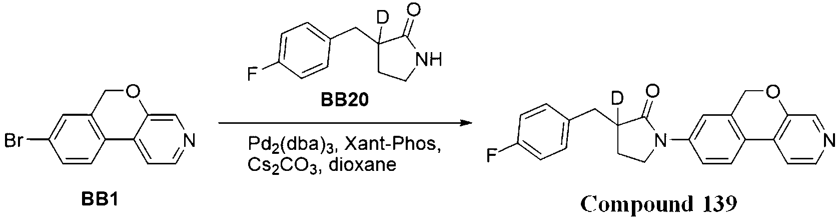

### Step 1: Compound 139 3-(4-fluorobenzyl)-1-(6H-isochromeno[3,4-c]pyridin-8-yl)pyrrolidin-2-one-3d

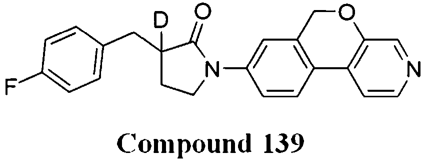

The synthesis of this step was performed with **intermediate BB1** 8-bromo-6H-isochromeno[3,4-c]pyridine and **intermediate BB20** 3-(4-fluorobenzyl)pyrrolidin-2-one-3-d as starting materials following Step 1 of the synthesis route of Example 1.

LC_MS: (ES⁺): m/z 376.2 [M+H]⁺.

¹H NMR (400 MHz, CDCl₃) δ 8.35 (s, 1H), 8.30 (d, *J =* 5.0 Hz, 1H), 7.74 (d, *J =* 8.6 Hz, 1H), 7.70 (d, *J* = 2.0 Hz, 1H), 7.59 - 7.53 (m, 2H), 7.26 - 7.20 (m, 2H), 7.06 - 6.99 (m, 2H), 5.22 (s, 2H), 3.77 (dt, *J =* 15.9, 7.9 Hz, 1H), 3.66 (td, *J =* 9.1, 3.3 Hz, 1H), 3.27 (dd, *J =* 13.8, 6.2 Hz, 1H), 2.85 (dd, *J =* 13.8, 5.5 Hz, 1H), 2.28 - 2.19 (m, 1H), 1.95 - 1.85 (m, 1H).

### Example 17: Synthesis Route of Compound 142

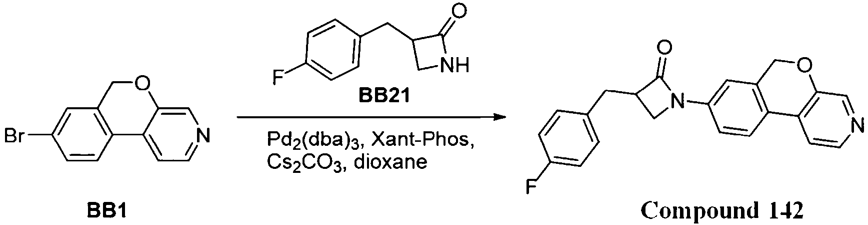

### Step 1: Compound 142 3-(4-fluorobenzyl)-1-(6H-isochromeno[3,4-c]pyridin-8-yl)azetidin-2-one

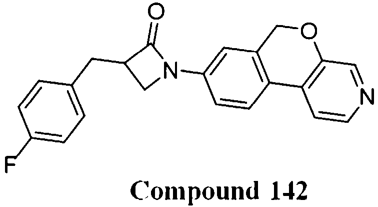

The synthesis of this step was performed with **intermediate BB1** 8-bromo-6H-isochromeno[3,4-c]pyridine and **intermediate BB21** 3-[(4-fluorophenyl)methyl]azetidin-2-one as starting materials following Step 1 of the synthesis route of Example 1.

LC_MS: (ES⁺): m/z 361.1 [M+H]⁺.

¹H NMR (400 MHz, CDCl₃) δ 8.34 (s, 1H), 8.29 (d, *J =* 5.0 Hz, 1H), 7.71 (d, *J* = 8.3 Hz, 1H), 7.51 (d, *J =* 5.0 Hz, 1H), 7.29 - 7.20 (m, 4H), 7.03 (t, *J =* 8.6 Hz, 2H), 5.18 (s, 2H), 3.77 (t, *J =* 5.7 Hz, 1H), 3.67 (dtd, *J =* 8.4, 5.6, 2.8 Hz, 1H), 3.42 (dd, *J* = 5.8, 2.6 Hz, 1H), 3.24 (dd, *J* = 14.6, 5.6 Hz, 1H), 3.05 (dd, *J =* 14.6, 8.8 Hz, 1H).

### Example 18: Synthesis Route of Compound 145

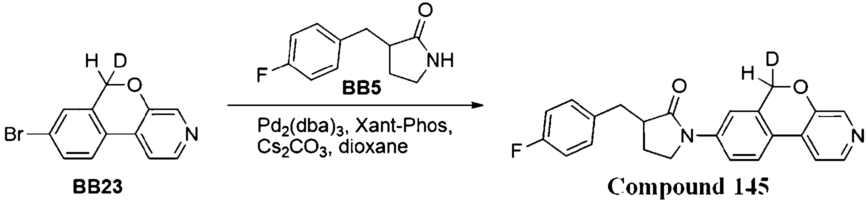

### Step 1: Compound 145 3-(4-fluorobenzyl)-1-(6H-isochromeno[3,4-c]pyridin-8-yl-6-d)pyrrolidin-2-one

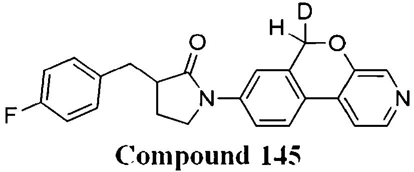

The synthesis of this step was performed with **intermediate BB23** 8-bromo-6H-isochromeno[3,4-c]pyridine-6-d and **intermediate BB5** 3-(4-fluorobenzyl)pyrrolidin-2-one as starting materials following Step 1 of the synthesis route of Example 1.

LC_MS: (ES⁺): m/z 376.1 [M+H]⁺.

¹H NMR (400 MHz, DMSO) δ 8.28 (s, 1H), 8.25 (d, *J =* 5.0 Hz, 1H), 7.97 (d, *J =* 8.6 Hz, 1H), 7.80 (dd, *J =* 12.2, 3.4 Hz, 2H), 7.67 (s, 1H), 7.32 (dd, *J =* 8.1, 5.8 Hz, 2H), 7.13 (t, *J =* 8.8 Hz, 2H), 5.22 (s, 1H), 3.81 - 3.65 (m, 2H), 3.12 (dd, *J =* 13.6, 4.1 Hz, 1H), 3.02 - 2.91 (m, 1H), 2.74 (dd, *J =* 13.5, 9.4 Hz, 1H), 2.15 - 2.02 (m, 1H), 1.85 - 1.71 (m, 1H).

### Example 19: Synthesis Route of Compound 146

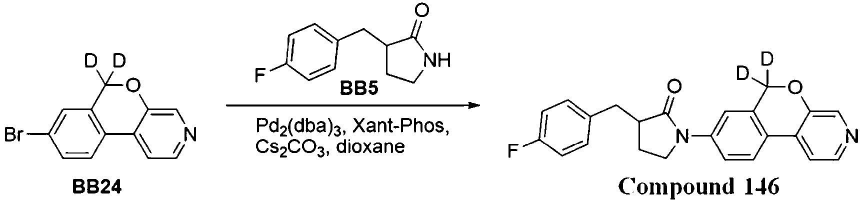

### Step 1: Compound 146 3-(4-fluorobenzyl)-1-(6H-isochromeno[3,4-c]pyridin-8-yl-6,6-d2)pyrrolidin-2-one

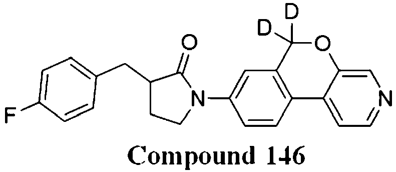

The synthesis of this step was performed using **intermediate BB24** 8-bromo-6H-isochromeno[3,4-c]pyridine-6,6-d2 and **intermediate BB5** 3-(4-fluorobenzyl)pyrrolidin-2-one as starting materials following Step 1 of the synthesis route of Example 1.

LC_MS: (ES⁺): m/z 377.1 [M+H]⁺.

¹H NMR (400 MHz, CDCl₃) δ 8.35 (s, 1H), 8.29 (d, *J =* 5.0 Hz, 1H), 7.74 (d, *J* = 8.5 Hz, 1H), 7.69 (d, *J =* 2.0 Hz, 1H), 7.61 - 7.52 (m, 2H), 7.22 (dd, *J =* 8.4, 5.5 Hz, 2H), 7.01 (t, *J =* 8.7 Hz, 2H), 3.76 (dd, *J =* 17.0, 8.3 Hz, 1H), 3.66 (td, *J* = 9.1, 3.2 Hz, 1H), 3.27 (dd, *J* = 13.7, 4.1 Hz, 1H), 3.00 - 2.91 (m, 1H), 2.85 (dd, *J =* 13.7, 8.8 Hz, 1H), 2.24 (ddd, *J =* 11.6, 8.4, 3.9 Hz, 1H), 1.90 (dq, *J* = 12.8, 8.6 Hz, 1H).

### Example 20: Synthesis Route of Compound 147

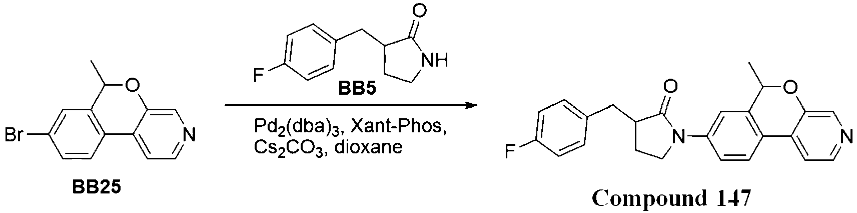

### Step 1: Compound 147 3-(4-fluorobenzyl)-1-(6-methyl-6H-isochromeno[3,4-c]pyridin-8-yl)pyrrolidin-2-one

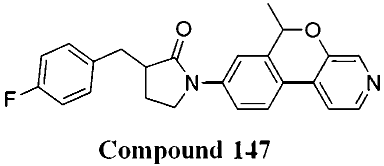

The synthesis of this step was performed using **intermediate BB25** 8-bromo-6-methyl-6H-isochromeno[3,4-c]pyridine and **intermediate BB5** 3-(4-fluorobenzyl)pyrrolidin-2-one as starting materials following Step 1 of the synthesis route of Example 1.

LC_MS: (ES⁺): m/z 389.1 [M+H]⁺.

¹H NMR (400 MHz, DMSO) δ 8.26 (s, 1H), 8.24 (d, *J =* 5.0 Hz, 1H), 7.99 (d, *J =* 8.7 Hz, 1H), 7.83 (d, *J =* 5.1 Hz, 1H), 7.79 (dd, *J =* 8.6, 1.8 Hz, 1H), 7.66 (dd, *J =* 3.8, 2.2 Hz, 1H), 7.36 - 7.29 (m, 2H), 7.13 (t, *J =* 8.7 Hz, 2H), 5.49 (q, *J =* 6.5 Hz, 1H), 3.85 - 3.69 (m, 2H), 3.14 (ddd, *J =* 13.7, 10.2, 5.2 Hz, 1H), 3.04 - 2.91 (m, 1H), 2.73 (ddd, *J =* 13.6, 9.4, 4.0 Hz, 1H), 2.08 (dt, *J =* 9.0, 6.4 Hz, 1H), 1.85 - 1.72 (m, 1H), 1.51 (dd, *J =* 6.5, 3.5 Hz, 3H).

### Example 21: Synthesis Route of Compound 143

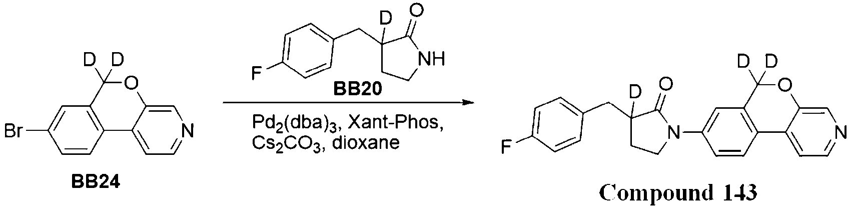

### Step 1: Compound 143 3-(4-fluorobenzyl)-1-(6H-isochromeno[3,4-c]pyridin-8-yl-6,6-d2)pyrrolidin-2-one-3d

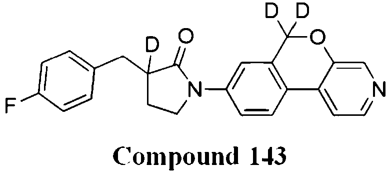

The synthesis of this step was performed using **intermediate BB24** 8-bromo-6H-isochromeno[3,4-c]pyridine-6,6-d2 and **intermediate BB20** 3-(4-fluorobenzyl)pyrrolidin-2-one-3-d as starting materials following Step 1 of the synthesis route of Example 1.

LC_MS: (ES⁺): m/z 378.1 [M+H]⁺.

¹H NMR (400 MHz, CDCl₃) δ 8.35 (s, 1H), 8.29 (d, *J =* 5.0 Hz, 1H), 7.74 (d, *J* = 8.6 Hz, 1H), 7.69 (d, *J =* 2.2 Hz, 1H), 7.59 - 7.52 (m, 2H), 7.22 (dd, *J =* 8.5, 5.5 Hz, 2H), 7.01 (t, *J =* 8.7 Hz, 2H), 3.76 (dd, *J =* 16.7, 8.6 Hz, 1H), 3.66 (td, *J =* 9.3, 3.3 Hz, 1H), 3.27 (dd, *J* = 13.8, 6.2 Hz, 1H), 2.86 (dd, *J =* 13.8, 5.5 Hz, 1H), 2.24 (ddd, *J =* 10.8, 8.6, 5.1 Hz, 1H), 1.95 - 1.83 (m, 1H).

### Example 22: Preparation of Compound 154 and Compound 155

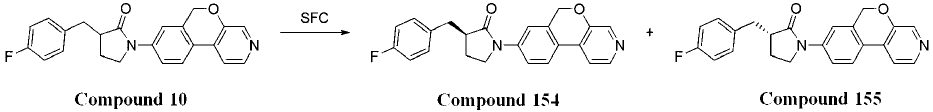

**Compound 10** (50 mg) was dissolved in methanol, and **compound 154** (21.2 mg) and **compound 155** (22.3 mg) were obtained by SFC preparative separation. The separation method was as follows, separation column: Chiral AD-H, pressure: 5 MPa, mobile phase: 35% isopropanol/n-hexane, detection wavelength: 280 nm, flow rate: 2 mL/min, column temperature: 25°C.

**Compound 156** and **compound 157, compound 158** and **compound 159,** and **compound 160** and **compound 161** were also obtained by the above SFC preparative separation method.

The synthesis route of the following compounds is shown below, which were synthesized using corresponding reagents following Step 1 of the synthesis route of Example 2:

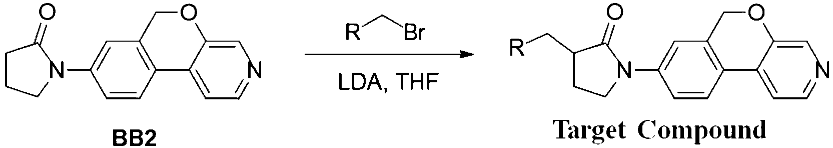

| **Compound Number** | **Structural Formula** | **Mass Spectrum and NMR** |
|---|---|---|
| **Compound 3** | 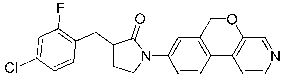 | LC_MS: (ES⁺): m/z 409.45[M+H]⁺. |
| | | ¹H NMR (400 MHz, CDCl₃) δ 8.33 (s, 1H), 8.28 (d, *J* = 5.0 Hz, 1H), 7.73 (d, *J* = 8.6 Hz, 1H), 7.68 (d, *J =* 2.2 Hz, 1H), 7.56 (dd, *J*₁ = 8.5 Hz, *J*₂ = 2.3 Hz, 1H), 7.52 (d, *J =* 5.1 Hz, 1H), 7.22 (d, *J=* 7.9 Hz, 1H), 7.13 - 7.04 (m, 2H), 5.20 (s, 2H), 3.81-3.71 (m, 2H), 3.32-3.28 (m, 1H), 2.96 (qd, *J*₁ = 8.9 Hz, *J*₂ = 4.5 Hz, 1H), 2.87-2.81 (m, 1H), 2.28-2.20 (m, 1H), 1.92-1.82 (m, 1H). |
| **Compound 4** | 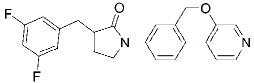 | LC_MS: (ES⁺): m/z 393.20 [M+H]⁺. |
| **Compound 5** | 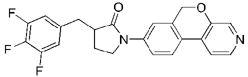 | LC_MS: (ES⁺): m/z 411.40 [M+H]⁺. |
| **Compound 6** | 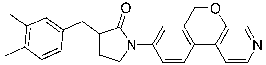 | LC_MS: (ES⁺): m/z 393.5 [M+H]⁺. |
| | | ¹H NMR (400 MHz, CDCl₃) δ 8.89 - 8.86 (m, 1H), 8.80 (dd, *J =* 4.6, 1.5 Hz, 2H), 8.01 - 7.95 (m, 3H), 7.11 (ddt, *J* = 9.8*,* 7.4, 5.2 Hz, 2H), 7.00 - 6.96 (m, 1H), 4.35 (ddd, *J =* 8.8, 7.7, 2.7 Hz, 1H), 4.10 (ddd, *J* = 11.4, 9.1, 7.4 Hz, 1H), 3.28 (dd, *J* = 14.0, 4.5 Hz, 1H), 3.10 - 3.00 (m, 1H), 2.85 (dd, *J =* 14.0, 8.9 Hz, 1H), 2.37 - 2.30 (m, 1H), 1.98 - 1.88 (m, 1H). |
| **Compound 7** | 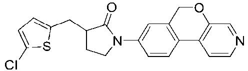 | LC_MS: (ES⁺): m/z 397.45 [M+H]⁺. |
| | | ¹H NMR (400 MHz, CDCl₃) δ 8.35 (s, 1H), 8.30 (d, *J* = 5.0 Hz, 1H), 7.76 (d, *J =* 8.5 Hz, 1H), 7.70 (d, *J =* 1.8 Hz, 1H), 7.58 (dd, *J =* 8.5, 2.1 Hz, 1H), 7.54 (d, *J* = 5.1 Hz, 1H), 6.77 (d, *J =* 3.7 Hz, 1H), 6.67 (d, *J =* 3.7 Hz, 1H), 5.23 (s, 2H), 3.82 (dd, *J =* 8.8, 7.5 Hz, 1H), 3.75 (td, *J =* 9.2, 2.6 Hz, 1H), 3.35 (dd, *J = 15.0,* 4.1 Hz, 1H), 3.07 (dd, *J =* 14.9, 8.2 Hz, 1H), 2.96 (dd, *J* = 9.4, 4.2 Hz, 1H), 2.42 - 2.31 (m, 1H), 1.96 (dd, *J =* 12.7, 9.5 Hz, 1H). |
| **Compound 8** | 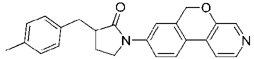 | LC_MS: (ES⁺): m/z 371.45 [M+H]⁺. |
| | | ¹H NMR (400 MHz, CDCl₃) δ 8.35 (s, 1H), 8.30 (d, *J* = 4.9 Hz, 1H), 7.75 (d, *J =* 8.6 Hz, 1H), 7.71 (d, *J =* 1.7 Hz, 1H), 7.59 - 7.53 (m, 2H), 7.18 - 7.12 (m, 4H), 5.22 (s, 2H), 3.76 (dd, *J =* 16.6, 8.5 Hz, 1H), 3.67 (dt, *J =* 9.3, 4.7 Hz, 1H), 3.28 (dd, *J =* 13.7, 4.2 Hz, 1H), 3.00 - 2.91 (m, 1H), 2.80 (dd, *J =* 13.7, 9.2 Hz, 1H), 2.35 (s, 3H), 2.23 (ddd, *J =* 12.0, 8.3, 4.2 Hz, 1H), 1.91 (ddd, *J =* 17.3, 12.7, 8.6 Hz, 1H). |
| **Compound 9** | 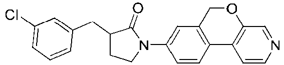 | LC_MS: (ES⁺): m/z 392.0 [M+H]⁺. |
| | | ¹H NMR (400 MHz, CDCl₃) δ 8.35 (s, 1H), 8.30 (d, *J* = 5.0 Hz, 1H), 7.75 (d, *J =* 8.5 Hz, 1H), 7.70 (d, *J =* 1.7 Hz, 1H), 7.60 - 7.53 (m, 2H), 7.26 (d, *J =* 6.7 Hz, 3H), 7.16 (d, *J =* 6.2 Hz, 1H), 5.23 (s, 2H), 3.79 (dd, *J =* 16.8, 8.6 Hz, 1H), 3.70 (td, *J =* 9.1, 3.0 Hz, 1H), 3.31 (dd, *J =* 13.8, 4.2 Hz, 1H), 2.97 (ddd, *J =* 18.0, 9.1, 4.2 Hz, 1H), 2.82 (dd, *J =* 13.8, 9.2 Hz, 1H), 2.32 - 2.21 (m, 1H), 1.90 (ddd, *J =* 17.9, 12.7, 8.8 Hz, 1H). |
| **Compound 10** | 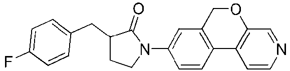 | LC_MS: (ES⁺): m/z 375.06 [M+H]⁺. |
| **Compound 11** | 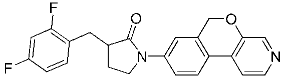 | LC_MS: (ES⁺): m/z 393.10 [M+H]⁺. |
| **Compound 12** | 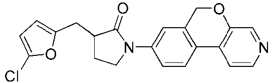 | LC_MS: (ES⁺): m/z 381.10 [M+H]⁺. |
| **Compound 13** | 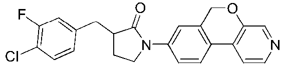 | LC_MS: (ES⁺): m/z 490.5 [M+H]⁺. |
| | | ¹H NMR (400 MHz, Chloroform-d) δ 8.40 - 8.25 (m, 2H), 7.81 - 7.46 (m, 4H), 7.34 (t, *J =* 7.9 Hz, 1H), 7.04 (dd, *J =* 30.1, 9.1 Hz, 2H), 5.21 (s, 2H), 3.87 - 3.63 (m, 2H), 3.27 (dd, *J =* 13.9, 4.3 Hz, 1H), 3.04 - 2.76 (m, 2H), 2.26 (dt, *J =* 14.7, 7.7 Hz, 1H), 1.91 - 1.83 (m, 1H). |
| **Compound 14** | 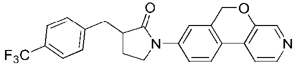 | LC_MS: (ES⁺): m/z 425.5 [M+H]⁺. |
| | | ¹H NMR (400 MHz, Chloroform-d) δ 8.34 (d, *J =* 20.7 Hz, 2H), 7.76 (d, *J =* 8.6 Hz, 1H), 7.70 *(d, J =* 2.2 Hz, 1H), 7.63 - 7.51 (m, 4H), 7.43 - 7.36 (m, 2H), 5.23 (s, 2H), 3.84 - 3.65 (m, 2H), 3.39 (dd, *J =* 13.6, 4.1 Hz, 1H), 3.06 - 2.86 (m, 2H), 2.26 (dtd, *J =* 15.7, 7.6, 2.9 Hz, 1H), 1.90 (dq, *J =* 12.8, 8.9 Hz, 1H). |
| **Compound 15** | 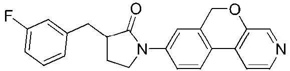 | LC_MS: (ES⁺): m/z 375.20 [M+H]⁺. |
| **Compound 16** | 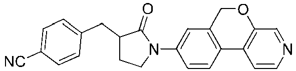 | LC_MS: (ES⁺): m/z 382.10 [M+H]⁺. |
| **Compound 17** | 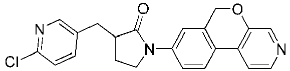 | LC_MS: (ES⁺): m/z 392.1 [M+H]⁺. |
| | | ¹H NMR (400 MHz, Chloroform-d) δ 9.31 (s, 1H), 9.05 (d, *J =* 10.6 Hz, 1H), 8.83 (s, 1H), 8.41 - 8.26 (m, 2H), 7.94 (d, *J =* 8.5 Hz, 1H), 7.77 (d, *J =* 8.7 Hz, 1H), 7.67 - 7.58 (m, 2H), 7.34 - 7.29 (m, 1H), 5.35 (s, 2H), 4.00 - 3.72 (m, 2H), 3.23 (ddd, *J =* 13.9, 12.0, 4.4 Hz, 1H), 3.07 - 2.84 (m, 2H), 2.25 (dp, *J =* 15.0, 8.8, 7.5 Hz, 1H), 1.97 - 1.80 (m, 1H). |
| **Compound 18** | 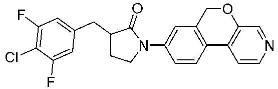 | LC_MS: (ES⁺): m/z 427.1 [M+H]⁺. |
| | | ¹H NMR (400 MHz, Chloroform-*d*) δ 8.34 (d, *J =* 21.2 Hz, 2H), 7.80 - 7.68 (m, 2H), 7.58 (dt, *J =* 9.5, 4.5 Hz, 2H), 6.93 (d, *J =* 7.8 Hz, 2H), 5.23 (s, 2H), 3.80 (dtd, *J* = 21.1, 9.4, 2.5 Hz, 2H), 3.28 (dd, *J =* 13.9, 4.4 Hz, 1H), 3.02 - 2.79 (m, 2H), 2.41 - 2.20 (m, 1H), 2.03 (d, *J* = 7.3 Hz, 1H). |
| **Compound 19** | 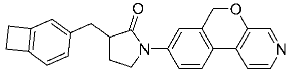 | LC_MS: (ES⁺): m/z 383.6 [M+H]⁺. |
| **Compound 54** | 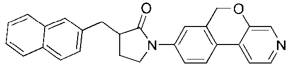 | LC_MS: (ES⁺): m/z 407.3 [M+H]⁺. |
| **Compound 55** | 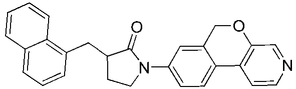 | LC_MS: (ES⁺): m/z 407.3 [M+H]⁺. |
| | | ¹H NMR (400 MHz, CDCl₃) δ 8.36 (s, 1H), 8.30 (d, *J* = 5.0 Hz, 1H), 8.18 (d, *J = 8.3* Hz, 1H), 7.93 - 7.89 (m, 1H), 7.80 (d, *J =* 7.9 Hz, 1H), 7.76 (t, *J =* 5.7 Hz, 2H), 7.64 - 7.61 (m, 1H), 7.60 - 7.51 (m, 3H), 7.47 - 7.39 (m, 2H), 5.24 (s, 2H), 4.06 (dd, *J =* 14.0, 3.6 Hz, 1H), 3.81 - 3.72 (m, 2H), 3.20 - 3.12 (m, 1H), 3.03 (dd, *J =* 14.0, 10.4 Hz, 1H), 2.21 - 2.11 (m, 1H), 2.01 - 1.90 (m, 1H). |
| **Compound 56** | 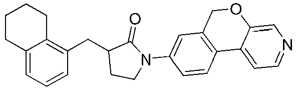 | LC_MS: (ES⁺): m/z 411.3 [M+H]⁺. |
| **Compound 57** | 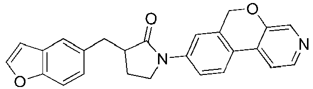 | LC_MS: (ES⁺): m/z 397.2 [M+H]⁺. |
| **Compound 58** | 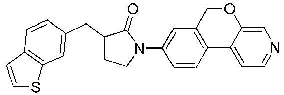 | LC_MS: (ES⁺): m/z 413.1 [M+H]⁺ |
| **Compound 59** | 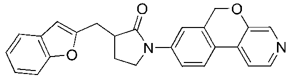 | LC_MS: (ES⁺): m/z 397.1 [M+H]⁺ |
| **Compound 60** | 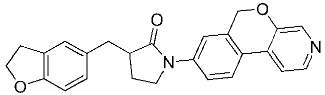 | LC_MS: (ES⁺): m/z 399.1 [M+H]⁺ |
| **Compound 61** | 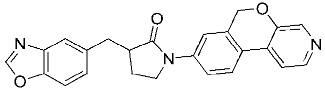 | LC_MS: (ES⁺): m/z 398.1 [M+H]⁺ |
| **Compound 62** | 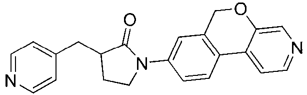 | LC_MS: (ES): m/z 358.1 [M+H]⁺ |
| | | ¹H NMR (400 MHz, DMSO-d6) δ 8.52 - 8.46 (m, 2H), 8.29 - 8.22 (m, 2H), 7.97 (d, *J =* 8.6 Hz, 1H), 7.85 - 7.78 (m, 2H), 7.68 (d, *J = 2.2* Hz, 1H), 7.36 - 7.29 (m, 2H), 5.25 (s, 2H), 3.78 (qd, *J =* 9.4, 6.5 Hz, 2H), 3.23 - 3.00 (m, 2H), 2.75 (dd, *J =* 13.7, 9.4 Hz, 1H), 2.12 (dddd, *J =* 12.1, 9.4, 6.5, 3.2 Hz, 1H), 1.78 (dq, *J =* 12.3, 9.0 Hz, 1H). |
| **Compound 149** | 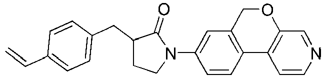 | LC_MS: (ES⁺): m/z 383.2 [M+H]⁺ |
| | | ¹H NMR (400 MHz, DMSO) δ 8.28 (s, 1H), 8.25 (d, *J* = 5.0 Hz, 1H), 7.97 (d, *J* = 8.6 Hz, 1H), 7.80 (dd, *J* = 11.8, 3.6 Hz, 2H), 7.68 (d, *J =* 1.9 Hz, 1H), 7.41 (d, *J =* 8.1 Hz, 2H), 7.26 (d, *J =* 8.1 Hz, 2H), 6.71 (dd, *J* = 17.7, 11.0 Hz, 1H), 5.80 (d, *J =* 17.7 Hz, 1H), 5.25 (s, 2H), 5.22 (d, *J =* 11.0 Hz, 1H), 3.82 - 3.68 (m, 2H), 3.12 (dd, *J =* 13.6, 4.3 Hz, 1H), 2.98 (qd, *J =* 9.2, 4.4 Hz, 1H), 2.74 (dd, *J =* 13.6, 9.3 Hz, 1H), 2.09 (ddd, *J =* 11.8, 10.8, 2.9 Hz, 1H), 1.78 (dq, *J =* 12.3, 8.8 Hz, 1H). |
| **Compound 150** | 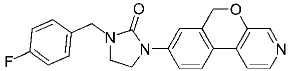 | LC_MS: (ES⁺): m/z 376.1 [M+H]⁺ |

The synthesis route of the following compounds is shown below, which were synthesized using corresponding reagents following Step 1 of the synthesis route of Example 3:

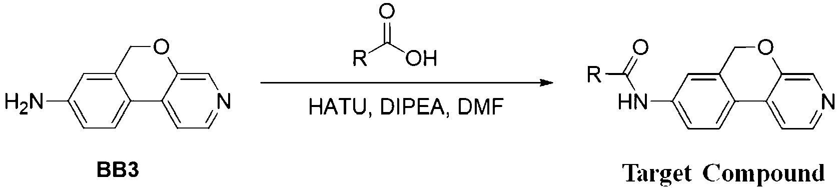

| Compound Number | Structural Formula | Mass Spectrum and NMR |
|---|---|---|
| **Compound 20** | 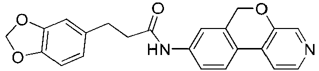 | LC_MS: (ES⁺): m/z 375.1 [M+H]⁺. |
| | | ¹H NMR (400 MHz, CDCl₃): *δ* 8.32 (s, 1H), 8.27 (s, 1H), 7.66 (d, *J =* 8.4 Hz, 1H), 7.58 (s, 1H), 7.49 (d, *J =* 5.0 Hz, 1H), 7.30 (dd, *J*₁ = 8.5 Hz, *J*₂ = 2.3 Hz, 1H), 7.18 (s, 1H), 6.84 - 6.62 (m, 3H), 5.93 (s, 2H), 5.16 (s, 2H), 2.99 (t, *J =* 7.4 Hz, 2H), 2.65 (t, *J =* 7.4 Hz, 2H). |
| **Compound 21** | 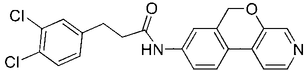 | LC_MS: (ES⁺): m/z 399.1, 401.0 [M+H]⁺. |
| | | ¹H NMR (400 MHz, DMSO-*d*₆): *δ* 10.18 (s, 1H), 8.25 (s, 1H), 8.22 (d, *J* = 5.0 Hz, 1H), 7.89 (d, *J =* 8.5 Hz, 1H), 7.75 (d, *J =* 5.0 Hz, 1H), 7.67 - 7.51 (m, 4H), 7.27 (dd, *J*₁ = 8.3 Hz, *J*₂ = 2.1 Hz, 1H), 5.20 (s, 2H), 2.92 (t, *J =* 7.5 Hz, 2H), 2.68 (t, *J =* 7.5 Hz, 2H). |
| **Compound 22** | 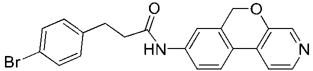 | LC_MS: (ES⁺): m/z 409.1, 411.0 [M+H]⁺. |
| | | ¹H NMR (400 MHz, DMSO-*d*₆): *δ* 10.17 (s, 1H), 8.37 - 8.18 (m, 2H), 7.88 (d, *J* = 8.5 Hz, 1H), 7.75 (d, *J* = 5.0 Hz, 1H), 7.65 - 7.54 (m, 2H), 7.47 (d, *J* = 8.4 Hz, 2H), 7.23 (d, *J =* 8.1 Hz, 2H), 5.20 (s, 2H), 2.89 (t, *J =* 7.5 Hz, 2H), 2.66 (t, *J =* 7.5 Hz, 2H). |
| **Compound 23** | 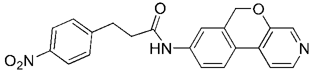 | LC_MS: (ES⁺): m/z 376.1 [M+H]⁺. |
| | | ¹H NMR (400 MHz, DMSO-*d*₆): *δ* 10.21 (s, 1H), 8.25 (s, 1H), 8.22 (d, *J =* 5.0 Hz, 1H), 8.20 - 8.14 (m, 2H), 7.88 (d, *J* = 8.5 Hz, 1H), 7.75 (d, *J =* 5.1 Hz, 1H), 7.64 - 7.51 (m, 4H), 5.20 (s, 2H), 3.06 (t, *J =* 7.5 Hz, 2H), 2.74 (t, *J =* 7.5 Hz, 2H). |
| **Compound 24** | 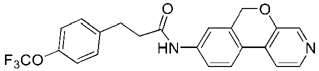 | LC_MS: (ES⁺): m/z 415.1 [M+H]⁺. |
| | | ¹H NMR (400 MHz, DMSO-*d*₆): *δ* 10.18 (s, 1H), 8.25 (s, 1H), 8.22 (d, *J =* 5.0 Hz, 1H), 7.88 (d, *J =* 8.5 Hz, 1H), 7.75 (d, *J =* 5.1 Hz, 1H), 7.65 - 7.51 (m, 2H), 7.45 - 7.21 (m, 4H), 5.20 (s, 2H), 2.95 (t, *J =* 7.6 Hz, 2H), 2.68 (t, *J =* 7.6 Hz, 2H). |
| **Compound 25** | 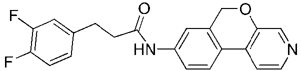 | LC_MS: (ES⁺): m/z 367.45 [M+H]⁺. |
| | | ¹H NMR (400 MHz, DMSO) δ 10.18 (s, 1H), 8.28 - 8.22 (m, 2H), 7.89 (d, *J* = 8.5 Hz, 1H), 7.76 (d, *J* = 5.1 Hz, 1H), 7.65 - 7.56 (m, 2H), 7.38 - 7.30 (m, 2H), 7.14 - 7.08 (m, 1H), 5.21 (s, 2H), 2.92 (t, *J =* 7.5 Hz, 2H), 2.68 (t, *J =* 7.6 Hz, 2H). |
| **Compound 26** | 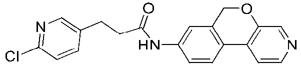 | LC_MS: (ES⁺): m/z 366.40 [M+H]⁺. |
| **Compound 27** | 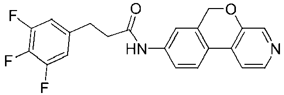 | LC_MS: (ES⁺): m/z 385.40 [M+H]⁺. |
| **Compound 28** | 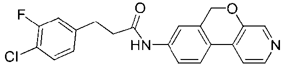 | LC_MS: (ES⁺): m/z 383.95 [M+H]⁺. |
| | | ¹H NMR (400 MHz, DMSO) δ 10.20 (s, 1H), 8.26 (s, 1H), 8.23 (d, *J =* 5.0 Hz, 1H), 7.89 (d, *J =* 8.5 Hz, 1H), 7.76 (d, *J =* 5.0 Hz, 1H), 7.62 (s, 1H), 7.58 (dd, *J =* 8.5, 2.0 Hz, 1H), 7.50 (t, *J =* 8.1 Hz, 1H), 7.34 (dd, *J =* 10.7, 1.9 Hz, 1H), 7.14 (dd, *J =* 8.2, 1.5 Hz, 1H), 5.21 (s, 2H), 2.94 (t, *J =* 7.5 Hz, 2H), 2.69 (t, *J =* 7.5 Hz, 2H). |
| **Compound 29** | 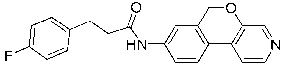 | LC_MS: (ES⁺): m/z 349.45 [M+H]⁺. |
| | | ¹H NMR (400 MHz, DMSO) δ 10.19 (s, 1H), 8.25 (s, 1H), 8.23 (d, *J =* 5.0 Hz, 1H), 7.89 (d, *J =* 8.5 Hz, 1H), 7.76 (d, *J =* 5.0 Hz, 1H), 7.63 (s, 1H), 7.59 (dd, *J =* 8.5, 2.0 Hz, 1H), 7.32 - 7.27 (m, 2H), 7.14 - 7.08 (m, 2H), 5.21 (s, 2H), 2.92 (t, *J =* 7.6 Hz, 2H), 2.66 (t, *J =* 7.6 Hz, 2H). |
| **Compound 30** | 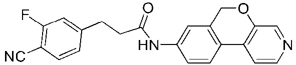 | LC_MS: (ES⁺): m/z 374.11 [M+H]⁺. |
| | | ¹H NMR (400 MHz, DMSO) δ 10.22 (s, 1H), 8.25 (s, 1H), 8.23 (d, *J =* 5.0 Hz, 1H), 7.89 (d, *J =* 8.5 Hz, 1H), 7.88 - 7.83 (m, 1H), 7.76 (d, *J* = 5.0 Hz, 1H), 7.61 (s, 1H), 7.60 - 7.55 (m, 1H), 7.48 (d, *J =* 10.8 Hz, 1H), 7.34 (dd, *J =* 8.0, 1.3 Hz, 1H), 5.21 (s, 2H), 3.03 (t, *J =* 7.4 Hz, 2H), 2.74 (t, *J =* 7.5 Hz, 2H). |
| **Compound 31** | 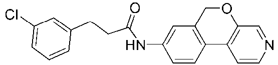 | LC_MS: (ES⁺): m/z 365.2 [M+H]⁺. |
| | | ¹H NMR (400 MHz, DMSO-*d*₆) *δ* 10.18 (*s*, 1H), 8.25 (*s,* 1H), 8.23 (*d*, *J =* 5.0 Hz, 1H), 7.89 (*d*, *J =* 8.5 Hz, 1H), 7.76 (*d*, *J =* 5.0 Hz, 1H), 7.62 (*d*, *J =* 2.1 Hz, 1H), 7.58 (*dd, J* = 8.5, 2.2 Hz, 1H), 7.35 (*t*, *J =* 2.2 Hz, 1H), 7.32 *(s,* 1H), 7.29-7.22 (*m,* 2H), 5.21 (*s,* 2H), 2.94 (*t*, *J =* 7.5 Hz, 2H), 2.69 (*t*, *J =* 7.5 Hz, 2H). |
| **Compound 32** | 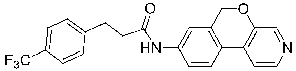 | LC_MS: (ES⁺): m/z 399.4 [M+H]⁺. |
| | | ¹H NMR (400 MHz, DMSO-*d*₆) *δ* 10.21 (*s,* 1H), 8.26 (*s,* 1H), 8.23 (*d, J =* 5.0 Hz, 1H), 7.89 (*d*, *J =* 8.5 Hz, 1H), 7.76 (*d, J =* 5.1 Hz, 1H), 7.69-7.62 (*m,* 3H), 7.58 (*dd, J* = 8.4, 2.2 Hz, 1H), 7.50 (*d*, *J =* 8.0 Hz, 2H), 5.21 *(s,* 2H), 3.02 (*t, J* = 7.5 Hz, 2H), 2.72 (*t*, *J =* 7.6 Hz, 2H). |
| **Compound 33** | 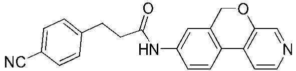 | LC_MS: (ES⁺): m/z 356.2 [M+H]⁺. |
| | | ¹H NMR (400 MHz, DMSO-*d*₆) *δ* 10.20 (*s,* 1H), 8.25 (*s,* 1H), 8.23 (*d, J =* 5.0 Hz, 1H), 7.89 (*d, J =* 8.5 Hz, 1H), 7.80-7.74 (*m,* 3H), 7.62 (*d, J =* 2.1 Hz, 1H), 7.58 (*dd, J =* 8.4, 2.2 Hz, 1H), 7.53-7.45 (*m,* 2H), 5.21 (*s,* 2H), 3.02 *(t, J* = 7.5 Hz, 2H), 2.72 (*t, J =* 7.5 Hz, 2H). |
| **Compound 34** | 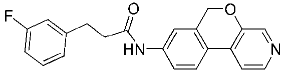 | LC_MS: (ES⁺): m/z 349.1 [M+H]⁺. |
| | | ¹H NMR (400 MHz, DMSO-*d*₆) *δ* 10.18 (*s,* 1H), 8.26 (*s,* 1H), 8.23 (*d, J =* 5.0 Hz, 1H), 7.89 (*d, J =* 8.5 Hz, 1H), *7.76* (*d, J* = 5.0 Hz, 1H), 7.63 (*d, J* = 2.1 Hz, 1H), 7.59 (*dd, J* = 8.5, 2.2 Hz, 1H), 7.33 (*dt, J =* 8.8, 6.7 Hz, 1H), 7.14-7.09 (*m,* 2H), 7.05-6.98 (*m,* 1H), 5.21 *(s,* 2H), 2.95 (*t, J* = 7.6 Hz, 2H), 2.69 (*t, J =* 7.6 Hz, 2H). |
| **Compound 35** | 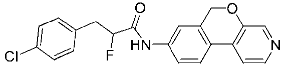 | LC_MS: (ES⁺): m/z 383.1 [M+H]⁺. |
| | | ¹H NMR (400 MHz, DMSO-*d*₆) *δ* 10.35 (*s,* 1H), 8.28 (*s,* 1H), 8.25 (*d, J* = 5.0 Hz, 1H), 7.97-7.90 (*m,* 1H), 7.79 (*d, J =* 5.1 Hz, 1H), 7.68 (*d, J* = 7.3 Hz, 2H), 7.42-7.36 (*m,* 2H), 7.34 (*d, J =* 8.5 Hz, 2H), 5.40 (*dd, J =* 7.9, 4.1 Hz, 0.5H), 5.28 *(dd, J* = 7.9*,* 4.1 Hz, 0.5H), 5.23 (*s,* 2H), 3.31-3.12 (*m,* 2H). |
| **Compound 36** | 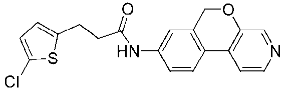 | LC_MS: (ES⁺): m/z 371.02 [M+H]⁺. |
| | | ¹H NMR (400 MHz, DMSO) δ 10.25 (s, 1H), 8.26 (s, 1H), 8.23 (d, *J =* 5.0 Hz, 1H), 7.90 (d, *J =* 8.5 Hz, 1H), 7.76 (d, *J =* 5.0 Hz, 1H), 7.64 (s, 1H), 7.60 (d, *J =* 8.5 Hz, 1H), 6.93 (d, *J =* 3.7 Hz, 1H), 6.78 (d, *J =* 3.6 Hz, 1H), 5.22 (s, 2H), 3.08 (t, *J=* 7.1 Hz, 2H), 2.70 (t, *J=* 7.1 Hz, 2H). |
| **Compound 37** | 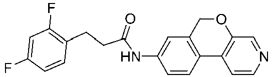 | LC_MS: (ES⁺): m/z 367.5 [M+H]⁺. |
| | | ¹H NMR (400 MHz, Chloroform-d) δ 8.34 (s, 1H), 8.28 (d, *J* = 5.1 Hz, 1H), 7.69 (d, *J =* 8.4 Hz, 1H), 7.64 - 7.58 (m, 1H), 7.51 (d, *J* = 5.1 Hz, 1H), 7.41 - 7.34 (m, 2H), 7.28 - 7.21 (m, 1H), 6.88 - 6.75 (m, 2H), 5.18 (s, 2H), 3.08 (t, *J =* 7.5 Hz, 2H), 2.70 (t, *J =* 7.5 Hz, 2H). |
| **Compound 38** | 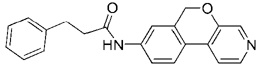 | LC_MS: (ES⁺): m/z 331.5 [M+H]⁺. |
| | | ¹H NMR (400 MHz, Chloroform-d) δ 8.34 (s, 1H), 8.28 (d, *J =* 5.1 Hz, 1H), 7.67 (d, *J =* 8.4 Hz, 1H), 7.58 (d, *J =* 2.2 Hz, 1H), 7.51 (d, *J* = 5.1 Hz, 1H), 7.37 - 7.23 (m, 7H), 5.18 (s, 2H), 3.09 (t, *J =* 7.5 Hz, 2H), 2.72 (t, *J =* 7.5 Hz, 2H) |
| **Compound 39** | 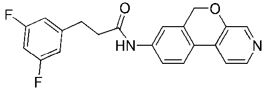 | LC_MS: (ES⁺): m/z 367.6 [M+H]⁺. |
| | | ¹H NMR (400 MHz, Chloroform-*d*) δ 8.34 (s, 1H), 8.29 (d, *J =* 5.0 Hz, 1H), 7.70 (d, *J =* 8.4 Hz, 1H), 7.63 - 7.59 (m, 1H), 7.39 - 7.30 (m, 2H), 6.84 - 6.76 (m, 2H), 6.69 (tt, *J =* 9.0, 2.3 Hz, 1H), 5.19 (s, 2H), 3.08 (t, *J =* 7.5 Hz, 2H), 2.71 (t, *J =* 7.5 Hz, 2H). |
| **Compound 40** | 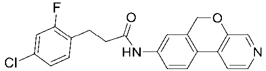 | LC_MS: (ES⁺): m/z 383.4 [M+H]⁺. |
| | | ¹H NMR (400 MHz, DMSO-*d*₆) δ 10.21 (s, 1H), 8.25 (s, 1H), 8.23 (d, *J =* 5.0 Hz, 1H), 7.89 (d, *J =* 8.5 Hz, 1H), 7.76 (d, *J =* 5.1 Hz, 1H), 7.61 (d, *J =* 2.1 Hz, 1H), 7.58 (d, *J* = 2.2 Hz, 1H), 7.41 - 7.34 (m, 2H), 7.23 (dd, *J =* 8.2, 2.1 Hz, 1H), 5.21 (s, 2H), 2.93 (t, *J =* 7.5 Hz, 2H), 2.70 - 2.66 (m, 2H). |
| **Compound 41** | 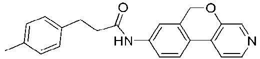 | LC_MS: (ES⁺): m/z 345.6 [M+H]⁺. |
| | | ¹H NMR (400 MHz, DMSO-*d*₆) δ 10.16 (s, 1H), 8.27 - 8.21 (m, 2H), 7.89 (d, *J* = 8.4 Hz, 1H), 7.76 (d, *J* = 5.0 Hz, 1H), 7.65 - 7.56 (m, 2H), 7.17 - 7.07 (m, 4H), 5.21 (s, 2H), 2.87 (d, *J =* 7.8 Hz, 2H), 2.64 (dd, *J* = 8.3, 7.0 Hz, 2H), 2.26 (s, 3H). |
| **Compound 43** | 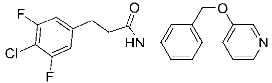 | LC_MS: (ES⁺): m/z 401.4 [M+H]⁺. |
| | | ¹H NMR (400 MHz, DMSO) δ 10.25 (s, 1H), 8.25 (s, 1H), 8.22 (d, *J =* 5.0 Hz, 1H), 7.89 (d, *J =* 8.5 Hz, 1H), 7.75 (d, *J =* 5.1 Hz, 1H), 7.62 (d, *J =* 1.5 Hz, 1H), 7.59 (dd, *J =* 8.5, 1.9 Hz, 1H), 7.27 (d, *J =* 8.4 Hz, 2H), 5.21 (s, 2H), 2.95 (t, *J =* 7.5 Hz, 2H), 2.74 - 2.68 (m, 2H). |
| **Compound 44** | 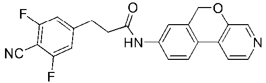 | LC_MS: (ES⁺): m/z 392.5 [M+H]⁺. |
| | | ¹H NMR (400 MHz, DMSO) δ 10.26 (s, 1H), 8.25 (s, 1H), 8.23 (d, *J =* 4.9 Hz, 1H), 7.89 (d, *J =* 8.4 Hz, 1H), 7.76 (d, *J* = 5.0 Hz, 1H), 7.62 (s, 1H), 7.58 (d, *J* = 8.5 Hz, 1H), 7.42 (s, 1H), 7.39 (s, 1H), 5.21 (s, 2H), 3.04 (t, *J =* 7.4 Hz, 2H), 2.76 (t, *J =* 7.5 Hz, 2H). |
| **Compound 50** | 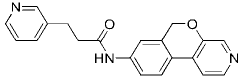 | LC_MS: (ES⁺): m/z 332.1 [M+H]⁺. |
| | | ¹H NMR (400 MHz, DMSO-*d*₆): *δ* 10.19 (s, 1H), 8.49 (d, *J* = 2.3 Hz, 1H), 8.40 (dd, *J* = 4.8 Hz, *J*₂ = 1.7 Hz, 1H), 8.25 (s, 1H), 8.22 (d, *J =* 5.0 Hz, 1H), 7.88 (d, *J =* 8.5 Hz, 1H), 7.75 (d, *J =* 5.0 Hz, 1H), 7.70 - 7.50 (m, 3H), 7.31 (dd, *J*₁ *=* 7.8 Hz, *J*₂ = 4.8 Hz, 1H), 5.20 (s, 2H), 2.94 (t, *J* = 7.5 Hz, 2H), 2.70 (t, *J =* 7.5 Hz, 2H). |
| **Compound 51** | 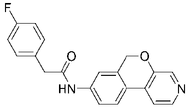 | LC_MS: (ES⁺): m/z 335.1 [M+H]⁺. |
| | | ¹H NMR (400 MHz, MeOH-*d*₄): *δ* 8.26 - 8.09 (m, 2H), 7.84 (d, *J* = 7.7 Hz, 1H), 7.74 (d, *J* = 5.2 Hz, 1H), 7.62-7.60 (m, 2H), 7.37 (dd, *J*₁ *=* 8.6 Hz, *J*₂ = 5.5 Hz, 2H), 7.06 (t, *J =* 8.8 Hz, 2H), 5.19 (s, 2H), 3.70 (s, 2H). |
| **Compound 73** | 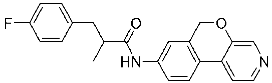 | LC_MS: (ES⁺): m/z 363.2 [M+H]⁺. |
| | | ¹H NMR (400 MHz, DMSO-*d*₆) *δ* 10.09 (s, 1H), 8.25 (s, 1H), 8.22 (d, *J =* 5.0 Hz, 1H), 7.87 (d, *J =* 8.5 Hz, 1H), 7.75 (d, *J =* 5.0 Hz, 1H), 7.63 - 7.52 (m, 2H), 7.31 - 7.20 (m, 2H), 7.15 - 7.03 (m, 2H), 5.20 (s, 2H), 2.94 (dd, *J*₁ *=* 13.3 Hz, *J*₂ = 7.8 Hz, 1H), 2.85 - 2.72 (m, 1H), 2.63 (dd, *J*₁ = 13.3 Hz, *J*₂ = 6.6 Hz, 1H), 1.11 (d, *J =* 6.7 Hz, 3H). |
| **Compound 74** | 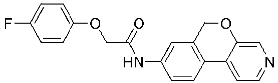 | LC_MS: (ES⁺): m/z 351.1 [M+H]⁺. |
| **Compound 78** | 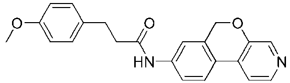 | LC_MS: (ES⁺): m/z 361.9 [M+H]⁺. |
| | | ¹H NMR (400 MHz, DMSO) δ 10.16 (s, 1H), 8.25 (s, 1H), 8.23 (d, *J =* 5.0 Hz, 1H), 7.89 (d, *J =* 8.5 Hz, 1H), 7.75 (d, *J =* 5.1 Hz, 1H), 7.63 (s, 1H), 7.59 (d, *J =* 8.4 Hz, 1H), 7.17 (d, *J =* 8.6 Hz, 2H), 6.87 - 6.83 (m, 2H), 5.21 (s, 2H), 3.71 (s, 3H), 2.86 (t, *J =* 7.6 Hz, 2H), 2.62 (t, *J =* 7.6 Hz, 2H). |
| **Compound 79** | 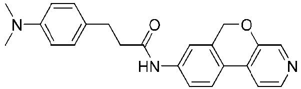 | LC_MS: (ES⁺): m/z 374.2 [M+H]⁺. |
| | | ¹H NMR (400 MHz, DMSO) δ 10.17 (s, 1H), 8.26 (s, 1H), 8.23 (d, *J =* 5.0 Hz, 1H), 7.90 (s, 1H), 7.76 (d, *J =* 5.0 Hz, 1H), 7.65 (d, *J =* 1.6 Hz, 1H), 7.60 (dd, *J =* 8.5, 2.0 Hz, 1H), 7.07 (d, *J =* 8.6 Hz, 2H), 6.66 (d, *J =* 8.7 Hz, 2H), 5.21 (s, 2H), 2.84 (s, 6H), 2.80 (d, *J =* 7.9 Hz, 2H), 2.60 (t, *J =* 7.6 Hz, 2H). |
| **Compound 80** | 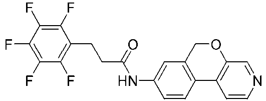 | LC_MS: (ES⁺): m/z 421.1 [M+H]⁺. |
| | | ¹H NMR (400 MHz, CDCl₃) δ 8.34 (s, 1H), 8.28 (d, *J* = 5.0 Hz, 1H), 7.70 (d, *J =* 8.4 Hz, 1H), 7.62 (s, 1H), 7.56 (s, 1H), 7.52 (d, *J =* 5.1 Hz, 1H), 7.39 (dd, *J =* 8.4, 1.6 Hz, 1H), 5.19 (s, 2H), 3.17 (t, *J* = 7.6 Hz, 2H), 2.74 (t, *J =* 7.7 Hz, 2H). |
| **Compound 81** | 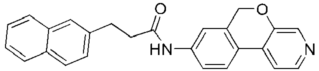 | LC_MS: (ES⁺): m/z 382.0 [M+H]⁺. |
| **Compound 82** | 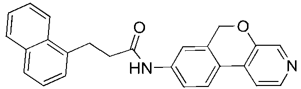 | LC_MS: (ES⁺): m/z 381.1 [M+H]⁺. |
| | | ¹H NMR (400 MHz, CDCl₃) δ 8.31 (s, 1H), 8.25 (d, *J =* 5.0 Hz, 1H), 8.08 (d, *J* = 8.1 Hz, 1H), 7.93 - 7.88 (m, 1H), 7.77 (dd, *J* = 6.6, 2.6 Hz, 1H), 7.63 (d, *J =* 8.4 Hz, 1H), 7.58 - 7.50 (m, 3H), 7.48 (d, *J =* 5.1 Hz, 1H), 7.44 (d, *J* = 4.8 Hz, 1H), 7.41 (dd, *J =* 6.7, 4.1 Hz, 2H), 7.26 (s, 1H), 5.15 (s, 2H), 3.55 (t, *J =* 7.6 Hz, 2H), 2.84 (t, *J* = *7.6* Hz, 2H). |
| **Compound 83** | 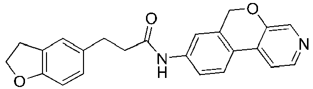 | LC_MS: (ES⁺): m/z 373.2 [M+H]⁺. |
| | | ¹H NMR (400 MHz, CDCl₃) δ 8.32 (s, 1H), 8.26 (d, *J =* 4.9 Hz, 1H), 7.66 (d, *J =* 8.4 Hz, 1H), 7.58 (s, 1H), 7.50 (d, *J* = 5.1 Hz, 2H), 7.31 (d, *J =* 8.3 Hz, 1H), 7.08 (s, 1H), 6.98 (d, *J =* 8.0 Hz, 1H), 6.74 (d, *J =* 8.1 Hz, 1H), 5.17 (s, 2H), 4.56 (t, *J* = 8.7 Hz, 2H), 3.17 (t, *J* = 8.6 Hz, 2H), 3.00 (t, *J =* 7.4 Hz, 2H), 2.67 (t, *J =* 7.4 Hz, 2H). |
| **Compound 75** | 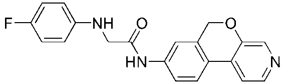 | LC_MS: (ES⁺): m/z 350.1 [M+H]⁺ |
| | | ¹H NMR (400 MHz, Chloroform-d) *δ* 8.76 (*s,* 1H), 8.34 (s, 1H), 8.29 (*d*, *J =* 5.1 Hz, 1H), 7.70 (*d, J =* 8.4 Hz, 1H), *7.64* (*d, J* = 2.2 Hz, 1H), 7.52 (*d, J =* 5.0 Hz, 1H), 7.46 (*dd, J* = 8.4, 2.2 Hz, 1H), 6.99 (*t, J* = 8.6 Hz, 2H), 6.71-6.64 (m, 2H), 5.19 *(s,* 2H), 3.93 (*s*, 2H). |
| **Compound 76** | 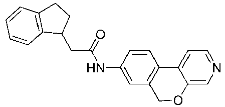 | LC_MS: (ES⁺): m/z 357.1 [M+H]⁺ |
| **Compound 132** | 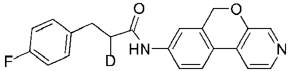 | LC_MS: (ES⁺): m/z 350.1 [M+H]⁺ |
| **Compound 136** | 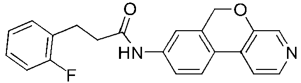 | LC_MS: (ES⁺): m/z 349.1 [M+H]⁺ |
| | | ¹H NMR (400 MHz, CDCl₃) δ 8.32 (s, 1H), 8.27 (d, *J* = 5.1 Hz, 1H), 7.66 (d, *J =* 8.4 Hz, 1H), 7.60 (d, *J =* 6.3 Hz, 2H), 7.50 (d, *J =* 5.1 Hz, 1H), 7.40 - 7.32 (m, 1H), 7.24 (ddd, *J =* 9.3, 8.3, 1.6 Hz, 2H), 7.13 - 7.01 (m, 2H), 5.17 (s, 2H), 3.11 (t, *J =* 7.5 Hz, 2H), 2.73 (t, *J =* 7.6 Hz, 2H). |
| **Compound 138** | 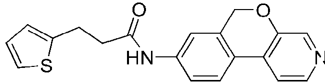 | LC_MS: (ES⁺): m/z 337.1 [M+H]⁺ |
| | | ¹H NMR (400 MHz, CDCl₃) δ 8.33 (s, 1H), 8.28 (d, *J*= 5.0 Hz, 1H), 7.68 (d, *J =* 8.4 Hz, 1H), 7.60 (s, 1H), 7.53 - 7.47 (m, 2H), 7.34 (d, *J* = 8.5 Hz, 1H), 7.18 (d, *J =* 5.1 Hz, 1H), 6.98 - 6.94 (m, 1H), 6.89 (d, *J =* 3.2 Hz, 1H), 5.18 (s, 2H), 3.31 (t, *J =* 7.3 Hz, 2H), 2.77 (t, *J =* 7.3 Hz, 2H). |
| **Compound 148** | 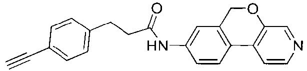 | LC_MS: (ES⁺): m/z 355.1 [M+H]⁺ |
| | | ¹H NMR (400MHz, DMSO) δ 10.19 (s, 1H), 8.26 (s, 1H), 8.23 (d, *J =* 5.0 Hz, 1H), 7.89 (d, *J =* 8.5 Hz, 1H), 7.76 (d, *J =* 5.1 Hz, 1H), 7.62 (s, 1H), 7.60 - 7.56 (m, 1H), 7.41 (d, *J =* 8.1 Hz, 2H), 7.28 (d, *J =* 8.1 Hz, 2H), 5.21 (s, 2H), 4.12 (s, 1H), 2.94 (t, *J =* 7.5 Hz, 2H), 2.67 (t, *J* = 7.6 Hz, 2H). |

The synthesis route of the following compounds is shown below, which were synthesized using corresponding reagents following Step 1 of the synthesis route of Example 5:

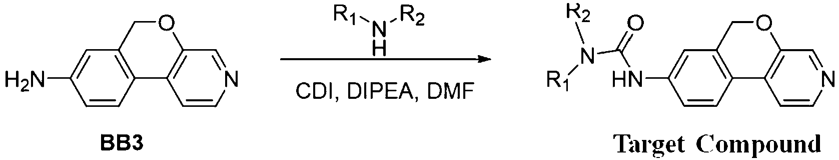

| **Compound Number** | **Structural Formula** | **Mass Spectrum and NMR** |
|---|---|---|
| **Compound 46** | 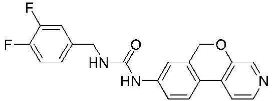 | LC_MS: (ES⁺): m/z 368.3 [M+H]⁺. |
| | | ¹H NMR (400 MHz, Chloroform-d) *δ* 8.32 (*s,* 1H), 8.27 (*d, J* = 5.0 Hz, 1H), 7.66 (*d, J =* 8.4 Hz, 1H), 7.50 (*d, J* = 5.1 Hz, 1H), 7.43 (*d, J =* 2.2 Hz, 1H), 7.25 (*dd, J =* 8.4, 2.4 Hz, 1H), 7.21-7.05 (*m,* 3H), 6.72 (*s,* 1H), 5.23 (*s*, 1H), 5.17 (*s,* 2H), 4.44 (*d, J =* 5.8 Hz, 2H). |
| **Compound 47** | 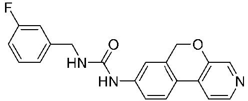 | LC_MS: (ES⁺): m/z 350.1 [M+H]⁺. |
| | | ¹H NMR (400 MHz, Chloroform*-d*) *δ* 8.32 *(s,* 1H), 8.27 (*d, J* = 5.0 Hz, 1H), 7.66 (*d, J =* 8.4 Hz, 1H), 7.50 (*d, J* = 5.1 Hz, 1H), 7.43 (*d, J =* 2.2 Hz, 1H), *7.25* (*dd, J =* 8.4, 2.4 Hz, 1H), 7.21-7.05 (*m,* 3H), 6.72 (*s,* 1H), 5.23 (*s*, 1H), 5.17 (*s,* 2H), 4.44 (*d, J =* 5.8 Hz, 2H). |
| **Compound 48** | 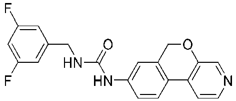 | LC_MS: (ES⁺): m/z 368.3 [M+H]⁺. |
| **Compound 63** | 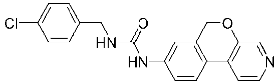 | LC_MS: (ES⁺): m/z 366.1 [M+H]⁺. |
| **Compound 64** | 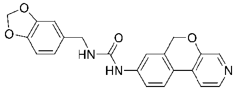 | LC_MS: (ES⁺): m/z 376.1 [M+H]⁺. |
| **Compound 65** | 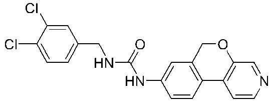 | LC_MS: (ES⁺): m/z 400.0 [M+H]⁺. |
| **Compound 67** | 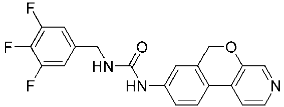 | LC_MS: (ES⁺): m/z 386.2 [M+H]⁺. |
| **Compound 68** | 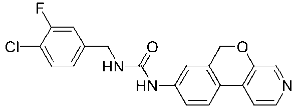 | LC_MS: (ES⁺): m/z 384.2 [M+H]⁺. |
| | | ¹H NMR (400 MHz, Chloroform-d) δ 8.29 (s, 1H), 8.24 (d, *J* = 5.1 Hz, 1H), 7.63 (d, *J =* 8.4 Hz, 1H), 7.49 (d, *J* = 5.1 Hz, 1H), 7.42 (d, *J* = 2.2 Hz, 1H), 7.36 (t, *J* = 7.8 Hz, 1H), 7.25 (dd, *J* = 8.5, 2.3 Hz, 1H), 7.15 (dd, *J =* 9.7, 2.0 Hz, 1H), 7.07 (dd, *J =* 8.0, 2.0 Hz, 1H), 6.95 (s, 1H), 5.15 (s, 2H), 4.45 (d, *J =* 5.9 Hz, 2H). |
| **Compound 69** | 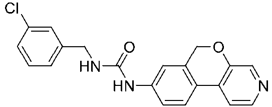 | LC_MS: (ES⁺): m/z 366.6 [M+H]⁺. |
| | | ¹H NMR (400 MHz, DMSO-d6) δ 9.01 (s, 1H), 8.25 - 8.18 (m, 2H), 7.82 (d, *J =* 9.1 Hz, 1H), 7.73 (d, *J* = 5.1 Hz, 1H), 7.45 (dd, *J =* 4.5, 2.4 Hz, 2H), 7.39 - 7.34 (m, 2H), 7.34 - 7.26 (m, 2H), 6.90 (t, *J =* 6.0 Hz, 1H), 5.18 (s, 2H), 4.32 (d, *J =* 6.0 Hz, 2H). |
| **Compound 70** | 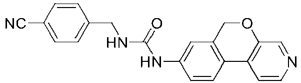 | LC_MS: (ES⁺): m/z 357.0 [M+H]⁺. |
| | | ¹H NMR (400 MHz, DMSO-d6) δ 9.08 (s, 1H), 8.25 - 8.18 (m, 2H), 7.82 (dd, *J =* 8.8, 2.8 Hz, 3H), 7.73 (d, *J =* 5.1 Hz, 1H), 7.53 - 7.48 (m, 2H), 7.44 (dq, *J =* 4.5, 2.3 Hz, 2H), 6.96 (t, *J =* 6.0 Hz, 1H), 5.18 (s, 2H), 4.40 (d, *J* = 6.0 Hz, 2H). |
| **Compound 71** | 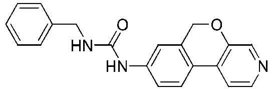 | LC_MS: (ES⁺): m/z 332.2 [M+H]⁺. |
| | | ¹H NMR (400 MHz, DMSO-d6) δ 8.91 (s, 1H), 8.25 - 8.18 (m, 2H), 7.85 - 7.79 (m, 1H), 7.75 - 7.71 (m, 1H), 7.47 - 7.42 (m, 2H), 7.38 - 7.30 (m, 4H), 7.28 - 7.22 (m, 1H), 6.78 (t, *J =* 5.9 Hz, 1H), 5.18 (s, 2H), 4.32 (d, *J* = 5.9 Hz, 2H). |
| **Compound 72** | 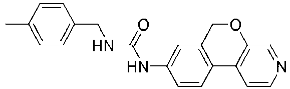 | LC_MS: (ES⁺): m/z 346.3 [M+H]⁺. |
| | | ¹H NMR (400 MHz, DMSO-d6) δ 8.87 (s, 1H), 8.26 - 8.17 (m, 2H), 7.85 - 7.78 (m, 1H), 7.72 (d, *J =* 5.1 Hz, 1H), 7.48 - 7.39 (m, 2H), 7.24 - 7.10 (m, 4H), 6.71 (t, *J* = 5.9 Hz, 1H), 5.18 (s, 2H), 4.26 (d, *J* = 5.8 Hz, 2H), 2.28 (s, 3H). |
| **Compound 66** | 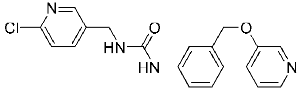 | LC_MS: (ES⁺): m/z 367.1 [M+H]⁺ |
| | | ¹H NMR (400 MHz, DMSO-d6) *δ* 9.00 *(s,* 1H), 8.36 *(d, J* = 2.4 Hz, 1H), 8.22 *(s,* 1H), 8.20 (d, *J =* 5.0 Hz, 1H), 7.84-7.77 *(m,* 2H), 7.72 (*d*, *J =* 5.1 Hz, 1H), 7.49 (d,*J =* 8.3 Hz, 1H), 7.42 (d, *J =* 6.5 Hz, 2H), 6.87 (t, *J =* 6.0 Hz, 1H), 5.17 *(s,* 2H), 4.32 (d, *J =* 5.9 Hz, 2H). |
| **Compound 135** | 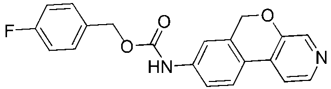 | LC_MS: (ES⁺): m/z 351.1 [M+H]⁺ |
| | | ¹H NMR (400 MHz, DMSO) δ 10.09 (s, 1H), 8.34 - 8.15 (m, 2H), 7.89 (d, *J =* 8.4 Hz, 1H), 7.75 (d, *J=* 4.9 Hz, 1H), 7.65 - 7.40 (m, 4H), 7.24 (t, *J =* 8.7 Hz, 2H), 5.20 (s, 2H), 5.16 (s, 2H). |

The synthesis route of the following compounds is shown below, which were synthesized using corresponding reagents following Step 1 of the synthesis route of Example 9:

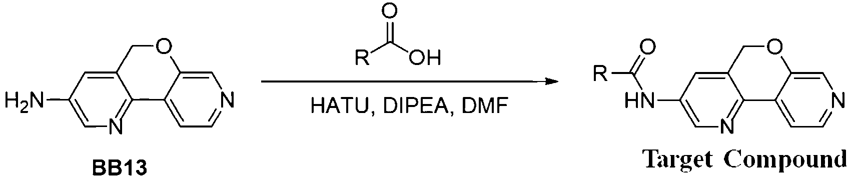

| **Compound Number** | **Structural Formula** | **Mass Spectrum and NMR** |
|---|---|---|
| | | LC_MS: (ES⁺): m/z 365.1 [M+H]⁺. |
| **Compound 84** | 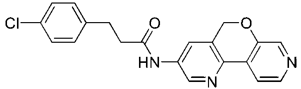 | ¹H NMR (400 MHz, CDCl₃) δ 8.35 (t, *J* = 5.7 Hz, 3H), 8.21 (s, 1H), 7.95 (d, *J =* 4.9 Hz, 1H), 7.62 (s, 1H), 7.31 (s, 2H), 7.19 (d, *J =* 8.2 Hz, 2H), 5.32 (s, 2H), 3.07 (t, *J =* 7.4 Hz, 2H), 2.74 (t, *J =* 7.4 Hz, 2H). |
| **Compound 85** | 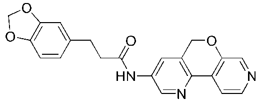 | LC_MS: (ES⁺): m/z 376.1 [M+H]⁺. |
| **Compound 86** | 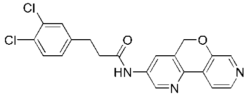 | LC_MS: (ES⁺): m/z 400.0 [M+H]⁺. |
| **Compound 87** | 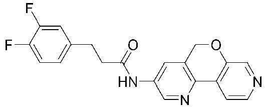 | LC_MS: (ES⁺): m/z 368.1 [M+H]⁺. |
| | | ¹H NMR (400 MHz, CDCl₃) δ 8.38 (d, *J =* 2.3 Hz, 1H), 8.33 (d, *J =* 5.5 Hz, 2H), 8.22 (s, 1H), 7.95 (d, *J =* 4.9 Hz, 1H), 7.76 (s, 1H), 7.14 - 7.03 (m, 2H), 7.00 - 6.93 (m, 1H), 5.32 (s, 2H), 3.05 (t, *J* = 7.4 Hz, 2H), 2.73 (t, *J =* 7.4 Hz, 2H). |
| **Compound 88** | 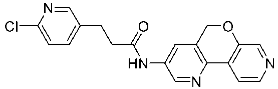 | LC_MS: (ES⁺): m/z 367.1 [M+H]⁺. |
| **Compound 89** | 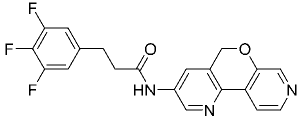 | LC_MS: (ES⁺): m/z 386.1 [M+H]⁺. |
| **Compound 90** | 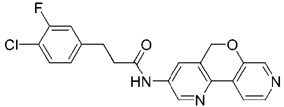 | LC_MS: (ES⁺): m/z 384.1 [M+H]⁺. |
| **Compound 92** | 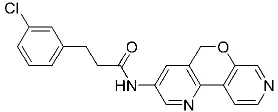 | LC_MS: (ES⁺): m/z 366.1 [M+H]⁺. |
| **Compound 93** | 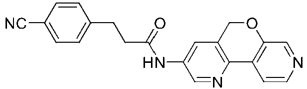 | LC_MS: (ES⁺): m/z 357.1 [M+H]⁺. |
| **Compound 94** | 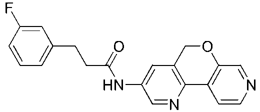 | LC_MS: (ES⁺): m/z 350.1 [M+H]⁺. |
| | | ¹H NMR (400 MHz, CDCl₃) δ 8.38 - 8.31 (m, 3H), 8.22 (s, 1H), 7.96 (d, *J =* 5.0 Hz, 1H), 7.54 (s, 1H), 7.31 (d, *J =* 7.9 Hz, 1H), 7.04 (d, *J =* 7.6 Hz, 1H), 7.00 - 6.92 (m, 2H), 5.32 (s, 2H), 3.10 (t, *J =* 7.4 Hz, 2H), 2.76 (t, *J =* 7.4 Hz, 2H). |
| **Compound 95** | 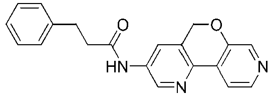 | LC_MS: (ES⁺): m/z 332.1 [M+H]⁺. |
| | | ¹H NMR (400 MHz, CDCl₃) δ 8.34 (s, 1H), 8.32 (d, *J* = 5.0 Hz, 1H), 8.29 (d, *J* = 2.3 Hz, 1H), 8.21 (s, 1H), 7.93 (d, *J =* 5.0 Hz, 1H), 7.78 (s, 1H), 7.37 - 7.30 (m, 2H), 7.26 (t, *J =* 6.0 Hz, 3H), 5.30 (s, 2H), 3.09 (t, *J =* 7.4 Hz, 2H), 2.76 (t, *J=* 7.4 Hz, 2H). |
| **Compound 96** | 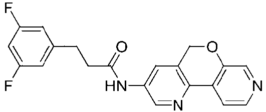 | LC_MS: (ES⁺): m/z 368.1 [M+H]⁺. |
| | | ¹H NMR (400 MHz, CDCl₃) δ 8.37 (d, *J =* 16.0 Hz, 3H), 8.23 (s, 1H), 7.96 (s, 1H), 7.65 (s, 1H), 6.74 (d, *J =* 36.9 Hz, 3H), 5.32 (s, 2H), 3.08 (s, 2H), 2.75 (s, 2H). |
| **Compound 97** | 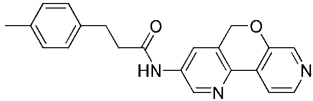 | LC_MS: (ES⁺): m/z 346.1 [M+H]⁺. |
| **Compound 98** | 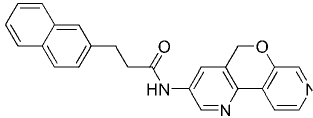 | LC_MS: (ES⁺): m/z 382.1 [M+H]⁺. |
| **Compound 99** | 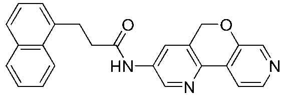 | LC_MS: (ES⁺): m/z 382.1 [M+H]⁺. |
| **Compound 100** | 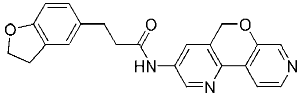 | LC_MS: (ES⁺): m/z 374.1 [M+H]⁺. |

The synthesis route of the following compounds is shown below, which were synthesized using corresponding reagents following Step 1 of the synthesis route of Example 12:

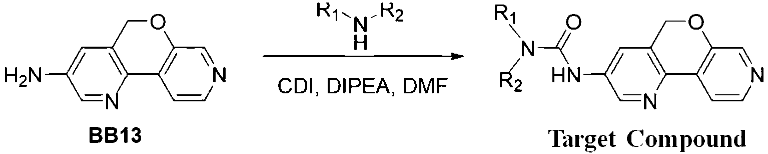

| **Compound Number** | **Structural Formula** | **Mass Spectrum and NMR** |
|---|---|---|
| **Compound 102** | 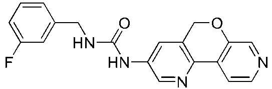 | LC_MS: (ES⁺): m/z 351.1 [M+H]⁺. |
| **Compound 103** | 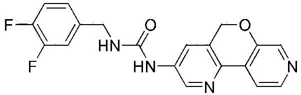 | LC_MS: (ES⁺): m/z 369.1 [M+H]⁺. |
| **Compound 104** | 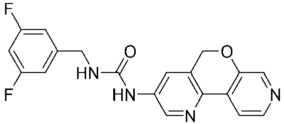 | LC_MS: (ES⁺): m/z 369.1 [M+H]⁺. |
| **Compound 153** | 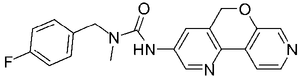 | LC_MS: (ES⁺): m/z 365.1 [M+H]⁺. |

The synthesis route of the following compounds is shown below, which were synthesized using corresponding reagents following Step 1 of the synthesis route of Example 10:

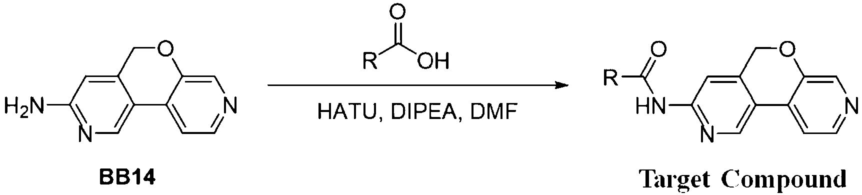

| **Compound Number** | **Structural Formula** | **Mass Spectrum and NMR** |
|---|---|---|
| **Compound 105** | 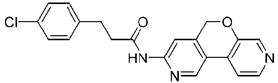 | LC_MS: (ES⁺): m/z 366.1 [M+H]⁺. |
| **Compound 106** | 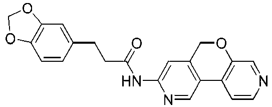 | LC_MS: (ES⁺): m/z 376.1 [M+H]⁺. |
| **Compound 107** | 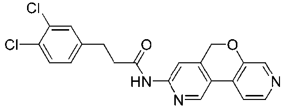 | LC_MS: (ES⁺): m/z 400.0 [M+H]⁺. |
| **Compound 108** | 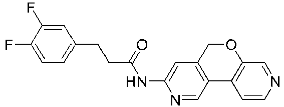 | LC_MS: (ES⁺): m/z 368.1 [M+H]⁺. |
| **Compound 109** | 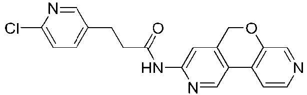 | LC_MS: (ES⁺): m/z 367.1 [M+H]⁺. |
| **Compound 110** | 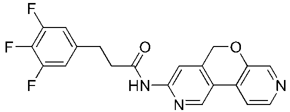 | LC_MS: (ES⁺): m/z 386.1 [M+H]⁺. |
| **Compound 111** | 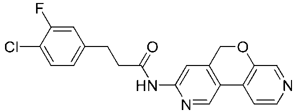 | LC_MS: (ES⁺): m/z 384.1 [M+H]⁺. |
| **Compound 113** | 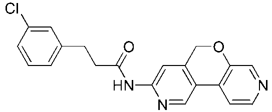 | LC_MS: (ES⁺): m/z 366.1 [M+H]⁺. |
| **Compound 114** | 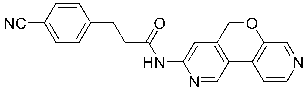 | LC_MS: (ES⁺): m/z 357.1 [M+H]⁺. |
| **Compound 115** | 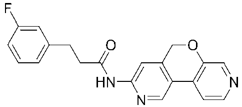 | LC_MS: (ES⁺): m/z 350.1 [M+H]⁺. |
| **Compound 116** | 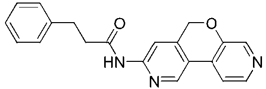 | LC_MS: (ES⁺): m/z 332.1 [M+H]⁺. |
| **Compound 117** | 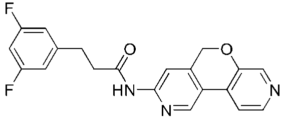 | LC_MS: (ES⁺): m/z 368.1 [M+H]⁺. |
| **Compound 118** | 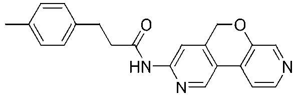 | LC_MS: (ES⁺): m/z 346.1 [M+H]⁺. |
| **Compound 119** | 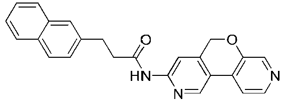 | LC_MS: (ES⁺): m/z 382.1 [M+H]⁺. |
| **Compound 120** | 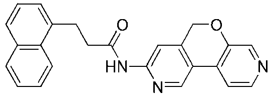 | LC_MS: (ES⁺): m/z 382.1 [M+H]⁺. |
| **Compound 121** | 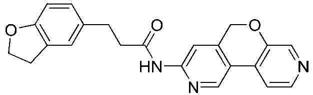 | LC_MS: (ES⁺): m/z 374.1 [M+H]⁺. |

The synthesis route of the following compounds is shown below, which were synthesized using corresponding reagents following Step 1 of the synthesis route of Example 13:

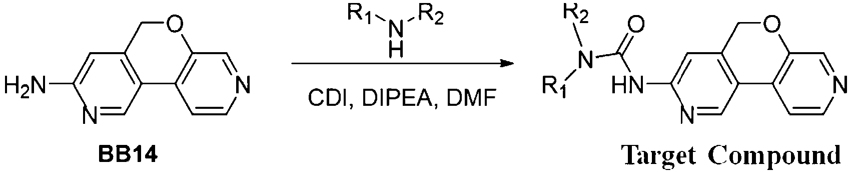

| **Compound Number** | **Structural Formula** | **Mass Spectrum and NMR** |
|---|---|---|
| **Compound 123** | 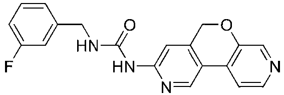 | LC_MS: (ES⁺): m/z 351.1 [M+H]⁺. |
| **Compound 124** | 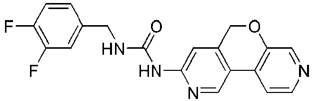 | LC_MS: (ES⁺): m/z 369.1 [M+H]⁺. |
| **Compound 125** | 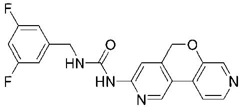 | LC_MS: (ES⁺): m/z 369.1 [M+H]⁺. |

The synthesis routes of the following compounds were as follows, which were synthesized using corresponding reagents following Step 1 of the synthesis route of Example 1:

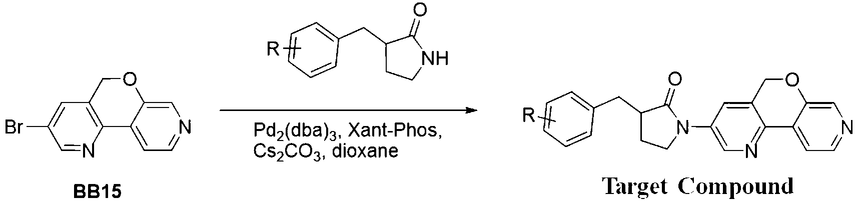

| **Compound Number** | **Structural Formula** | **Mass Spectrum and NMR** |
|---|---|---|
| **Compound 126** | 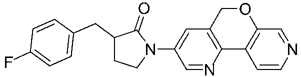 | LC_MS: (ES⁺): m/z 376.1 [M+H]⁺. |
| **Compound 127** | 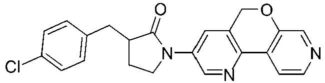 | LC_MS: (ES⁺): m/z 392.1 [M+H]⁺. |
| **Compound 152** | 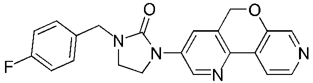 | LC_MS: (ES⁺): m/z 377.1 [M+H]⁺. |

The synthesis routes of the following compounds were as follows, which were synthesized using corresponding reagents following the synthesis route of the first step of Example 1:

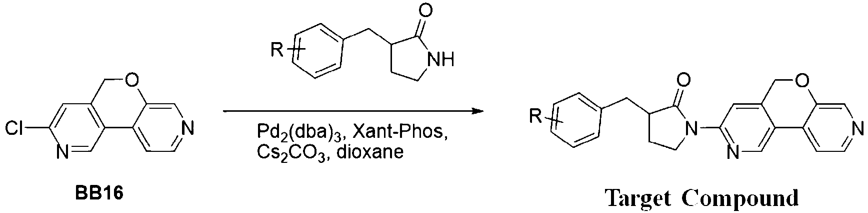

| Compound Number | Structural Formula | Mass Spectrum and NMR | |
|---|---|---|---|
| | | | LC_MS: (ES⁺): m/z 376.1 [M+H]⁺. |
| **Compound 128** | 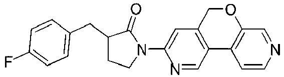 | ¹H NMR (400 MHz, DMSO-*d*₆) *δ* 8.99 (s, 1H), 8.31-8.26 (m, 3H), 7.93 (d, *J =* 5.1 Hz, 1H), 7.33-7.29 (m, 2H), 7.15-7.10 (m, 2H), 5.33 (s, 2H), 4.02-3.96 (m, 1H), 3.84-3.78 (m, 1H), 3.16-3.03 (m, 2H), 2.76 (dd, *J*₁ *=* 13.3, *J*₂ =8.7 Hz, 1H), 2.10-2.03 (m, 1H), 1.81-1.71 (m, 1H). | |
| **Compound 129** | 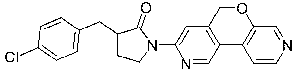 | LC_MS: (ES⁺): m/z 392.1 [M+H]⁺. | |
| **Compound 151** | 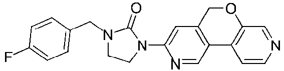 | LC_MS: (ES⁺): m/z 377.1 [M+H]⁺. | |

The synthesis routes of the following compounds were as follows, which were synthesized following the synthesis method of the first step of Example 1 and using corresponding reagents:

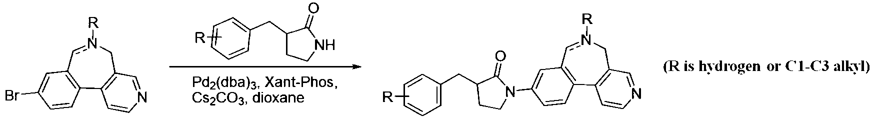

| Compound Number | Structural Formula | Mass Spectrum and NMR | |
|---|---|---|---|
| **Compound 133** | 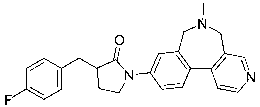 | | LC_MS: (ES⁺): m/z 402.2 [M+H]⁺. |
| | | ¹H NMR (400 MHz, CDCl₃) δ 8.69 (d, *J* = 5.0 Hz, 1H), 8.60 (s, 1H), 7.83 (dd, *J =* 8.4, 2.1 Hz, 1H), 7.67 (d, *J =* 2.1 Hz, 1H), 7.55 (d, *J =* 8.4 Hz, 1H), 7.42 (d, *J =* 5.0 Hz, 1H), 7.24 (dd, *J =* 8.4, 5.5 Hz, 2H), 7.02 (t, *J =* 8.6 Hz, 2H), 3.81 (dd, *J =* 16.8, 8.4 Hz, 1H), 3.71 (td, *J =* 9.0, 3.1 Hz, 1H), 3.43 (s, 4H), 3.29 (dd, *J =* 13.7, 4.0 Hz, 1H), 2.97 (ddd, *J* = 17.6, 8.9, 4.1 Hz, 1H), 2.86 (dd, *J =* 13.7, 9.0 Hz, 1H), 2.52 (s, 3H), 2.26 (ddd, *J =* 12.8, 8.2, 3.9 Hz, 1H), 1.91 (ddd, *J =* 17.5, 12.7, 8.7 Hz, 1H). | |
| **Compound 134** | 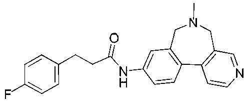 | | LC_MS: (ES⁺): m/z 376.2 [M+H]⁺. |
| | | ¹H NMR (400 MHz, CDCl₃) δ 8.70 (d, *J =* 5.0 Hz, 1H), 8.59 (s, 1H), 7.72 (d, *J* = 7.9 Hz, 1H), 7.51 (t, *J =* 10.6 Hz, 3H), 7.41 (d, *J* = 5.0 Hz, 1H), 7.23 (dd, *J =* 8.3, 5.5 Hz, 2H), 7.01 (t, *J =* 8.7 Hz, 2H), 3.44 (d, *J =* 8.4 Hz, 4H), 3.07 (t, *J =* 7.5 Hz, 2H), 2.71 (t, *J =* 7.5 Hz, 2H), 2.54 (s, 3H). | |
| **Compound 144** | 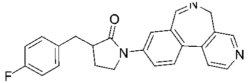 | LC_MS: (ES⁺): m/z 385.1 [M+H]⁺. | |

The synthesis routes of the following compounds were as follows, which were synthesized using corresponding reagents following Step 1 of the synthesis route of Example 1:

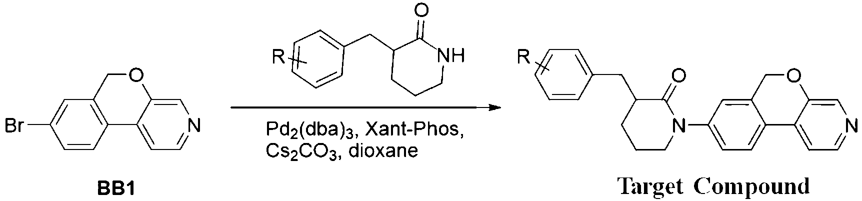

| **Compound Number** | **Structural Formula** | **Mass Spectrum and NMR** | |
|---|---|---|---|
| | | | LC_MS: (ES⁺): m/z 389.1 [M+H]⁺. |
| **Compound 137** | 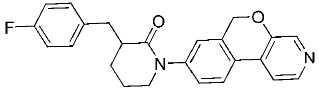 | ¹H NMR (400 MHz, CDCl₃) δ 8.37 (s, 1H), 8.31 (d, *J =* 4.7 Hz, 1H), 7.78 (d, *J =* 8.2 Hz, 1H), 7.56 (d, *J =* 4.7 Hz, 1H), 7.31 (d, *J =* 8.3 Hz, 1H), 7.25 - 7.19 (m, 2H), 7.15 (s, 1H), 7.01 (t, *J =* 8.3 Hz, 2H), 5.20 (s, 2H), 3.66 (d, *J =* 4.2 Hz, 2H), 3.41 - 3.33 (m, 1H), 2.90 - 2.81 (m, 1H), 2.79 - 2.70 (m, 1H), 1.96 (dd, *J =* 18.2, 8.9 Hz, 3H), 1.69 - 1.58 (m, 1H). | |

The synthesis routes of the following compounds were as follows, which were synthesized using corresponding reagents following Step 1 of the synthesis route of Example 14:

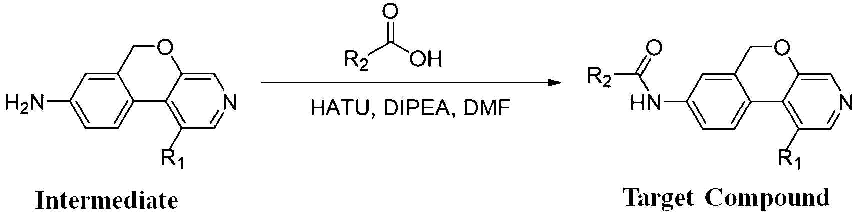

| **Compound Number** | **Structural Formula** | **Mass Spectrum and NMR** |
|---|---|---|
| **Compound 140** | 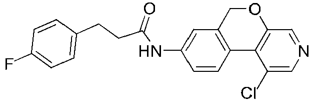 | LC_MS: (ES⁺): m/z 383.1 [M+H]⁺. |
| **Compound 141** | 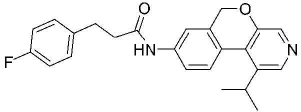 | LC_MS: (ES⁺): m/z 391.2 [M+H]⁺. |

### Biological Activity Testing Examples:

### Biological Activity Example 1: Preparation of SARM1 Enzyme

### Preparation of Test Compounds:

The stock solution concentrations of the test compounds were 200 µM or 10 mM (in DMSO), which were further diluted to required compound concentrations during in vitro SARM1 enzyme assays and inhibitor screening.

### Expression and Purification of SARM1 Protein

### (1) Plasmid Construction

The gene sequence of SARM1 protein was amplified using PCR, and the PCR amplification product was constructed into pcDNA3.1-HIS-C plasmid. The above construction was completed by YouBio(Hunan, China).

### (2) Plasmid Transfection

HEK293 cells were cultured in 10 cm dishes using DMEM, and the cells were transfected with 15 µg pcDNA3.1-SARM1-HIS-C expression plasmid on the following day.

### (3) Cell Collection and Protein Extraction

Cells were collected 48 hours after transfection, and SARM1 protein expressed in the cells was obtained by digitonin lysis for in vitro activity assay experiments. The specific procedure was as follows:
Cells were digested with trypsin-EDTA, then centrifuged at 1000 rpm for 5 minutes, washed once with PBS, and then resuspended in PBS containing 100 µM digitonin, with 0.6 mL PBS for the cells in per 10 cm dish, and lysed for 5 minutes. Cells were taken and observed under a microscope after adding trypan blue, if more than 90% of the cells had been lysed. Then the supernatant containing SARM1 protein was collected by centrifugation at 5000 rpm for 10 minutes.

### Biological Activity Example 2: In Vitro Biochemical Assay for Inhibiting SARM1 Enzyme Activity (IC₅₀):

First, 200 µM compound was added to 50 mM Tris-HCl (pH 7.5) solution containing 0.05 µg/ml SARM1, then half was taken and mixed with an equal volume of 50 mM Tris-HCl (pH 7.5) solution containing 0.05 µg/ml SARM1, and so on to dilute the compound 6 times, with final serial concentrations of 200, 100, 50, 25, 12.5, 6.25, 3.125 µM, or 200, 50, 12.5, 3.125, 0.78, 0.195, 0.049 µM, respectively. A group without inhibitor was used as control group. All mixtures were incubated at room temperature for 10 minutes.

Then, 50 µM NAD and 50 µM PC6 as substrates and 50 µM NMN as an activator were added to the SARM1 protein incubated with the inhibitor, and the reaction was carried out at room temperature for 30 minutes. The concentrations of each component were the final concentrations in the reaction system.

During the reaction, PC6 fluorescence spectrum kinetics were detected by a microplate reader, with excitation wavelength and emission wavelength of 390 nm and 520 nm, respectively. Finally, the reaction rate was used to represent the protein activity and calculate the half-maximal inhibitory concentration. A higher reaction rate indicated stronger protein activity and lower inhibition efficiency of the compound.

The dose-response curves for compounds inhibiting SARM1 NADase activity were obtained using the above method.

Table 1 below provides the IC₅₀ ranges of these compounds determined by the assay:

IC₅₀ ranges for inhibiting SARM1 enzyme activity: A <0.1 µM; B: 0.1-1 µM; C: 1- 10 µM

**Table 1**

| **Compound Number** | **IC₅₀** |
|---|---|
| 1 | A |
| 2 | B |
| 3 | C |
| 4 | B |
| 5 | C |
| 6 | B |
| 7 | C |
| 8 | C |
| 9 | C |
| 10 | A |
| 11 | C |
| 12 | B |
| 13 | C |
| 14 | C |
| 15 | C |
| 16 | C |
| 17 | B |
| 18 | C |
| 19 | C |
| 20 | A |
| 21 | A |
| 22 | B |
| 23 | C |
| 24 | B |
| 25 | A |
| 26 | A |
| 27 | A |
| 28 | B |
| 29 | A |
| 30 | B |
| 31 | A |
| 32 | B |
| 33 | A |
| 34 | A |
| 35 | B |
| 36 | C |
| 37 | B |
| 38 | A |
| 39 | A |
| 40 | C |
| 41 | B |
| 42 | A |
| 43 | A |
| 44 | A |
| 45 | A |
| 46 | B |
| 47 | B |
| 48 | B |
| 49 | C |
| 50 | B |
| 51 | C |
| 52 | B |
| 53 | C |
| 56 | C |
| 57 | B |
| 58 | C |
| 59 | C |
| 61 | B |
| 62 | B |
| 63 | B |
| 64 | C |
| 65 | C |
| 66 | B |
| 67 | A |
| 68 | B |
| 69 | C |
| 70 | C |
| 71 | C |
| 72 | B |
| 73 | B |
| 74 | A |
| 75 | A |
| 76 | B |
| 77 | A |
| 78 | B |
| 79 | C |
| 80 | A |
| 81 | C |
| 82 | C |
| 83 | B |
| 84 | A |
| 87 | A |
| 91 | A |
| 94 | A |
| 95 | A |
| 96 | A |
| 101 | A |
| 112 | A |
| 126 | A |
| 128 | A |
| 130 | A |
| 131 | C |
| 133 | B |
| 134 | B |
| 135 | B |
| 136 | A |
| 137 | B |
| 138 | A |
| 139 | A |
| 140 | B |
| 141 | C |
| 142 | B |
| 143 | A |
| 144 | A |
| 145 | A |
| 146 | A |
| 147 | A |
| 148 | A |
| 149 | C |
| 150 | A |
| 153 | A |
| 154 | A |
| 155 | A |

### Biological Activity Example 3: Axonal Degeneration Index

The experimental method was based on Gerdts J et al. (Gerdts J, Sasaki Y, Vohra B, Marasa J, Milbrandt J. Image-based screening identifies novel roles for IkappaB kinase and glycogen synthase kinase 3 in axonal degeneration. J Biol Chem. 2011 Aug 12;286(32):28011-8).

### Mouse DRG Explant Culture

Dorsal root ganglia were dissected from 2-month-old C57BL/6 male mice and placed in 35 mm culture dishes containing pre-cooled HBSS buffer. Excess tissue from each dorsal root ganglion was removed and cut into 2-3 explants using a scalpel, and the explants were planted in wells of a 96-well plate coated with 100 ng/mL Matrigel (Corning). 100 µL of DRG culture medium (Neurobasal medium (Gibco) containing 2% B-27 (Gibco), 1% GlutaMax (Gibco), and 10 ng/mL NGF (MCE)) was added, then cultured in a 37°C incubator for one week, with medium change on day 4, and experiments were performed on day 8.

### Axonal Wallerian Degeneration Assay

0.1 µL of 10 mM test compound was dissolved in 100 µL DRG culture medium to prepare new culture medium containing 10 µM test compound. The culture medium in the 96-well plate was aspirated and new culture medium containing the test compound was added. Then the dorsal root ganglion explants were incubated with the medium in a 37°C incubator for 2 hours for pre-protection. After 2-hour pretreatment, a glass electrode was used to transect axons in the area near the tissue block, and bright-field images of axons in the same area were captured at 0 hour and 24 hours post axonal degeneration using a 20x objective lens of a microscope (Olympus). Axonal integrity was evaluated using an in-house developed script.

The compounds of the present disclosure exhibited neuroprotective effects in the explant axonal Wallerian degeneration assay at a concentration of 10 µM, and were classified according to the degree of protection: substantial protection (A) >50%, partial protection (B): 20% to 50%. The results are listed in Table 2 below.

**Table 2**

| **Compound Number** | **Degree of Protection Against Axonal Wallerian Degeneration** |
|---|---|
| 1 | A |
| 2 | A |
| 4 | A |
| 5 | A |
| 7 | A |
| 9 | A |
| 10 | A |
| 11 | A |
| 12 | B |
| 13 | A |
| 15 | A |
| 16 | A |
| 17 | B |
| 18 | B |
| 19 | A |
| 21 | A |
| 22 | A |
| 25 | A |
| 27 | B |
| 28 | A |
| 29 | A |
| 30 | A |
| 32 | B |
| 33 | A |
| 34 | A |
| 36 | A |
| 37 | A |
| 39 | A |
| 40 | A |
| 41 | A |
| 42 | A |
| 45 | A |
| 46 | A |
| 47 | A |
| 50 | A |
| 56 | B |
| 57 | B |
| 58 | B |
| 59 | B |
| 62 | A |
| 63 | A |
| 65 | B |
| 67 | A |
| 68 | A |
| 70 | B |
| 71 | B |
| 72 | B |
| 73 | A |
| 74 | B |
| 75 | B |
| 76 | A |
| 77 | B |
| 78 | A |
| 80 | A |
| 84 | B |
| 87 | A |
| 91 | A |
| 94 | A |
| 95 | B |
| 96 | A |
| 101 | A |
| 112 | A |
| 126 | A |
| 128 | A |
| 130 | B |
| 133 | B |
| 134 | B |
| 135 | A |
| 136 | A |
| 137 | A |
| 138 | A |
| 139 | A |
| 140 | B |
| 142 | A |
| 143 | A |
| 144 | B |
| 145 | A |
| 146 | A |
| 147 | A |

## Claims

1. A compound of Formula (I): 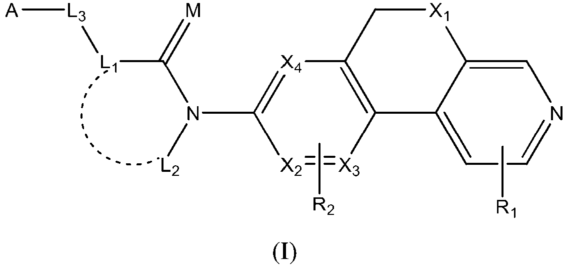
or a racemate, an enantiomer, a diastereomer, a deuterated compound, or a pharmaceutically acceptable salt thereof,
wherein,
X₁ is selected from the group consisting of -O-, -CH₂-, -OCH₂-, -NR₃CH₂-, -CH₂NR₃-, -N=CH-, and -CH=N-, preferably -O-;
wherein R₃ is selected from the group consisting of H and C₁-C₃ alkyl;
X₂, X₃, and X₄ are each independently selected from the group consisting of CH and N;
R₁ is independently selected from the group consisting of H, halogen, C₁-C₃ alkyl, cyano, trifluoromethyl, amino, hydroxy, methoxy, and -C(O)NH₂, preferably H, F, Cl, methyl, cyano, and -C(O)NH₂;
R₂ is independently selected from the group consisting of H, halogen, C₁-C₃ alkyl, cyano, trifluoromethyl, amino, hydroxy, methoxy, C₁-C₃ alkyl-OC(O)-, and C₁-C₃ alkyl-NHC(O)-, preferably H, cyano, Cl, methyl, and methoxy;
M represents O, S, or NH, preferably O;
L₁ and L₃ are each independently selected from the group consisting of -CH(R')-, -O-, -S-, and -N(R')-, provided that at least one of L₁ and L₃ is -CH(R')-, wherein R' is hydrogen or C₁-C₃ alkyl;
L₂ is selected from the group consisting of H, -CH₃, -CH₂-CH₃, -CH₂-CH₂-CH₃, -OR₈, and -NHR₉;
wherein R₈ and R₉ are independently selected from the group consisting of H and C₁-C₃ alkyl;
A is selected from the group consisting of H, a 5- to 10-membered aryl, a 5- to 10-membered heteroaryl, a 5- to 10-membered cycloalkyl, a 5- to 10-membered heterocycloalkyl, a 5- to 10-membered aryl fused with a 3- to 6-membered cycloalkyl, and a 5- to 10-membered aryl fused with a 3- to 6-membered heterocycloalkyl, wherein the heteroaryl or heterocycloalkyl may contain 1 or 2 heteroatoms selected from N, O, and S, and the 5- to 10-membered aryl, 5- to 10-membered heteroaryl, 5- to 10-membered aryl fused with a 3- to 6-membered cycloalkyl, and 5- to 10-membered aryl fused with a 3- to 6-membered heterocycloalkyl may be substituted by 1, 2, or 3 substituents selected from the group consisting of halogen, C₁-C₃ alkyl, C₁-C₃ alkoxy, halogenated C₁-C₃ alkyl, halogenated C₁-C₃ alkoxy, cyano, nitro, and (R^{a}R^{a}')N-, wherein R^{a} and R^{a'} are each independently selected from hydrogen and C₁-C₃ alkyl; preferably, the substituent is selected from the group consisting of methyl, methoxy, trifluoromethyl, trifluoromethoxy, F, Cl, and Br;
L₁ may be connected to L₂ to form a 5-membered ring or a 6-membered ring, preferably forming a 5-membered ring.

2. The compound of Formula (I) or a racemate, an enantiomer, a diastereomer, a deuterated compound, or a pharmaceutically acceptable salt thereof according to claim 1, wherein the compound of Formula (I) has a structure of Formula (II) as follows: 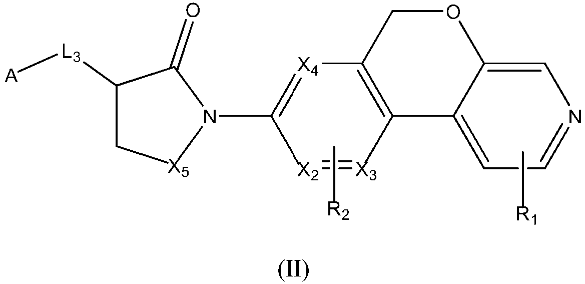 wherein,
X₂, X₃, X₄, R₁, R₂, L₃, and A are as defined in claim 1;
X₅ is selected from the group consisting of CH₂, O, and NH, preferably CH₂.

3. The compound of Formula (I) or a racemate, an enantiomer, a diastereomer, a deuterated compound, or a pharmaceutically acceptable salt thereof according to claim 1, wherein the compound of Formula (I) has a structure of Formula (III) as follows: 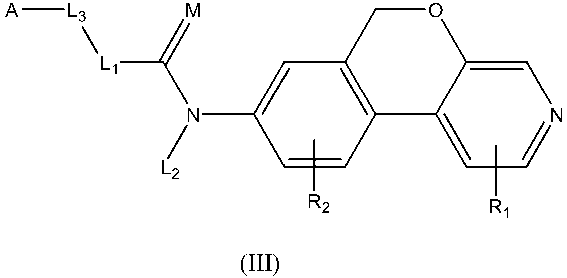 wherein,
R₁, R₂, M, L₁, L₂, L₃, and A are as defined in claim 1.

4. The compound of Formula (I) or a racemate, an enantiomer, a diastereomer, a deuterated compound, or a pharmaceutically acceptable salt thereof according to claim 1, wherein the compound of Formula (I) has a structure of Formula (IV) as follows: 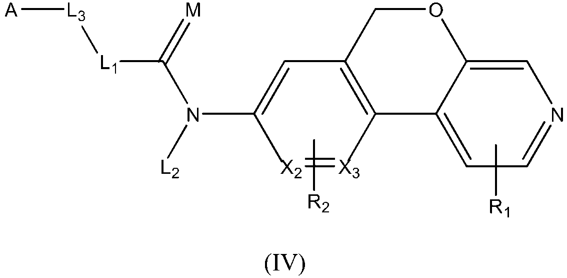 wherein,
X₂ and X₃ are selected from the group consisting of CH and N, and X₂ and X₃ are not both CH;
R₁, R₂, M, L₁, L₂, L₃, and A are as defined in claim 1.

5. The compound of Formula (II) or a racemate, an enantiomer, a diastereomer, a deuterated compound, or a pharmaceutically acceptable salt thereof according to claim 2, wherein the compound of Formula (II) has a structure of Formula (V) as follows: 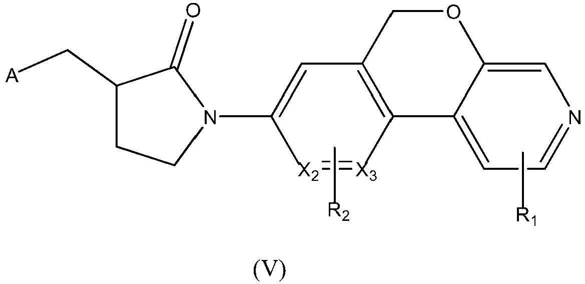 wherein,
R₁, R₂, X₂, X₃, and A are as defined in claim 1.

6. The compound of Formula (III) or a racemate, an enantiomer, a diastereomer, a deuterated compound, or a pharmaceutically acceptable salt thereof according to claim 3, wherein the compound of Formula (III) has a structure of Formula (VI) as follows: 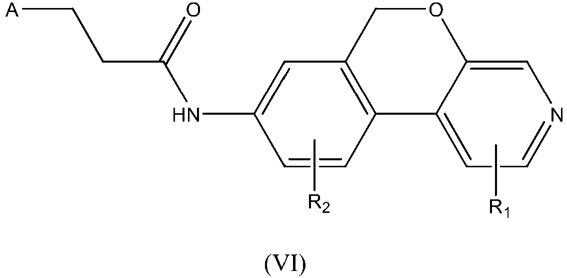 wherein,
R₁, R₂, and A are as defined in claim 1.

7. The compound of Formula (IV) or a racemate, an enantiomer, a diastereomer, a deuterated compound, or a pharmaceutically acceptable salt thereof according to claim 4, wherein the compound of Formula (IV) has a structure of Formula (VII) as follows: 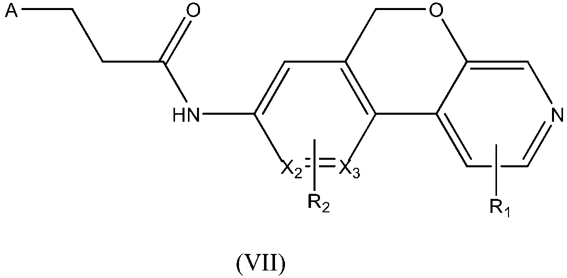 wherein,
X₂ and X₃ are selected from the group consisting of CH and N, and X₂ and X₃ are not both CH;
R₁, R₂, and A are as defined in claim 1.

8. A compound, or a racemate, an enantiomer, a diastereomer, a deuterated compound, or a pharmaceutically acceptable salt thereof, wherein the compound has a structure selected from the following:

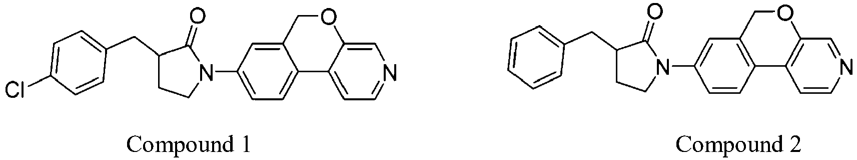

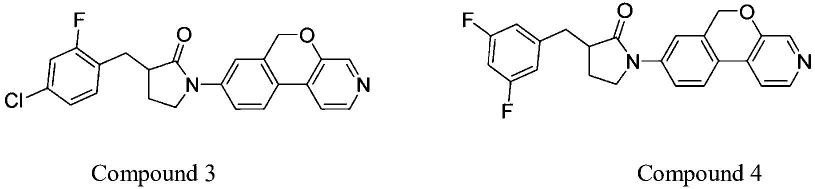

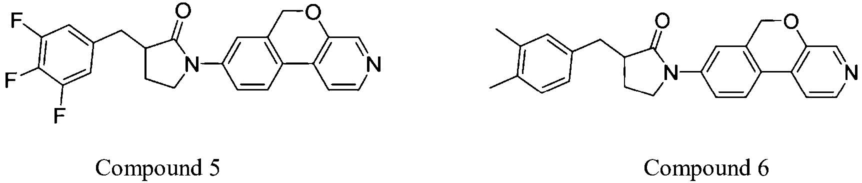

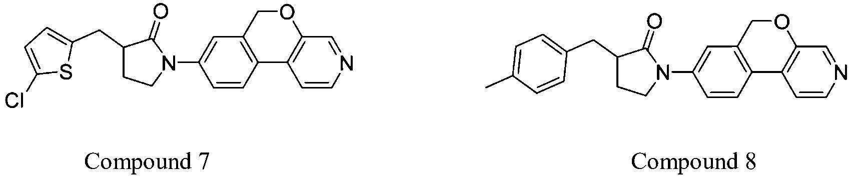

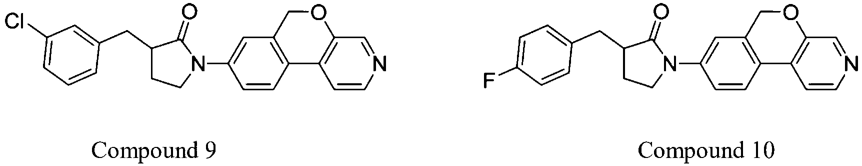

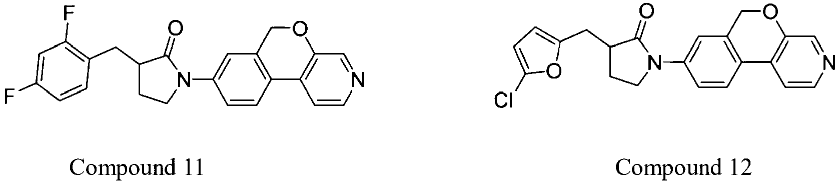

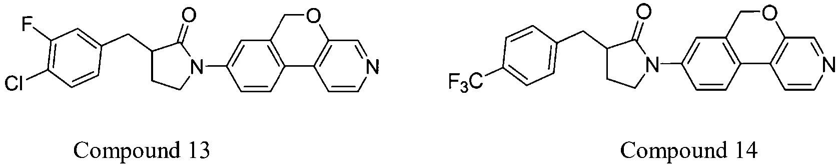

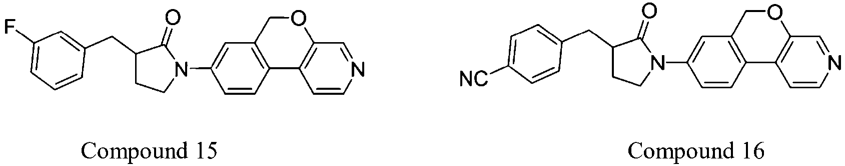

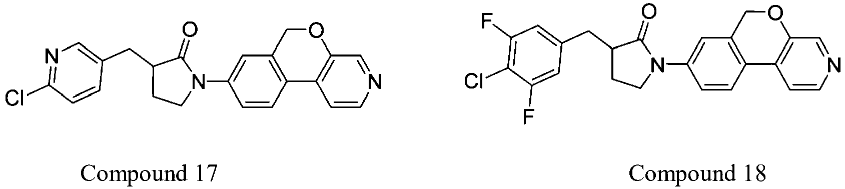

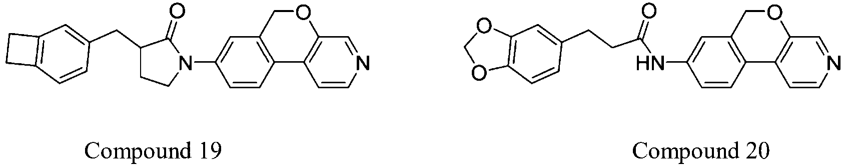

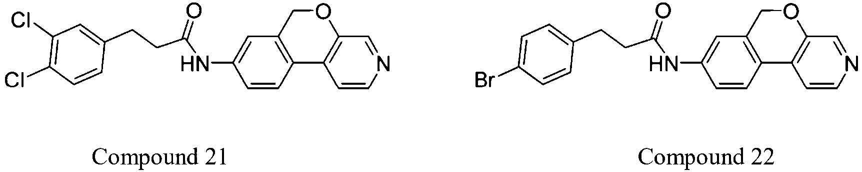

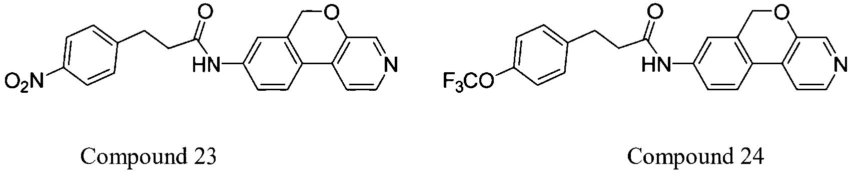

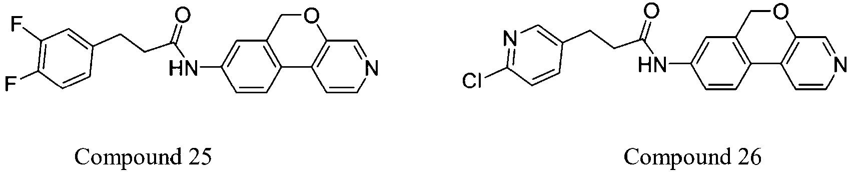

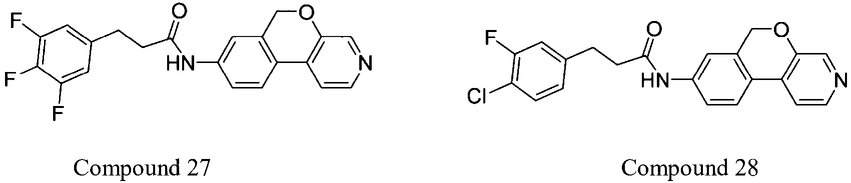

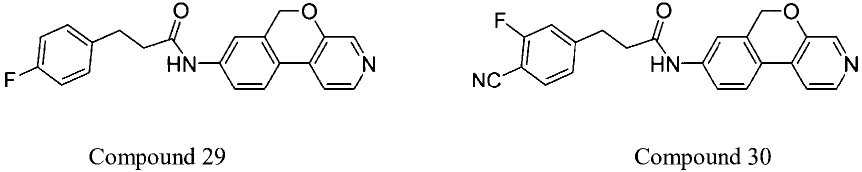

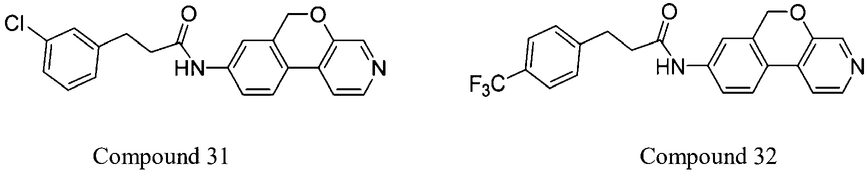

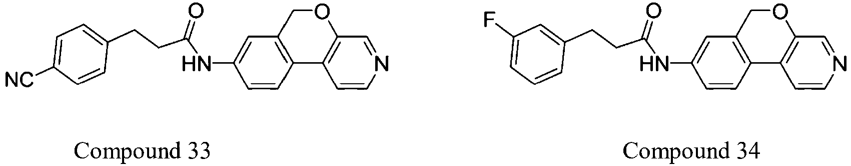

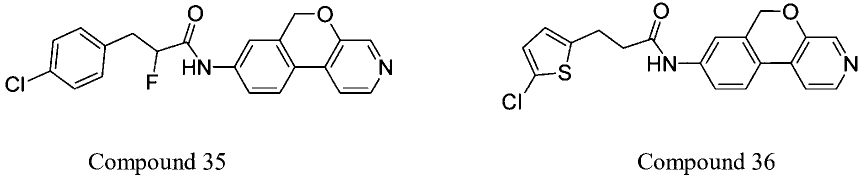

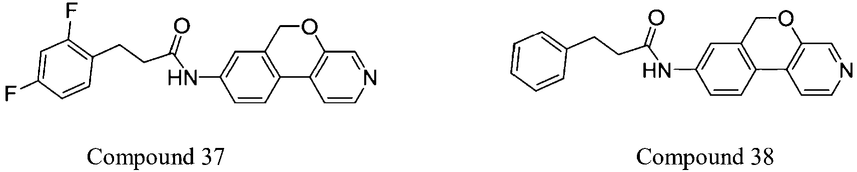

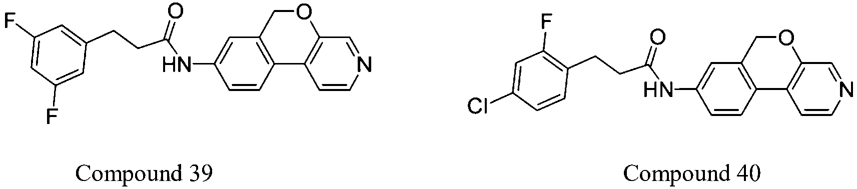

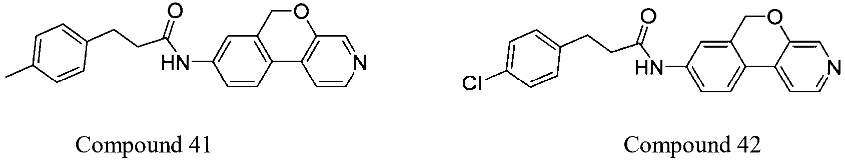

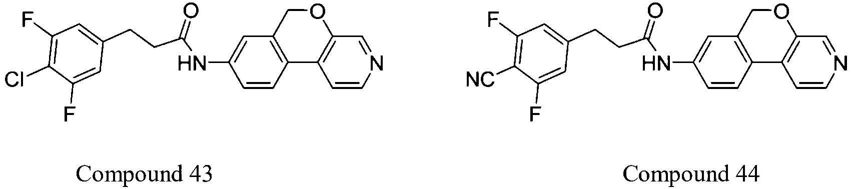

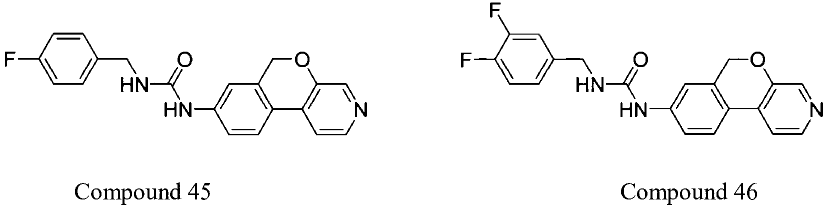

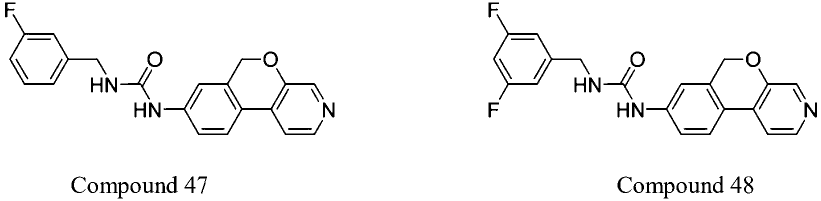

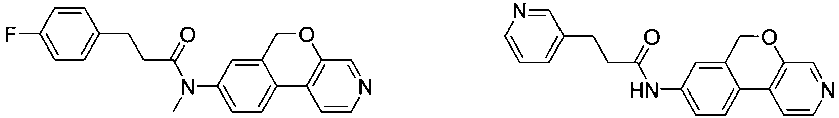

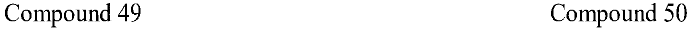

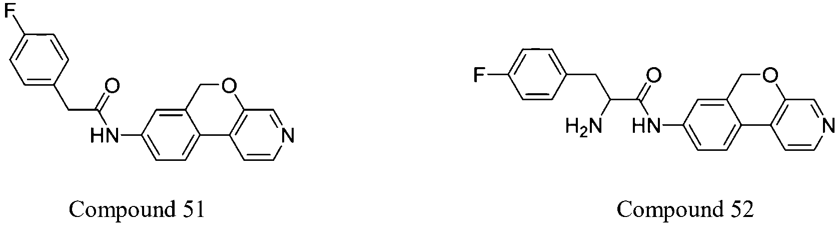

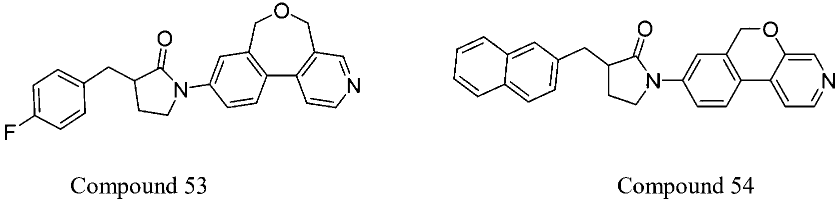

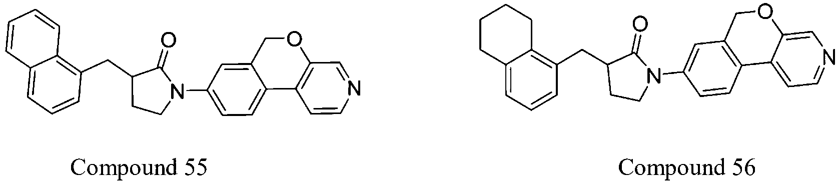

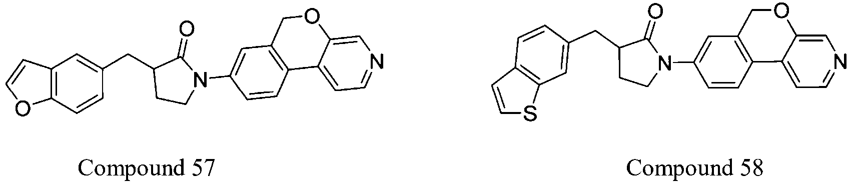

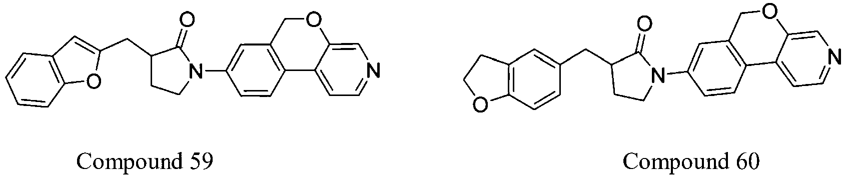

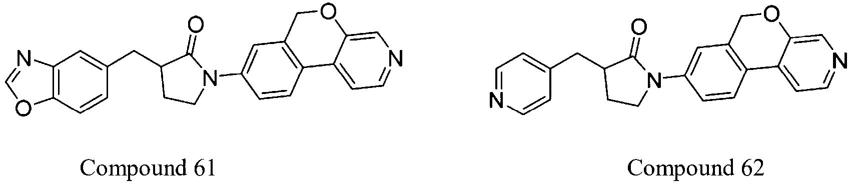

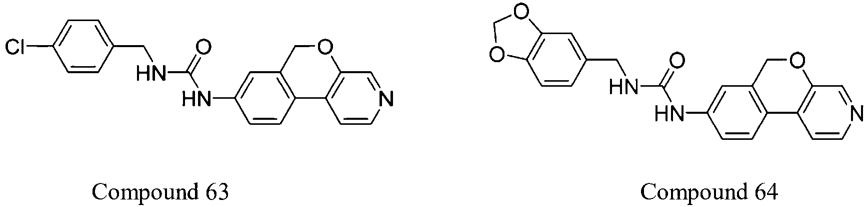

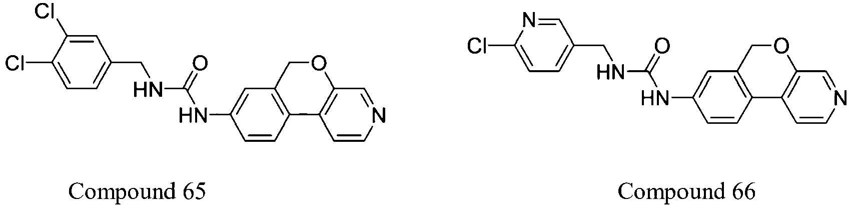

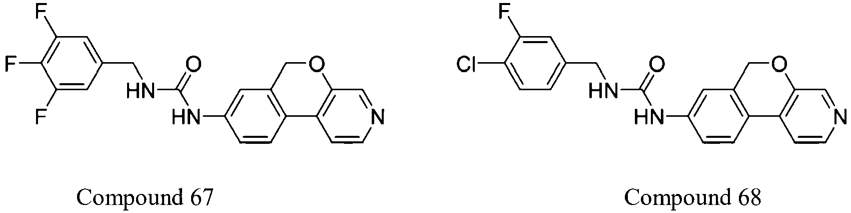

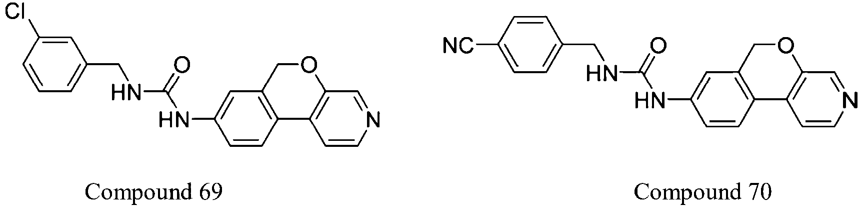

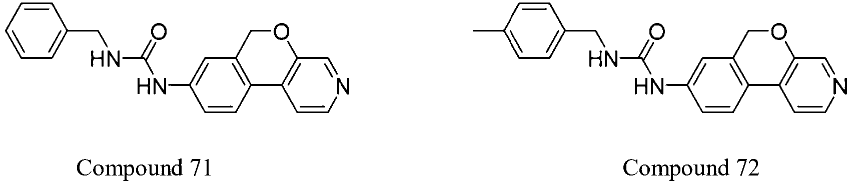

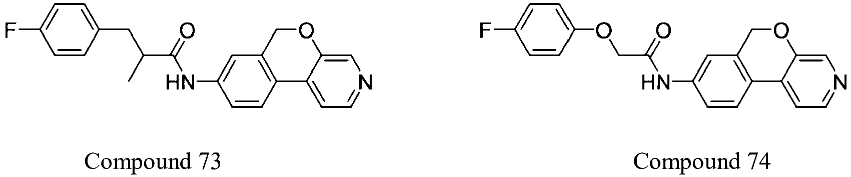

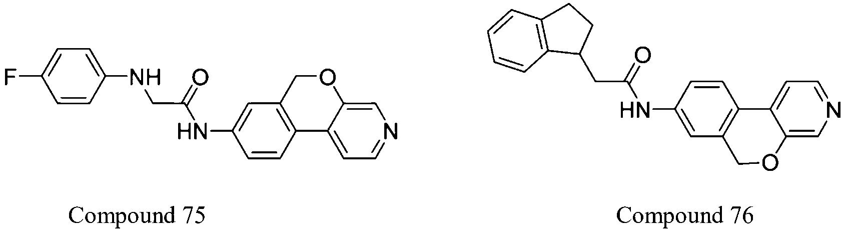

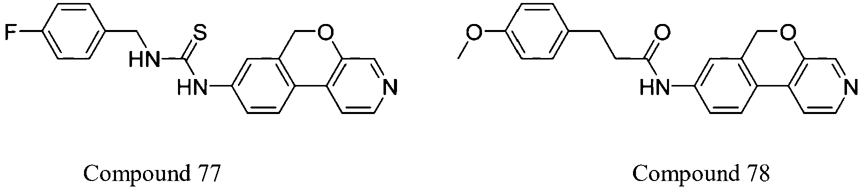

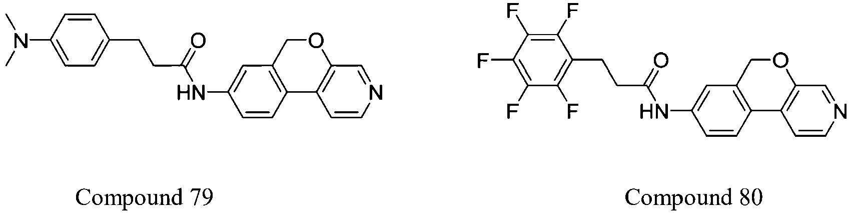

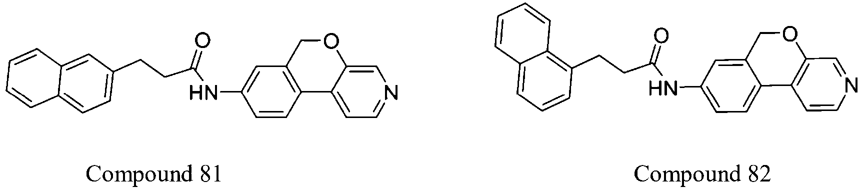

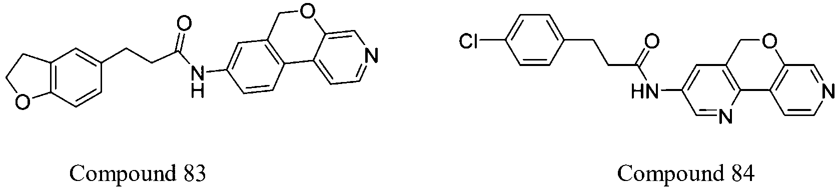

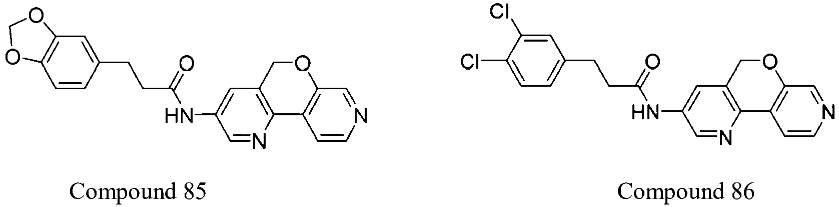

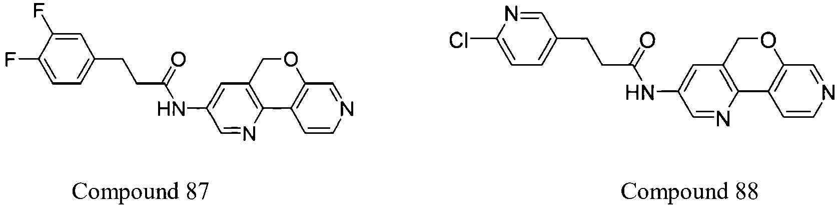

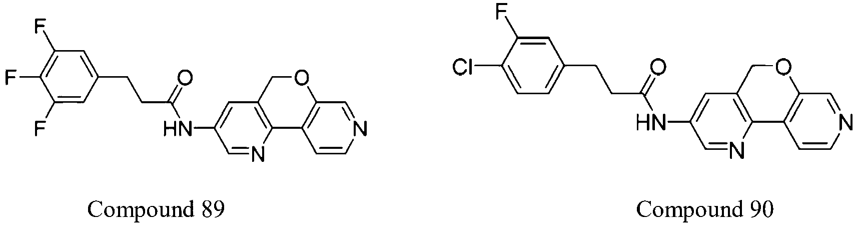

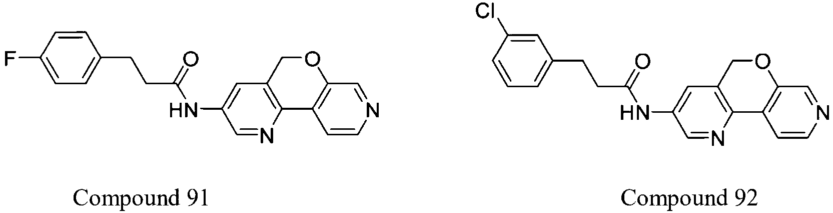

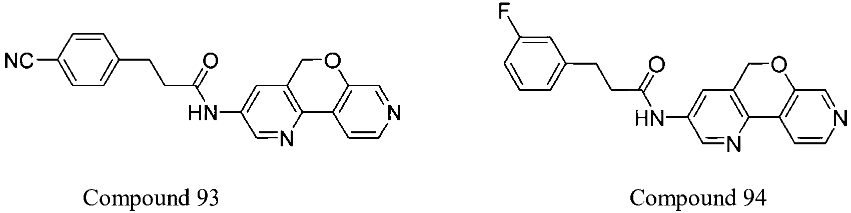

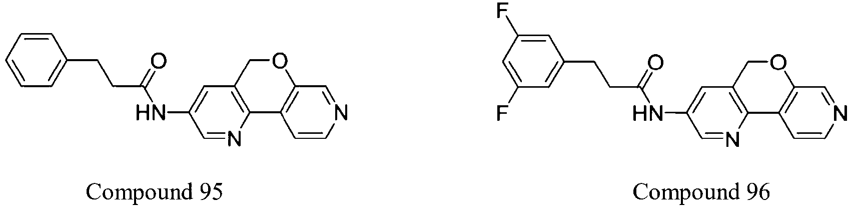

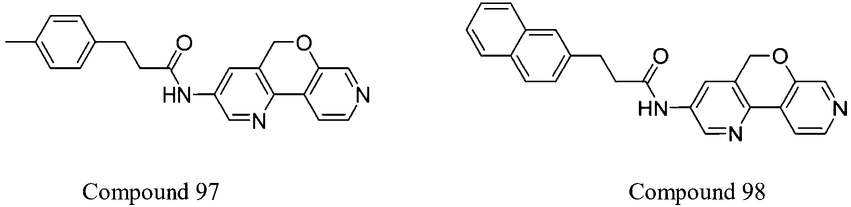

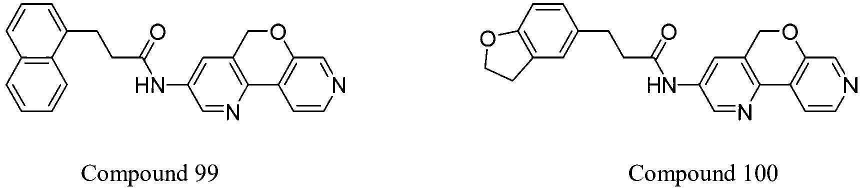

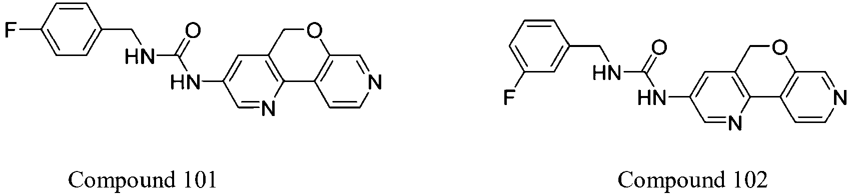

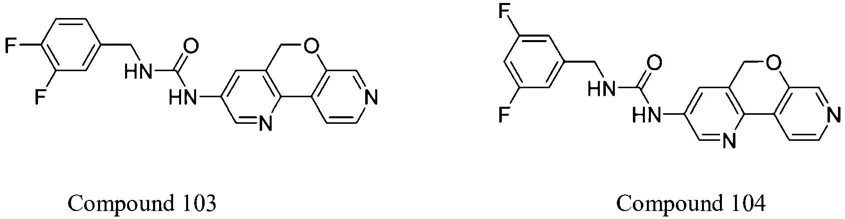

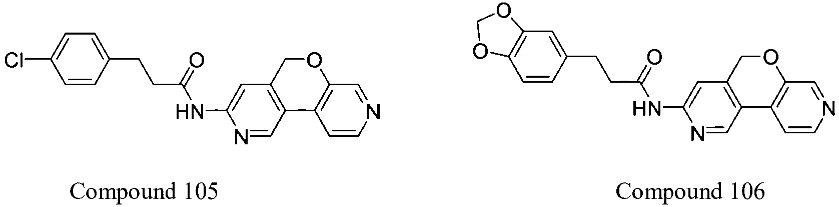

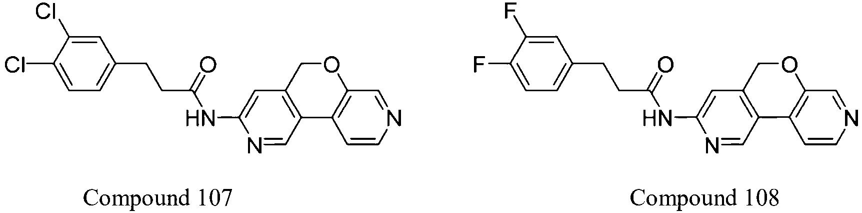

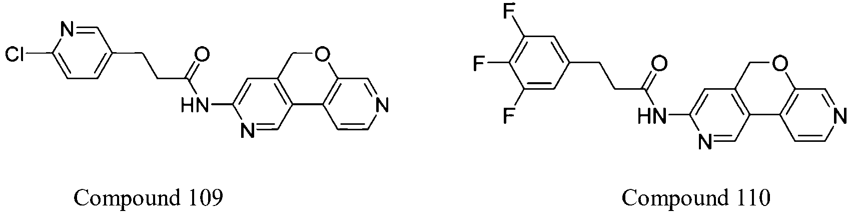

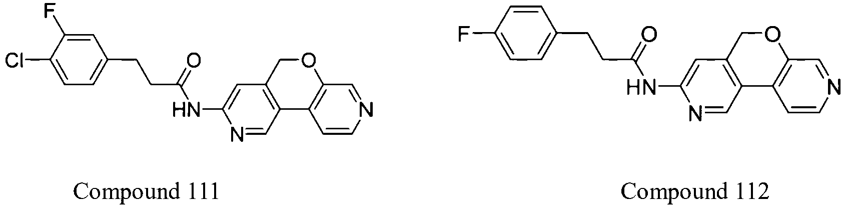

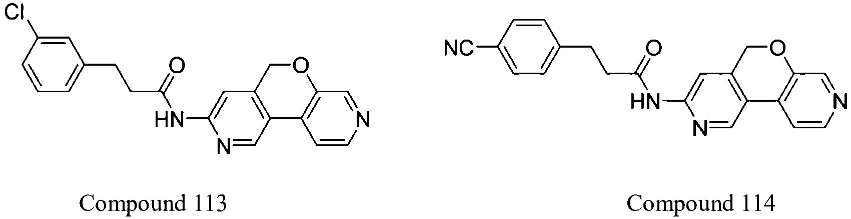

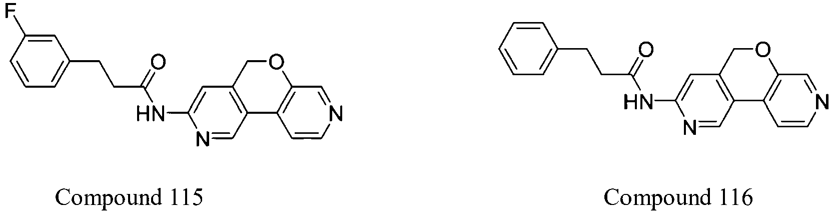

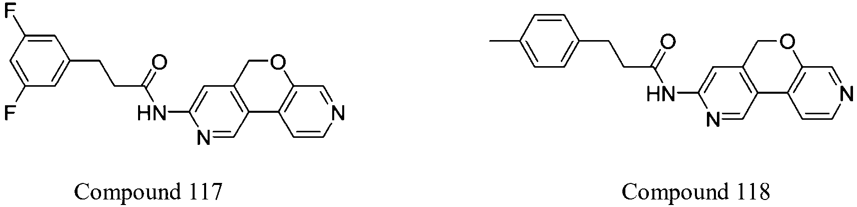

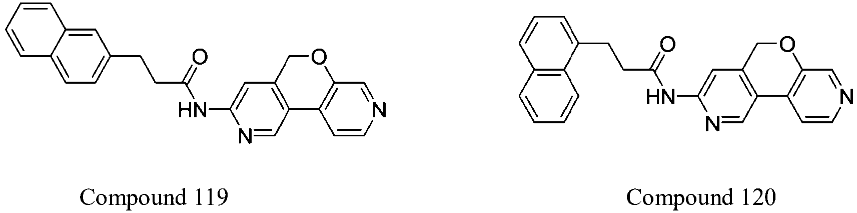

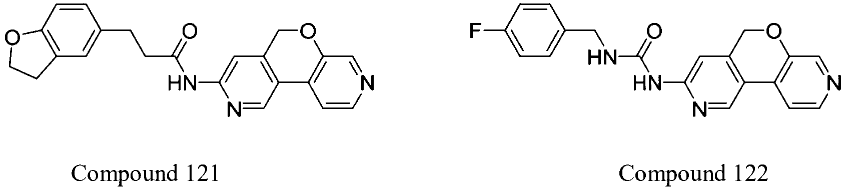

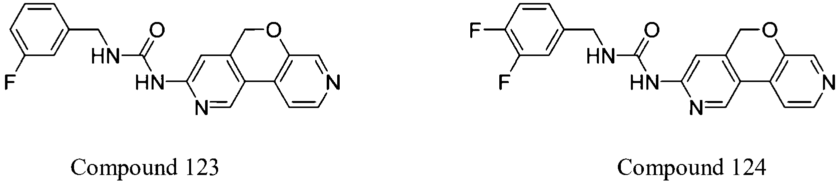

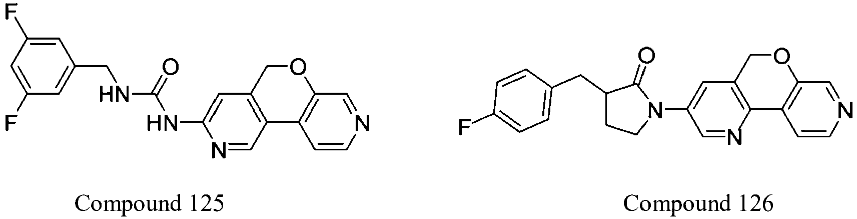

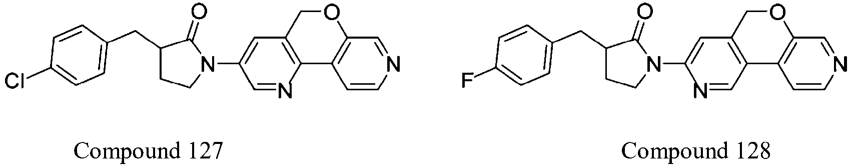

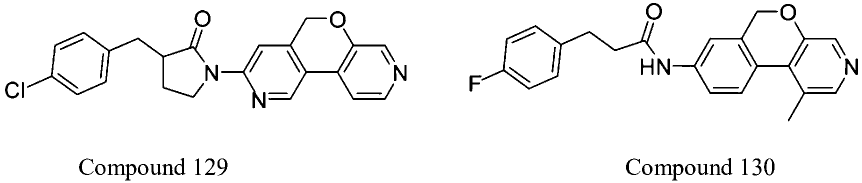

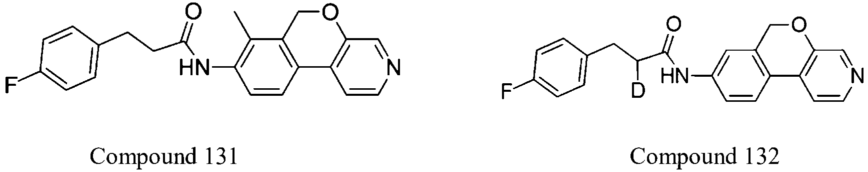

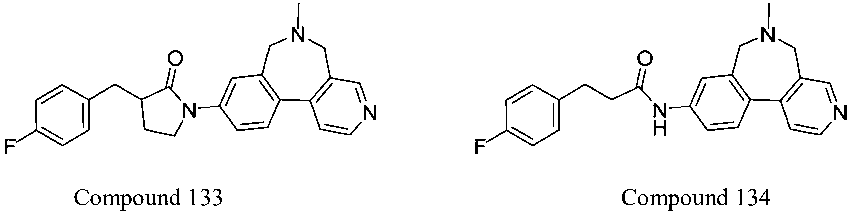

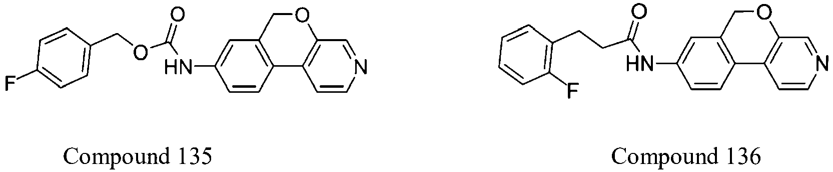

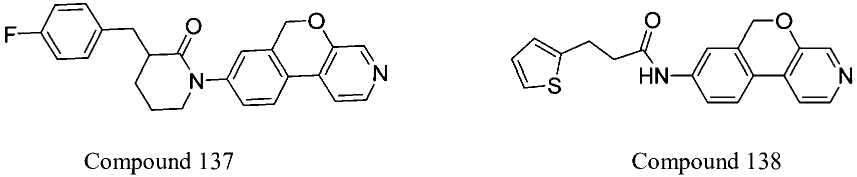

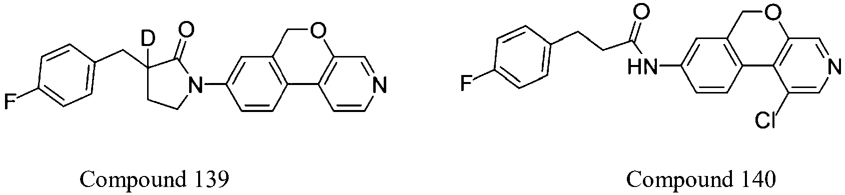

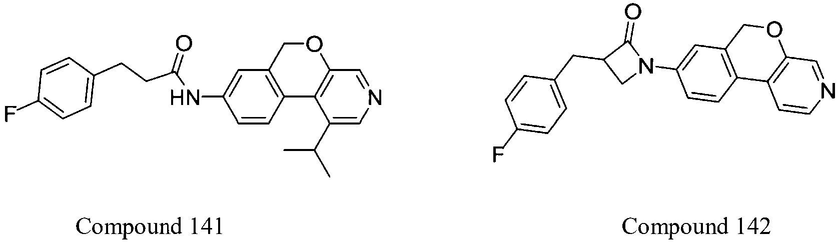

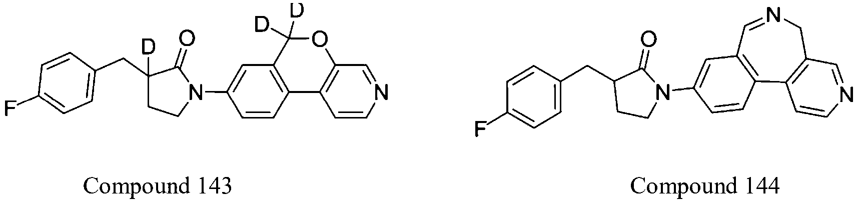

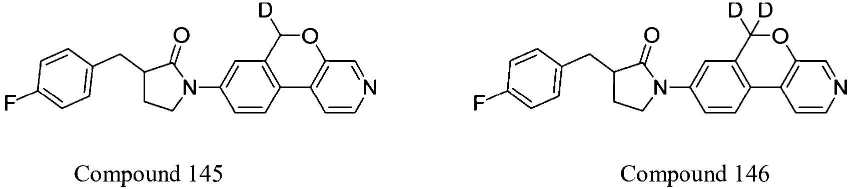

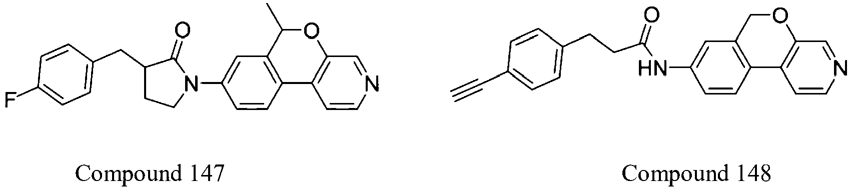

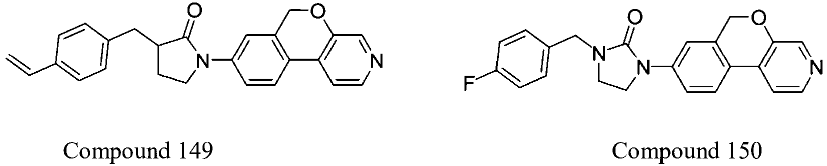

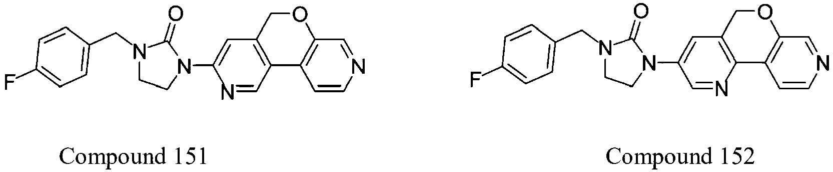

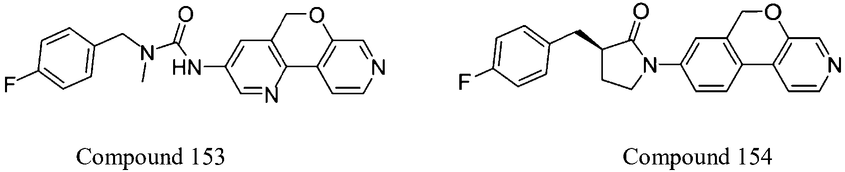

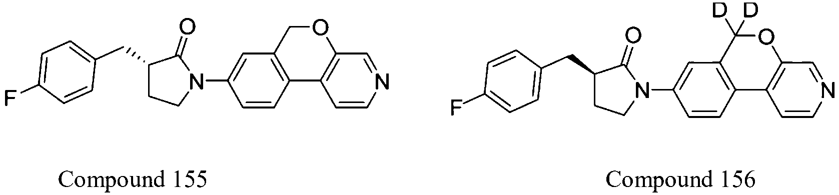

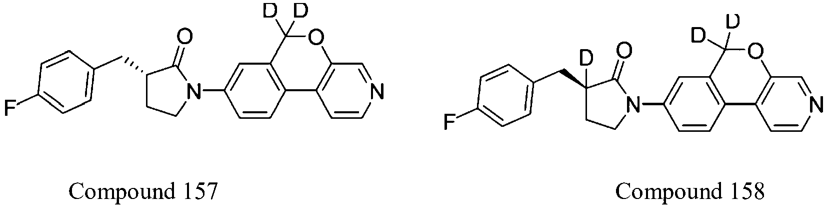

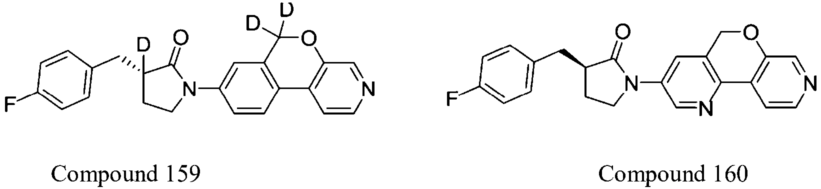

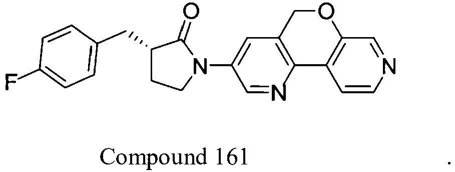

9. Use of the compound or a racemate, an enantiomer, a diastereomer, a deuterated compound, or a pharmaceutically acceptable salt thereof according to any one of claims 1 to 8 in the manufacture of a SARM1 enzyme activity inhibitor.

10. Use of the compound or a racemate, an enantiomer, a diastereomer, a deuterated compound, or a pharmaceutically acceptable salt thereof according to any one of claims 1 to 8 in the manufacture of a medicament for treating or preventing a disease or condition associated with axonal degeneration.

11. Use of the compound or a racemate, an enantiomer, a diastereomer, a deuterated compound, or a pharmaceutically acceptable salt thereof according to any one of claims 1 to 8 in the manufacture of a medicament for treating or preventing a neurodegenerative disease or a neurological disease or condition.

12. The use according to claim 9 or 10 or 11, wherein the neurodegenerative disease or the neurological disease or condition or the disease or condition associated with axonal degeneration is selected from the group consisting of Alzheimer's disease, Parkinson's disease, multiple sclerosis, amyotrophic lateral sclerosis, and peripheral neuropathy.
